# EUROPEAN PATENT SPECIFICATION

(11) **EP 3 433 620 B1**
(45) Date of publication and mention of the grant of the patent: **20.01.2021**
(21) Application number: 17714548.9
(22) Date of filing: 24.03.2017
(51) Int. Cl.: G01N 33/68

(54) **DETECTING SEPSIS**
NACHWEIS VON SEPSIS
DÉTECTION DE SEPSIE

(30) Priority: 24.03.2016 GB 201605110
(43) Date of publication of application: 30.01.2019
(60) Divisional application: 20169092.2
(73) Proprietor: MOLOGIC LTD., Thurleigh, Bedfordshire MK44 2YP (GB)
(72) Inventor: DAVIS, Paul, Thurleigh Bedfordshire MK44 2YP (GB); PAREKH, Gita, Thurleigh Bedfordshire MK44 2YP (GB); DUNSTON, Chris, Thurleigh Bedfordshire MK44 2YP (GB)
(74) Representative: Boult Wade Tennant LLP
(86) International application number: PCT/GB2017/050847
(87) International publication number: WO 2017/163087

(56) References cited:
- WO-A1-2009/062948
- WO-A2-2007/041623
- WO-A2-2013/083781
- US-A1- 2009 004 755
- HIROSHI TANAKA ET AL: "Role of Granulocyte Elastase in Tissue Injury in Patients with Septic Shock Complicated by Multiple-organ Failure :", ANNALS OF SURGERY., vol. 213, no. 1, 1 January 1991 (1991-01-01), pages 81-85, XP055374943, US ISSN: 0003-4932, DOI: 10.1097/00000658-199101000-00014
- ALCARAZ A ET AL: "Activation of the protein C pathway in acute sepsis", THROMBOSIS RESEARCH, TARRYTOWN, NY, US, vol. 79, no. 1, 1 July 1995 (1995-07-01), pages 83-93, XP002471126, ISSN: 0049-3848, DOI: 10.1016/0049-3848(95)00093-7

## Description

### FIELD OF THE INVENTION

The present invention relates to the identification of markers that predict or diagnose systemic inflammatory response syndrome (SIRS) and sepsis. In particular, the invention relates to monitoring subjects subjected to a surgical procedure for SIRS and sepsis based upon measuring markers at multiple time points.

### BACKGROUND TO THE INVENTION

Sepsis is a major and increasing public health concern. The clinical presentation of sepsis is such that it is difficult to diagnose and, in particular, to distinguish from Systemic Inflammatory Response Syndrome (SIRS). A number of diagnostic tools (i.e. lactate, blood culture, WBC counts, CRP etc.) are available to assist in the identification of sepsis but these lack sensitivity and/or specificity. The pressures to initiate treatment of sepsis as early as possible (because mortality risk increases for every hour of delay before initiation of therapy) can lead to the inappropriate use of broad spectrum antibiotics before confirmation of diagnosis, which can take up to 72 hours. Rapid decision making is critical to the appropriate treatment of the patient and the reduction of morbidity and mortality. Incorrect prescription of antibiotics fuels antibiotic resistance and in many cases causes severe adverse drug reactions. WO2007/041623 discloses methods and compositions for diagnosis and/or prognosis in SIRS. Tanaka et al. (Ann Surg. 1991 Jan;213(1):81-5) and Alcaraz et al. (Thromb Res. 1995 Jul 1 ;79(1):83-93) describe studies relating to biomarkers and sepsis.

### DESCRIPTION OF THE INVENTION

There is a need for a more sensitive and specific assay for sepsis that could be deployed in near patient settings or at the point of care. The present inventors have identified combinations of markers that are effective in predicting and diagnosing SIRS and sepsis.

Accordingly, in a first aspect, the invention provides a method for predicting or diagnosing systemic inflammatory response syndrome (SIRS) and/or sepsis in a subject, the method comprising determining levels of at least CCL23 (chemokine (C-C motif) ligand 23), A1AT (alpha-1 antitrypsin) andCRP (C-reactive protein), optionally together with at least one or more of PLA2 (phospholipase A2), sICAM (soluble Intercellular Adhesion Molecule 1), IL-6 (interleukin-6), procalcitonin, , MMP8 (Matrix Metalloproteinase-8), TNFalpha (Tumour Necrosis Factor alpha), AcPGP (N-acetyl Proline-Glycine-Proline), enzymatic MMP activity, TIMP1 (Tissue Inhibitor of Metalloproteinase 1), sRAGE (soluble form of Receptor for Advanced Glycated End-products) and desmosine in a sample taken from the subject, wherein the combined levels of the at least three markers are used to predict or diagnose SIRS and/or sepsis.

It should be noted that throughout the specification the term "comprising" is intended to represent open-ended (i.e. including) language. However, for the avoidance of doubt, wherever the term "comprising" is used it is envisaged that the corresponding feature may be limited to that specified (i.e. consisting) as necessary.

The methods may be applied to patients who are potentially at high risk of developing sepsis. They may for example be applied to routinely test patients admitted to hospital, or to patients in the intensive care unit. The patients may be immunocompromised patients. The methods may be repeated at regular intervals in relation to the same patient, in particular to monitor and predict sepsis (including its development from SIRS).

SIRS is an inflammatory state affecting the whole body. The four SIRS criteria are (Bone et al. Crit Care Med. 1992; 20(6)864-874):
1. Temperature >38°C or <36°C
2. Heart rate >90/min
3. Respiratory rate >20/min or PaCO₂ <32 mm Hg (4.3 kPa)
4. White blood cell count >12000/mm³ or <4000/mm³ or >10% immature bands When two or more of these criteria are met patients may be diagnosed with SIRS.

Sepsis presents as a syndrome with physiological, pathological and biochemical abnormalities caused by infection. As with many conditions the definition of sepsis has been updated over time. Sepsis may be defined as infection with at least 2 SIRS criteria. Sepsis may also be defined as SIRS in the presence of a confirmed or suspected infection. A new definition of sepsis as a life-threatening organ dysfunction caused by a dysregulated host response to infection has been proposed (Singer et al. JAMA 2016; 315(8):801-810). The present invention is considered to be equally applicable to the evolving definitions of sepsis. The present invention is also applicable to identifying infection in a SIRS patient, irrespective of sepsis-related organ dysfunction assessment (SOFA) score. Thus, sepsis may be distinguished from SIRS.

SIRS and sepsis are commonly developed after surgery. The present inventors have shown that the markers described herein can be used for the surveillance of patients undergoing surgery. The combinations of markers described herein have been shown by the inventors to predict sepsis and to allow sepsis and SIRS to be distinguished. This will enable improved management of surgical patients resulting in improved patient outcomes and substantial health-economic benefits.

Thus, the invention provides a method for monitoring a subject at risk of developing sepsis, the method comprising determining levels of at least CCL23, A1AT and CRP in samples taken from the subject at multiple time points, wherein the monitored levels of CCL23, A1AT and CRP are used to predict or diagnose SIRS and/or sepsis.

More specifically, the invention also provides a method for monitoring a subject subjected to a surgical procedure, the method comprising determining levels of at least CCL23, A1AT and CRP in samples taken from the subject at multiple time points including a first sample taken from the subject prior to the surgery to provide baseline levels of the markers and at least one further sample taken from the subject following surgery, wherein the monitored levels of CCL23, A1AT and CRP are used to predict or diagnose SIRS and/or sepsis following surgery.

In certain embodiments the methods of the invention discriminate SIRS from sepsis. The method may provide a prediction of impending sepsis. For example, the method may predict impending sepsis 1, 2, or 3 days before sepsis develops or would develop in the absence of treatment. The prediction of impending sepsis may result in treatment of the infection. In certain embodiments, the subject is not treated (e.g. with an antibiotic) unless and until impending sepsis is predicted or sepsis is diagnosed (according to the methods of the invention). In this way unnecessary treatment is avoided.

In certain embodiments according to all aspects of the invention the markers are at least CCL23, A1AT and CRP, optionally together with at least one or more of PLA2, sICAM, IL-6, procalcitonin, MMP8, TNFalpha, AcPGP, enzymatic MMP activity, TIMP1, sRAGE and desmosine. The PLA2 may be of group IIA i.e. PLA2G2A.

In some embodiments, the methods of the invention are performed on a subject with SIRS. In such embodiments, the methods may be used to identify an infection in the subject. Markers for use in such methods are at least CCL23, A1AT and CRP, optionally together with at least 1, 2, 3, 4, 5, 6, or all of desmosine, TNF, IL-6, AcPGP, PLA2g2A, , sICAM (optionally measured by ELISA), sICAM/VCAM-1 (optionally measured by lateral flow). More specifically, the markers may comprise all of CCL23, A1AT, sICAM (optionally measured by ELISA), sICAM/VCAM-1 (optionally measured by lateral flow) and CRP. The inventors have discovered that their lateral flow format for detecting sICAM1, described in further detail herein, relies on antibody binding to tandem N-terminal Ig-like domains (D1 and D2) of sICAM (CD54) and VCAM-1 (CD106) markers. sICAM specific measurements are also possible, using different antibodies (e.g. the ELISA formats discussed in greater detail herein). Thus, the methods of the invention may detect both sICAM individually and a combination of sICAM and VCAM-1. The combination is referred to herein as a single marker "sICAM/VCAM-1 ". Both measurements have been shown to give diagnostically useful information. Where "sICAM" is mentioned without further reference to "sICAM/VCAM-1" it is intended that either sICAM alone and/or sICAM/VCAM-1 may be measured.

For general monitoring, at least 2, 3, 4, 5, 6, 7, 8, 9, 10, 11, 12, 13 or 14 or more samples may be taken from the subject at different times and the levels of the at least three markers is determined. The samples may be taken every 1, 2, 3, 4, 5, 6 days or weekly for example. This depends on the nature of the subject and the perceived risk of sepsis. For example, samples may be taken more frequently, such as daily for patients in intensive case. They may be taken less frequently for immunocompromised patients, where the risks associated with taking a blood sample have to be balanced. The frequency of sampling may vary depending on the results of the previous test. Thus, more frequent testing may be undertaken where altered levels of the multiple markers are detected but that do not yet predict or diagnosis sepsis and/or SIRS. Conversely, detection of stable levels in multiple samples may lead to a reduction in frequency of testing, or cessation.

In relation to post-surgical monitoring, at least 2, 3, 4, 5, 6, 7, 8, 9, 10, 11, 12, 13 or 14 or more samples are taken from the subject at different times after surgery and the levels of the at least three markers is determined. The samples may be taken every 1, 2, 3, 4, 5, 6, 12 or 24 hours. For example, samples may be taken daily. The frequency of sampling may vary depending on the time post-surgery. For example, for the first 24 hours following surgery samples may be taken hourly. After the first 24 hours the frequency of sampling may decrease, for example to every 6, 12 or 24 hours, particularly where no indication of sepsis has been detected in the first 24 hours. Sampling may continue for 1, 2, 3, 4, 5, 6, 7, 8, 9, 10, 11, 12, 13 or 14 days following surgery. After 14 days the occurrence of sepsis or SIRS as a result of surgery is very low.

The methods of the invention may be implemented in a composition of matter which may take the form of a system or test kit. Such a system or test kit is preferably suitable for use, and ideally very easy to use (e.g. with a readily interpreted output), by clinicians in a healthcare setting such as a hospital.

Accordingly, the invention also provides a system or test kit for predicting or diagnosing systemic inflammatory response syndrome (SIRS) and/or sepsis in a subject, comprising:
a. One or more testing devices for determining levels of at least CCL23, A1AT and CRP optionally together with at least one or more of PLA2, , sICAM, IL-6, procalcitonin, , MMP8, TNFalpha, AcPGP, enzymatic MMP activity, TIMP1, sRAGE and desmosine in a sample
b. A processor; and
c. A storage medium comprising a computer application that, when executed by the processor, is configured to:
   i. Access and/or calculate the determined levels of each marker in the sample on the one or more testing devices
   ii. Calculate a test score from the levels of the markers in the sample that predicts or diagnoses SIRS and/or sepsis; and
   iii. Output from the processor the predicted or diagnostic result for the subject.

Also provided is a system or test kit for monitoring a subject, comprising:
a. One or more testing devices for determining levels of at least CCL23, A1AT and CRP optionally together with at least one or more of PLA2, sICAM, IL-6, procalcitonin, MMP8, TNFalpha, AcPGP, enzymatic MMP activity, TIMP1, sRAGE and desmosine TNFalpha in a sample at multiple time points
b. A processor; and
c. A storage medium comprising a computer application that, when executed by the processor, is configured to:
   i. Access and/or calculate the determined levels of each marker in the sample on the one or more testing devices
   ii. Calculate a test score from the levels of the markers in the sample, optionally including a comparison of the levels with those taken at one or more earlier time points, to thereby predict or diagnose SIRS and/or sepsis; and
   iii. Output from the processor the predicted or diagnostic result for the subject.

Also provided is a system or test kit for monitoring a subject subjected to a surgical procedure, comprising:
a. One or more testing devices for determining levels of at CCL23, A1AT and CRP optionally together with at least one or more of PLA2, sICAM, IL-6, procalcitonin, MMP8, TNFalpha, AcPGP, enzymatic MMP activity, TIMP1, sRAGE and desmosine in a sample at multiple time points including a first sample taken from the subject prior to the surgery to provide baseline levels of the markers and at least one further sample taken from the subject following surgery
b. A processor; and
c. A storage medium comprising a computer application that, when executed by the processor, is configured to:
   i. Access and/or calculate the determined levels of each marker in the sample on the one or more testing devices
   ii. Calculate a test score from the levels of the markers in the sample by comparing the levels with those taken at one or more earlier time points to thereby predict or diagnose SIRS and/or sepsis; and
   iii. Output from the processor the predicted or diagnostic result for the subject.

Also disclosed is a corresponding computer application for use in the system or test kit.

The invention provides for patient selection for therapy and, thus, avoids unnecessary treatment with antibiotics. Incorrect use of antibiotics fuels antibiotic resistance and can cause severe adverse drug reactions.

Accordingly, the invention also relates to a method of selecting a subject for treatment with an antibiotic comprising performing a method described herein and selecting the subject for treatment where sepsis is predicted or diagnosed.

Described here is a method of predicting responsiveness of a subject to treatment with an antibiotic comprising performing a method described herein and predicting responsiveness of the subject to treatment where sepsis is predicted or diagnosed.

Described herein is a method of treating sepsis comprising administering an antibiotic to the subject suffering from sepsis, wherein the subject has been selected for treatment by performing a method described herein.

Described herein is a method of treating sepsis comprising administering an antibiotic to the subject suffering from sepsis, wherein the subject displays, in a sample, an altered level of at least one, two or three markers selected from PLA2, CRP, CCL23, sICAM, IL-6, procalcitonin, A1AT, MMP8, TNFalpha, AcPGP, enzymatic MMP activity, TIMP1, sRAGE and desmosine.

In yet a further aspect, the present invention provides an antibiotic for use in a method of treating sepsis, wherein the subject has been selected for treatment by performing the method described herein.

According to a further aspect of the invention there is provided an antibiotic for use in a method of treating sepsis, wherein the subject displays, in a sample, an altered level of at least CCL23, A1AT and CRP optionally together with an altered level of at least one of PLA2, , sICAM, IL-6, procalcitonin, MMP8, TNFalpha, AcPGP, enzymatic MMP activity, TIMP1, sRAGE and desmosine.

In certain embodiments the antibiotic is a broad spectrum antibiotic. This is particularly useful if sepsis is diagnosed but where the origin of the infection has not yet been characterised. Once sepsis has been diagnosed, the infection may be characterised so as to allow more targeted therapy (e.g. as bacterial, which may be gram-positive or gram-negative), or fungal. The antibiotic may be selected from an aminoglycoside, a cephalosporin, a glycopeptide, a penicillin, a quinolone, aztreonam, clindamycin, imipenem-cilastin, linezolid, metronidazole, rifampin and an antifungal. Thus, combinations of broad spectrum antibiotics and more focussed therapies may be employed as part of the methods described herein.

The invention also provides a testing device, testing kit or testing composition of matter comprising:
a. A sample receiving zone to which a sample from a subject is added
b. A conjugate zone comprising at least three labelled binding reagents, each of which specifically binds to one of CCL23, A1AT and CRP, optionally together with at least one further labelled binding reagent which specifically binds to PLA2, sICAM, IL-6, procalcitonin, MMP8, TNFalpha, AcPGP, enzymatic MMP activity, TIMP1, sRAGE and desmosine
c. A solid support defining a liquid flow path for the sample and comprising corresponding test lines for each of the at least three markers, each test line comprising an immobilised further binding reagent that also specifically binds to one of CCL23, A1AT and CRP thereby immobilising the marker at the test line to produce a signal via the labelled binding reagent also specifically bound to the marker.

The testing device, testing kit or testing composition of matter may further comprise:
d. At least one labelled control binding reagent that binds to a binding partner immobilised at a control line downstream of the test lines for the at least one, two or three markers and thus confirms that the test has completed successfully.

The testing device, testing kit or testing composition of matter may further comprise:
e. An absorbent material downstream of the test (and control, where present) lines to absorb excess sample.

In specific embodiments the sample receiving zone is proportioned to receive between 10 and 100 µl of serum, such as around 80 µl of serum

In specific embodiments, the solid support comprises a chromatographic medium or a capillary flow device. The invention may be provided in a test strip format in some embodiments.

The testing device, testing kit or testing composition of matter may further comprise a reader to quantify levels of the markers at the respective test lines. The reader may further comprise:
a. A processor; and
b. A storage medium comprising a computer application that, when executed by the processor, is configured to:
   i. Access and/or calculate the determined levels of each marker in the sample on the one or more testing devices
   ii. Calculate a test score from the levels of the markers in the sample that predicts or diagnoses SIRS and/or sepsis; and
   iii. Output from the processor the predicted or diagnostic result for the subject.

In other embodiments the reader further comprises:
a. A processor; and
d. A storage medium comprising a computer application that, when executed by the processor, is configured to:
   i. Access and/or calculate the determined levels of each marker in the sample on the one or more testing devices
   ii. Calculate a test score from the levels of the markers in the sample by comparing the levels with those taken at one or more earlier time points to thereby predict or diagnose SIRS and/or sepsis; and
   iii. Output from the processor the predicted or diagnostic result for the subject.

According to all aspects of the invention the levels of at least CCL23, A1AT and CRP are determined.

As discussed above, markers of the invention may be measured in samples from a subject with SIRS. In such embodiments, the markers may be used to identify an infection in the subject. Markers methods are at least CCL23, A1AT and CRP, optionally together with at least one or more of desmosine, TNF, IL-6, AcPGP, PLA2g2A, , sICAM (optionally measured by ELISA), and sICAM/VCAM-1 (optionally measured by lateral flow). More specifically, the markers may be all of CCL23, A1AT, sICAM (optionally measured by ELISA), sICAM/VCAM-1 (optionally measured by lateral flow) and CRP. The markers may include both sICAM individually and a combination of sICAM and VCAM-1. The combination is referred to herein as a single marker "sICAM/VCAM-1". Both measurements have been shown to give diagnostically useful information. Where "sICAM" is mentioned without further reference to "sICAM/VCAM-1" it is intended that either sICAM alone and/or sICAM/VCAM-1 may be measured.

Markers that have been shown to be particularly useful individually in predicting or diagnosing sepsis include CRP, PLA2 and sICAM. In some embodiments, the at least three markers comprise sICAM, CCL23, A1AT, CRP, IL-6, PLA2G2A and TNFalpha.

Specific marker combinations described herein include CRP, sICAM and TNFalpha. In certain embodiments the levels of at least four markers are determined. Thus, a further specific marker combination described herein is PLA2, IL-6, CRP and TNFalpha. A further specific marker combination which may be useful in the invention is sICAM, CCL23, A1AT, CRP, IL-6 and TNFalpha. A still further marker combination described herein comprises or is CRP, sICAM and IL-6. In certain embodiments sICAM is measured by an ELISA and a lateral flow assay (i.e. both sICAM and sICAM/VCAM-1 may be measured).

The markers of the invention must include at least CCL23, A1AT and CRP and may optionally also comprise one or more, up to all, of IL-6, TIMP1, sICAM-1 and PLA2. IL-6, TIMP1, sICAM-1 and PLA2 are early markers of sepsis. They may be more useful than CRP in early detection or prediction of sepsis as shown herein. Accordingly, these markers will usefully be measured rapidly following an indication that a subject may have sepsis, for example in testing high risk patients such as immunocompromised patients and those in intensive care and/or following surgery. For example, these markers may be measured within 6 hours of an indication of sepsis or following surgery. In certain embodiments these markers are measured every 1, 2, 3, 4, 5 or 6 hours for up to 14 days following the indication of sepsis or the surgery.

By "altered" levels is meant an increase or decrease compared to threshold levels. Threshold levels may be defined from population studies or be specific to the individual. Where specific to the individual, the levels may reflect those taken at an earlier time point. In some embodiments, that earlier time point is prior to surgery. Threshold levels may be set with reference to a training data set comprising samples defined in relation to sepsis and/or SIRS status. Because the threshold levels vary according to the measuring technique adopted they are not stated as fixed values but can be implemented according to the present invention by one skilled in the art. Example thresholds are set forth herein for information purposes.

According to the invention, the following markers are typically increased in level to predict or diagnose sepsis: PLA2, CRP, CCL23, sICAM, IL-6, procalcitonin, A1AT, MMP8, TNFalpha, a substrate of enzymatic MMP activity, TIMP1, desmosine and sRAGE. According to the invention, AcPGP may be decreased in level to predict or diagnose sepsis.

The subject is a mammalian subject, typically a human.

It should be noted that the invention is performed *in vitro* based upon isolated whole blood, plasma or serum samples. The methods of the invention do not include steps of obtaining a sample for testing. The sample may be obtained simply, for example by finger prick. This is particularly advantageous from a compliance perspective and provides adequate volume for the various testing devices described herein, in particular the lateral flow formats in which multiple markers are determined from a single sample. Similarly, in some embodiments, the systems and test kits include suitable vessels for receiving a sample. The container may be coloured to protect any light sensitive analytes.

There are various known techniques by which marker levels may be measured. Thus, by marker levels is meant the level of expression and/or activity and/or amount and/or concentration of the marker. Expression levels of the markers may be measured in blood. Expression levels may correlate with activity and can thus be used as a surrogate of activity. Expression levels may be measured at the level of protein or mRNA according to any suitable method. Protein modifications, such as glycosylation may also be relevant and can be measured by any suitable method. Many such methods are well known in the art and include use of mass spectrometry (e.g. MALDI-TOF mass spectrometry). MicroRNAs may also be measured in samples as post-transcriptional regulators of gene expression. A platform such as that offered by Exiqon may be utilised to provide high-throughput microRNA profiling. Such platforms may be array and/or PCR based.

The expression level and/or amount and/or concentration of a marker (e.g. a protein) may rely upon a binding reagent such as an antibody or aptamer that binds specifically to the marker of interest (e.g. protein). The antibody may be of monoclonal or polyclonal origin. Fragments and derivative antibodies may also be utilised, to include without limitation Fab fragments, ScFv, single domain antibodies, nanoantibodies, heavy chain antibodies, aptamers etc. which retain specific binding function and these are included in the definition of "antibody". Such antibodies are useful in the methods of the invention. They may be used to measure the level of a particular marker (e.g. protein, or in some instances one or more specific isoforms of a protein. The skilled person is well able to identify epitopes that permit specific isoforms to be discriminated from one another).

Methods for generating specific antibodies are known to those skilled in the art. Antibodies may be of human or non-human origin (e.g. rodent, such as rat or mouse) and be humanized etc. according to known techniques (Jones et al., Nature (1986) May 29-Jun. 4;321(6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In certain embodiments the expression level and/or amount and/or concentration of a marker is determined using an antibody or aptamer conjugated to a label. By label is meant a component that permits detection, directly or indirectly. For example, the label may be an enzyme, optionally a peroxidase, or a fluorophore. Gold labels may be utilised, e.g. in the form of colloidal gold.

A label is an example of a detection agent. By detection agent is meant an agent that may be used to assist in the detection of the antibody-marker (e.g. protein) complex. Where the antibody is conjugated to an enzyme the detection agent may comprise a chemical composition such that the enzyme catalyses a chemical reaction to produce a detectable product. The products of reactions catalysed by appropriate enzymes can be, without limitation, fluorescent, luminescent, or radioactive or they may absorb or reflect visible or ultraviolet light. Examples of detectors suitable for detecting such detectable labels include, without limitation, x-ray film, radioactivity counters, scintillation counters, spectrophotometers, colorimeters, fluorometers, luminometers, photodetectors and densitometers. In certain embodiments the detection agent may comprise a secondary antibody. The expression level is then determined using an unlabelled primary antibody that binds to the target protein and a secondary antibody conjugated to a label, wherein the secondary antibody binds to the primary antibody.

Additional techniques for determining expression level at the level of protein and/or the amount and/or concentration of a marker include, for example, Western blot, immunoprecipitation, immunocytochemistry, mass spectrometry, ELISA and others (see ImmunoAssay: A Practical Guide, edited by Brian Law, published by Taylor & Francis, Ltd., 2005 edition). To improve specificity and sensitivity of an assay method based on immunoreactivity, monoclonal antibodies are often used because of their specific epitope recognition. Polyclonal antibodies have also been successfully used in various immunoassays because of their increased affinity for the target as compared to monoclonal antibodies. Levels of protein may be detected using a lateral flow assay in some embodiments (discussed in further detail herein).

Measuring mRNA in a biological sample may be used as a surrogate for detection of the level of the corresponding protein in the urine sample. Thus, the expression level of any of the relevant markers described herein can also be detected by detecting the appropriate RNA.

Accordingly, in specific embodiments the expression level is determined by microarray, northern blotting, or nucleic acid amplification. Nucleic acid amplification includes PCR and all variants thereof such as real-time and end point methods and qPCR. Other nucleic acid amplification techniques are well known in the art, and include methods such as NASBA, 3SR and Transcription Mediated Amplification (TMA). Other suitable amplification methods include the ligase chain reaction (LCR), selective amplification of target polynucleotide sequences (US Patent No. 6,410,276), consensus sequence primed polymerase chain reaction (US Patent No 4,437,975), arbitrarily primed polymerase chain reaction (WO 90/06995), invader technology, strand displacement technology, recombinase polymerase amplification (RPA), nicking enzyme amplification reaction (NEAR) and nick displacement amplification (WO 2004/067726). This list is not intended to be exhaustive; any nucleic acid amplification technique may be used provided the appropriate nucleic acid product is specifically amplified. Design of suitable primers and/or probes is within the capability of one skilled in the art. Various primer design tools are freely available to assist in this process such as the NCBI Primer-BLAST tool. Primers and/or probes may be at least 15, 16, 17, 18, 19, 20, 21, 22, 23, 24 or 25 (or more) nucleotides in length. mRNA expression levels may be measured by reverse transcription quantitative polymerase chain reaction (RT-PCR followed with qPCR). RT-PCR is used to create a cDNA from the mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

RNA expression may be determined by hybridization of RNA to a set of probes. The probes may be arranged in an array. Microarray platforms include those manufactured by companies such as Affymetrix, Illumina and Agilent. RNA expression may also be measured using next generation sequencing methods, such as RNA-seq. This may require conversion of RNA to cDNA or may be a direct RNA sequencing methodology.

Similarly, activity, such as enzymatic activity, may be measured in the sample. Enzymatic activity may be measured for example by detecting processing of a substrate, which may be labelled, in the sample. For example, the assay may be a fluorogenic substrate assay. Enzyme activity may be detected using a suitable lateral flow assay. Examples of suitable assay formats include the assays set forth in International Patent Applications WO2009/024805, WO2009/063208, WO2007/128980, WO2007/096642, WO2007/096637, WO2013/156794 and WO2013/156795.

Some examples of suitable assay formats useful for particular markers are outlined in the table below:

| **Marker** | **Assay Type** | **Format** | **Supplier** |
|---|---|---|---|
| CRP | ELISA | Sandwich | R&D Systems |
| CRP | Lateral flow | Sandwich | Mologic |
| sICAM1 | ELISA | Sandwich | R&D Systems |
| sICAM1 | Lateral flow | Sandwich | Mologic |
| CRP/sICAM1 Duplex | Lateral flow | Sandwich | Mologic |
| IL-6 | ELISA | Sandwich | R&D Systems |
| IL-6 | Lateral flow | Sandwich | Mologic |
| PLA2 | ELISA | Sandwich | Mologic |
| PLA2 | Lateral flow | Sandwich | Mologic |
| IL-6/PLA2 Duplex | Lateral flow | Sandwich | Mologic |
| A1AT | ELISA | Sandwich | Mologic |
| A1AT | Lateral flow | Sandwich | Mologic |
| TNFα | ELISA | Sandwich | R&D Systems |
| TNFα | Lateral flow | Sandwich | Mologic |
| CCL23 | ELISA | Sandwich | R&D Systems |
| Ac-PGPv3 | ELISA | Competition | Mologic |
| MMP activity | Substrate | Fluorescent | Mologic |
| Creatinine | Substrate | Colourmetric | R&D Systems |
| Desmosine | ELISA | Competition | Mologic |
| MMP8 | ELISA | Sandwich | R&D Systems |
| Procalcitonin | ELISA | Sandwich | Abcam |
| Procalcitonin | ELISA | Sandwich | Mologic |

Thus, it can be readily seen that ELISA and lateral flow formats are particularly applicable to the present invention. In some embodiments, double antibody sandwich immunoassay (DASIA), also called sandwich ELISA, or immunometric assay formats may be employed.

The inventors have devised various assays for determining the levels of the markers described herein.

One marker useful in the present invention is CCL23, a chemokine secreted by a variety of cell types, including monocytes, macrophages, and dendritic cells. It has been shown to be induced by the chemokines IL-4 and IL-13. According to the invention CCL23 may be detected by an immunoassay such as an ELISA (enzyme-linked immunosorbent assay). The method of the invention thus may include an ELISA for detecting CCL23 in a sample comprising:
(a) contacting the sample with an immunocapture surface (such as an ELISA plate) on which is immobilised a reagent (such as an antibody, as defined herein, one specific example being mouse anti-human CCL23) that specifically binds to CCL23 in the sample
(b) adding a further reagent (such as an antibody, as defined herein, one specific example being goat polyclonal CCL23 antibody) that specifically binds to CCL23 to the sample, which reagent is conjugated to a label (such as biotin)
(c) Adding an enzyme (such as horseradish peroxidase) conjugated to a binding partner for the label (for example streptavidin)
(d) removing reagent not bound to the immunocapture surface
(e) measuring the levels of enzyme activity at the immunocapture surface as an indication of the levels of CCL23 in the sample.

More specifically, the method of the invention may include an ELISA for detecting CCL23 in a sample comprising:
(a) contacting the sample with an immunocapture surface (such as an ELISA plate) on which is immobilised a reagent (such as an antibody, as defined herein, one specific example being mouse anti-human CCL23, catalogue number 841474 R&D systems) that specifically binds to CCL23 in the sample (for a defined time such as overnight)
(b) removing reagent not bound to the immunocapture surface with a wash step (wash buffer 50mM tris buffered saline pH8, supplemented with 0.1% (v/v) Tween 20)
(c) blocking unbound sites on the surface with the addition of a blocking solution (such as R&D diluent, catalogue number DY995 R&D systems) for a defined time such as 1 hour.
(d) removing reagent not bound to the immunocapture surface with a wash step (wash buffer 50mM tris buffered saline pH8, supplemented with 0.1% (v/v) Tween 20)
(e) addition of sample diluted 1 in 10 in sample diluent PBS, pH6.9, supplemented with 1% BSA incubated for a defined time such as 2 hours
(f) removing reagent not bound to the immunocapture surface with a wash step (wash buffer 50mM tris buffered saline pH8, supplemented with 0.1% (v/v) Tween 20)
(g) adding a further reagent (such as an antibody, as defined herein, one specific example being goat polyclonal CCL23 antibody catalogue number 841475 R&D systems) that specifically binds to CCL23 in the sample, which reagent is conjugated to a label (such as biotin) for a defined time such as 2 hours
(h) removing reagent not bound to the immunocapture surface with a wash step (wash buffer 50mM tris buffered saline pH8, supplemented with 0.1% (v/v) Tween 20)
(i) Adding an enzyme (such as horseradish peroxidase) conjugated to a binding partner for the label (for example streptavidin, catalogue number 890803 R&D systems) for a defined time such as 20 minutes
(j) Adding a substrate that initiates the colour change (such as OPD, catalogue number P9187 Sigma-Aldrich)
(k) measuring the levels of enzyme activity at the immunocapture surface as an indication of the levels of CCL23 in the sample with a specified sensitivity (such as 15.6pg/mL) and an assay range (such as 0.156-10ng/mL)

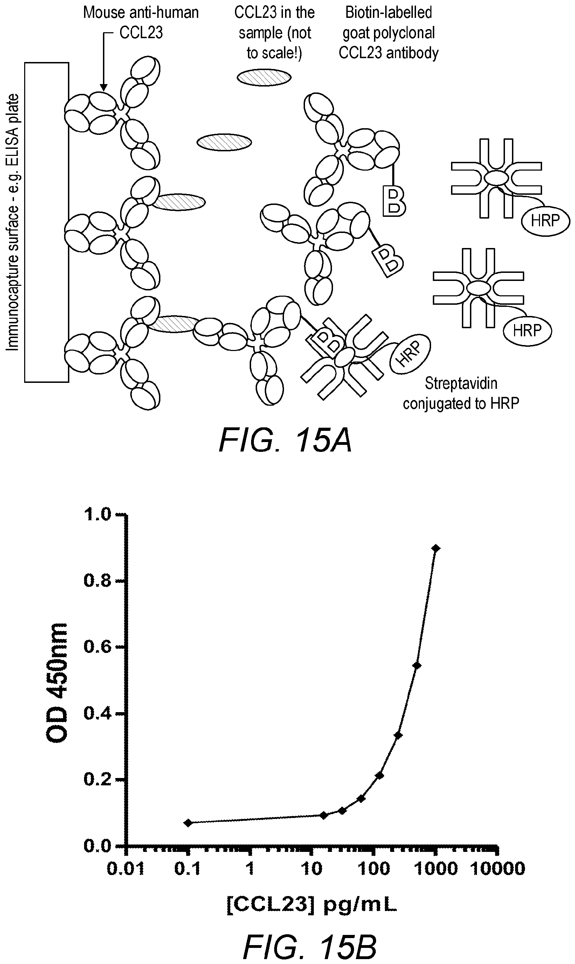

A schematic representation of a suitable assay format is shown in Figure 15A and a representative calibration curve for this assay is shown in Figure 15B.

CCL23 may be detected in a lateral flow format in other embodiments. A suitable assay may comprise steps of:
(a) contacting the sample with an immunocapture surface (such as an immunocapture line on nitrocellulose membrane) on which is immobilised a reagent (such as an antibody, as defined herein, one specific example being mouse anti-CCL23 Fab 01041-BSA conjugated immobilised at a concentration of 1mg/mL) that specifically binds to CCL23 in the sample
(b) addition of sample diluted 1 in 2 in sample diluent PBS, pH6.9, supplemented with 1% (v/v) Tween 20 + 1% BSA
(c) CCL23 in the sample complexes with a reagent present in conjugate pad (such as an antibody, as defined herein, one specific example being mouse anti-CCL23 Fab 01341 BSA conjugated antibody), which reagent is conjugated to a label (such as a gold particle, optionally a 40 nm gold particle at 15µg/mL in TES, pH7.5 buffer)
(d) measuring the levels of label at the immunocapture surface as an indication of the levels of CCL23 in the sample after a specific read time (such as 10 minutes) with an immunochromatographic reader.
(e) A control system such as an BSA biotin capture (immobilised at a concentration of 0.25mg/mL) and an anti-biotin gold conjugate (catalogue number BA.GAB, BBI at OD2) indicates that the test is complete.

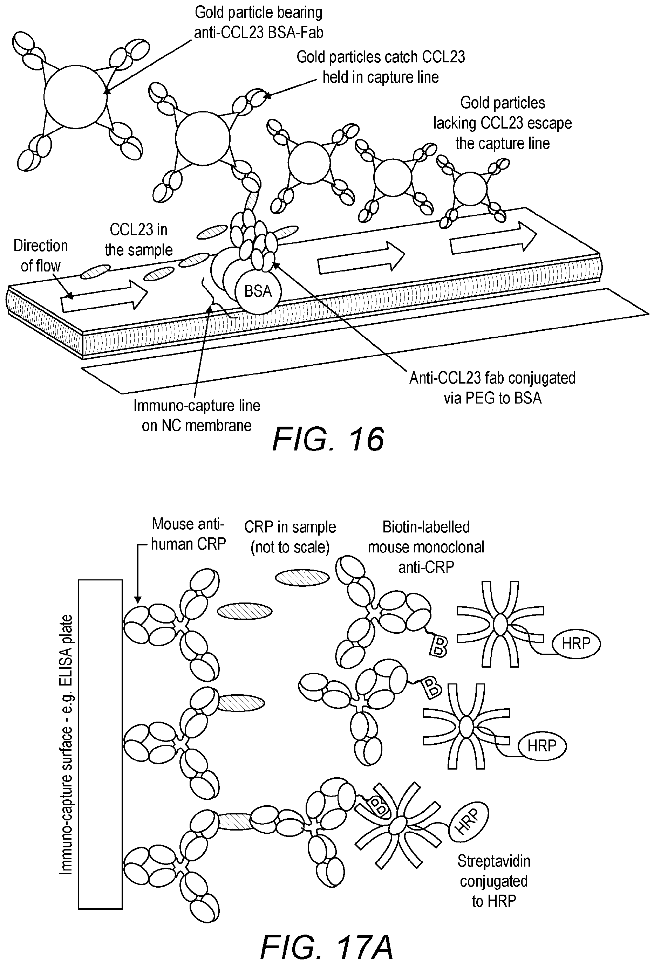

A suitable assay format is shown schematically in Figure 16.

Another marker useful in the present invention is CRP (C-reactive protein). CRP levels increase in patients with infection beyond levels found in non-sepsis patients with similar organ dysfunction (Castelli, Pognani, Meisner, Stuami, Bellomi, & Sgarbi, 2004). It is an acute phase protein, and is elevated in the event of tissue damage (surgery causes increased CRP levels), inflammation and infection. It is produced and secreted as a result of NFκβ cascade activation, and binds to pathogen cell surfaces to allow complement binding. According to the invention CRP may be detected by an immunoassay such as an ELISA (enzyme-linked immunosorbent assay). The method of the invention thus may include an ELISA for detecting CRP in a sample comprising:
(a) contacting the sample with an immunocapture surface (such as an ELISA plate) on which is immobilised a reagent (such as an antibody, as defined herein, one specific example being mouse anti-human CRP) that specifically binds to CRP in the sample
(b) adding a further reagent (such as an antibody, as defined herein, one specific example being mouse monoclonal anti-CRP antibody) that specifically binds to CRP to the sample, which reagent is conjugated to a label (such as biotin)
(c) Adding an enzyme (such as horseradish peroxidase) conjugated to a binding partner for the label (for example streptavidin)
(d) removing reagent not bound to the immunocapture surface
(e) measuring the levels of enzyme activity at the immunocapture surface as an indication of the levels of CRP in the sample.

More specifically, the method of the invention may include an ELISA for detecting CRP in a sample comprising:
(a) contacting the sample with an immunocapture surface (such as an ELISA plate) on which is immobilised a reagent (such as an antibody, as defined herein, one specific example being mouse anti-human CRP, catalogue number 842676 R&D systems) that specifically binds to CRP in the sample (for a defined time such as overnight)
(b) removing reagent not bound to the immunocapture surface with a wash step (wash buffer 50mM tris buffered saline pH8, supplemented with 0.1% (v/v) Tween 20)
(c) blocking unbound sites on the surface with the addition of a blocking solution (such as R&D diluent, catalogue number DY995 R&D systems) for a defined time such as 1 hour.
(d) removing reagent not bound to the immunocapture surface with a wash step (wash buffer 50mM tris buffered saline pH8, supplemented with 0.1% (v/v) Tween 20)
(e) addition of sample diluted 1 in 100K in sample diluent PBS, pH6.9, supplemented with 1% BSA incubated for a defined time such as 2 hours
(f) removing reagent not bound to the immunocapture surface with a wash step (wash buffer 50mM tris buffered saline pH8, supplemented with 0.1% (v/v) Tween 20)
(g) adding a further reagent (such as an antibody, as defined herein, one specific example being mouse anti CRP catalogue number 842677 R&D systems) that specifically binds to CRP in the sample, which reagent is conjugated to a label (such as biotin) for a defined time such as 2 hours
(h) removing reagent not bound to the immunocapture surface with a wash step (wash buffer 50mM tris buffered saline pH8, supplemented with 0.1% (v/v) Tween 20)
(i) Adding an enzyme (such as horseradish peroxidase) conjugated to a binding partner for the label (for example streptavidin, catalogue number 890803 R&D systems) for a defined time such as 20 minutes
(j) Adding a substrate that initiates the colour change (such as OPD, catalogue number P9187 Sigma-Aldrich)
(k) measuring the levels of enzyme activity at the immunocapture surface as an indication of the levels of CRP in the sample with a specified sensitivity (such as 15.6pg/mL) and an assay range (such as 1.56-100µg/mL)

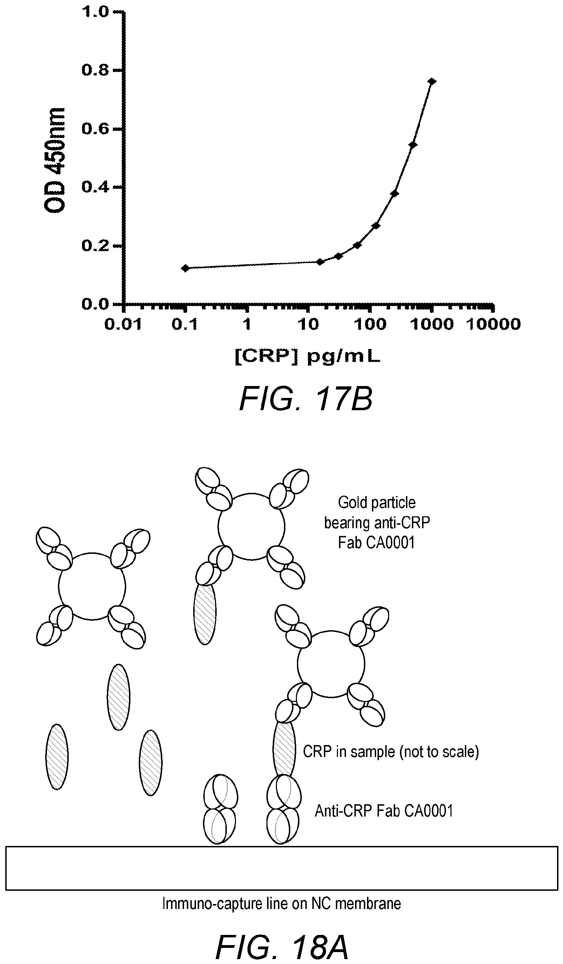

A schematic representation of a suitable assay format is shown in Figure 17A and a representative calibration curve for this assay is shown in Figure 17B.

CRP may be detected in a lateral flow format in other embodiments. The method of the invention thus may include a lateral flow assay for detecting CRP in a sample comprising:
(a) contacting the sample with an immunocapture surface (such as an immunocapture line on nitrocellulose membrane) on which is immobilised a reagent (such as an antibody, as defined herein, one specific example being anti-CRP Fab CA0001) that specifically binds to CRP in the sample
(b) adding a further reagent (such as an antibody, as defined herein, one specific example being anti-CRP Fab CA0001 antibody) that specifically binds to CRP to the sample, which reagent is conjugated to a label (such as a gold particle, optionally a 40 nm gold particle)
(c) measuring the levels of label at the immunocapture surface as an indication of the levels of CRP in the sample.

More specifically, CRP may be detected in a lateral flow format in other embodiments comprising:
(a) contacting the sample with an immunocapture surface (such as an immunocapture line on nitrocellulose membrane) on which is immobilised a reagent (such as an antibody, as defined herein, one specific example being mouse anti-CRP Fab CA0001 immobilised at a concentration of 1mg/mL) that specifically binds to CRP in the sample
(b) addition of sample diluted 1 in 30 in sample diluent PBS, pH6.9, supplemented with 1% (v/v) Tween 20 + 1% BSA
(c) CRP in the sample complexes with a reagent present in conjugate pad (such as an antibody, as defined herein, one specific example being mouse anti-CRP Fab CA0001 antibody), which reagent is conjugated to a label (such as a gold particle, optionally a 40 nm gold particle at 15µg/mL in TAPS, pH8.5 buffer)
(d) measuring the levels of label at the immunocapture surface as an indication of the levels of CRP in the sample after a specific read time (such as 10 minutes) with an immunochromatographic reader.
(e) A control system such as an BSA biotin capture (immobilised at a concentration of 0.25mg/mL) and an anti-biotin gold conjugate (catalogue number BA.GAB, BBI at OD2) indicates that the test is complete
(f) specified sensitivity (such as 3.9ng/mL) and an assay range (such as 19.5-1250µg/mL)

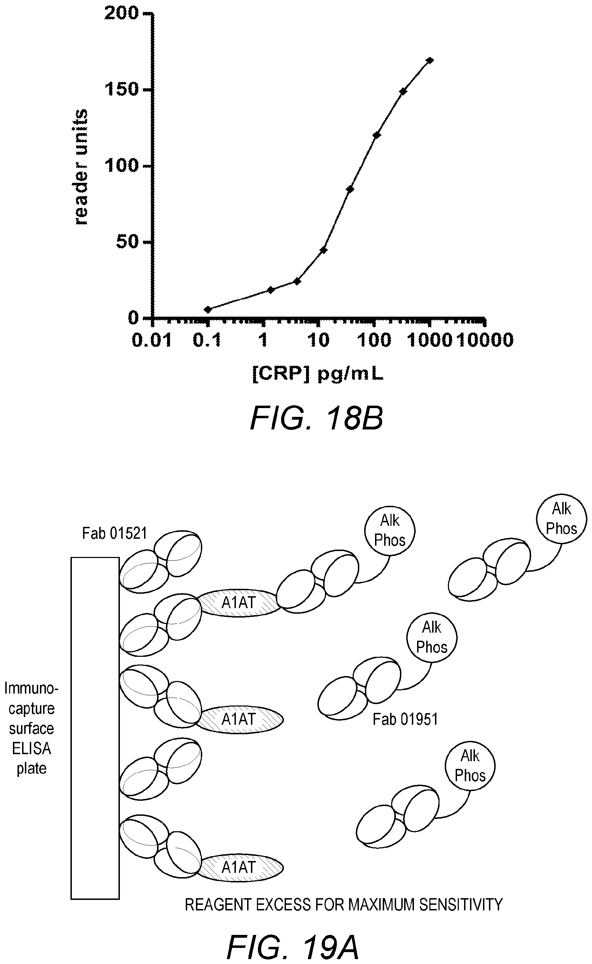

A schematic representation of a suitable assay format is shown in Figure 18A and a representative calibration curve for this assay is shown in Figure 18B. The following example components may be used:

| | Name | Cat No | Source | Concentration |
|---|---|---|---|---|
| Capture antibody | Anti CRP Fab | CA0001 | Alere San Diego | 1mg/mL |
| Detector antibody | Anti CRP Fab 20nm gold conjugated | CA0001 | Alere San Diego | 15µg/mL |
| Control line | BSA biotin | N/A | Mologic | 0.25mg/mL |
| Control gold | Anti-biotin gold | BA.GAB | BBI | OD2 |
| Standard | CRP | 140-11R | Lee Biosolutions | 3.9-250ng/mL |

Another marker useful in the present invention is α1AT (alpha 1 antitrypsin) a serine protease inhibitor which is synthesized in the liver, which is strongly associated with inhibition of neutrophil elastase. In this capacity it protects lung elastin from degradation by neutrophil elastase. α1AT deficiency potentially leads to COPD and liver disease. Further to its protective anti-protease activity, α1AT is involved in inflammation and the immune response in the lung. It has been shown to control the migration of neutrophils into the lung (Bergin, et al., 2010). α1AT was found to be significantly elevated in one study of neonatal sepsis (Suri, Thirupuram, & Sharma, 1991), and could be used for predicting sepsis severity. According to the invention α1AT may be detected by an immunoassay such as an ELISA (enzyme-linked immunosorbent assay). The method of the invention thus may include an ELISA for detecting α1AT in a sample comprising:
(a) contacting the sample with an immunocapture surface (such as an ELISA plate) on which is immobilised a reagent (such as an antibody, as defined herein, one specific example being Fab 01521) that specifically binds to α1AT in the sample
(b) adding a further reagent (such as an antibody, as defined herein, one specific example being Fab 01951) that specifically binds to α1AT to the sample, which reagent is conjugated to an enzyme (such as alkaline phosphatase)
(c) removing reagent not bound to the immunocapture surface
(d) measuring the levels of enzyme activity at the immunocapture surface as an indication of the levels of α1AT in the sample.

More specifically, it may include an ELISA for detecting A1AT in a sample comprising:
(a) contacting the sample with an immunocapture surface (such as an ELISA plate) on which is immobilised a reagent (such as an antibody, as defined herein, one specific example being mouse anti-human A1AT fab, catalogue number 01521 Alere San Diego) that specifically binds to A1AT in the sample (for a defined time such as overnight)
(b) removing reagent not bound to the immunocapture surface with a wash step (wash buffer 50mM tris buffered saline pH8, supplemented with 0.1% (v/v) Tween 20)
(c) blocking unbound sites on the surface with the addition of a blocking solution (such as PBS, pH6.9, supplemented with 1% BSA) for a defined time such as 1 hour.
(d) removing reagent not bound to the immunocapture surface with a wash step (wash buffer 50mM tris buffered saline pH8, supplemented with 0.1% (v/v) Tween 20)
(e) addition of sample diluted 1 in 200K in sample diluent PBS, pH6.9, supplemented with 1% (v/v) Tween 20 + 1% BSA, incubated for a defined time such as 1 hour
(f) removing reagent not bound to the immunocapture surface with a wash step (wash buffer 50mM tris buffered saline pH8, supplemented with 0.1% (v/v) Tween 20)
(g) adding a further reagent (such as an antibody, as defined herein, one specific example being mouse anti-human A1AT fab, catalogue number 01951 Alere San Diego) that specifically binds to A1AT in the sample, which reagent is conjugated to a label (such as alkaline phosphatase, catalogue number 702-0005, Innova bioscience) for a defined time such as 1 hour
(h) removing reagent not bound to the immunocapture surface with a wash step (wash buffer 50mM tris buffered saline pH8, supplemented with 0.1% (v/v) Tween 20)
(i) Adding a substrate that initiates the colour change (such as PNPP, catalogue number PNPP-001, Biopanda)
(j) measuring the levels of enzyme activity at the immunocapture surface as an indication of the levels of A1AT in the sample with a specified sensitivity (such as 260pg/mL) and an assay range (such as 0.28 - 260µg/mL)

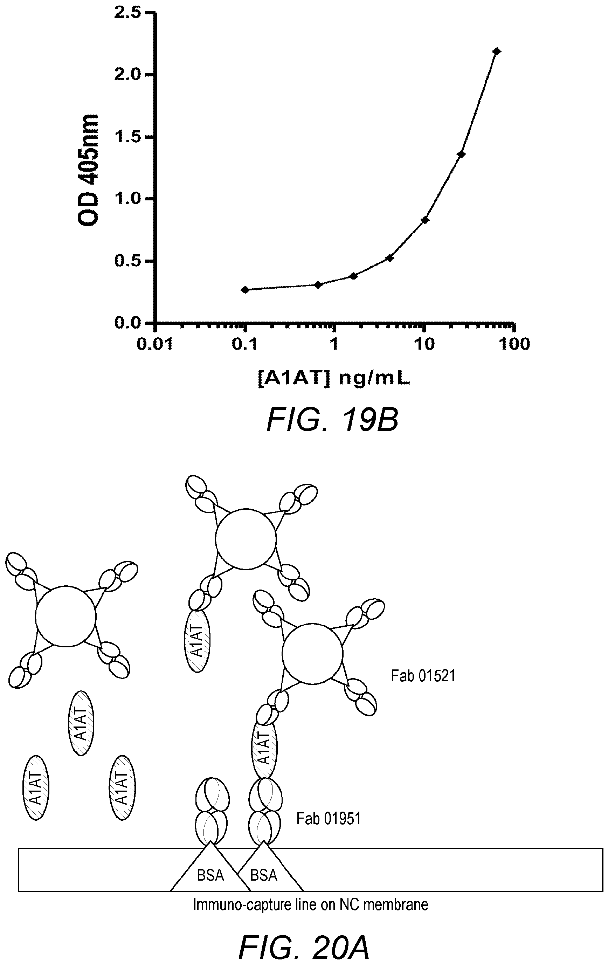

A schematic representation of a suitable assay format is shown in Figure 19A and a representative calibration curve for this assay is shown in Figure 19B.

The following example components may be used:

| | Name | Cat No | Source | Concentration |
|---|---|---|---|---|
| Capture antibody | Anti A1AT Fab | 01521 | Alere San Diego | 4µg/mL |
| Detector antibody | Anti A1AT Fab AP conjugated | 01951 | Alere San Diego | AP |
| Standard | A1AT | 178251 | Calbiochem | 0.26-160ng/mL |

α1AT may be detected in a lateral flow format in other embodiments. The method of the invention thus may include a lateral flow assay for detecting α1AT in a sample comprising:
(a) contacting the sample with an immunocapture surface (such as an immunocapture line on nitrocellulose membrane) on which is immobilised a reagent (such as an antibody, as defined herein, one specific example being Fab 01951) that specifically binds to α1AT in the sample
(b) adding a further reagent (such as an antibody, as defined herein, one specific example being Fab 01521) that specifically binds to α1AT to the sample, which reagent is conjugated to a label (such as a gold particle, optionally a 40 nm gold particle)
(c) measuring the levels of label at the immunocapture surface as an indication of the levels of α1AT in the sample.

More specifically, A1AT may be detected in a lateral flow format in other embodiments, comprising:
(a) contacting the sample with an immunocapture surface (such as an immunocapture line on nitrocellulose membrane) on which is immobilised a reagent (such as an antibody, as defined herein, one specific example being mouse anti-A1AT Fab 01951 Alere San Diego, immobilised at a concentration of 1 mg/mL) that specifically binds to A1AT in the sample
(b) addition of sample diluted 1 in 200K in sample diluent PBS, pH6.9, supplemented with 1% (v/v) Tween 20 + 1% BSA
(c) A1AT in the sample complexes with a reagent present in conjugate pad (such as an antibody, as defined herein, one specific example being mouse anti-A1AT Fab 01521 antibody, Alere San Diego), which reagent is conjugated to a label (such as a gold particle, optionally a 40 nm gold particle at 15µg/mL in Borate, pH9.3 buffer)
(d) measuring the levels of label at the immunocapture surface as an indication of the levels of A1AT in the sample after a specific read time (such as 10 minutes) with an immunochromatographic reader.
(e) A control system such as an BSA biotin capture (immobilised at a concentration of 0.25mg/mL) and an anti-biotin gold conjugate (catalogue number BA.GAB, BBI at OD2) indicates that the test is complete
(f) specified sensitivity (such as 260pg/mL) and an assay range (such as 0.26-160µg/mL)

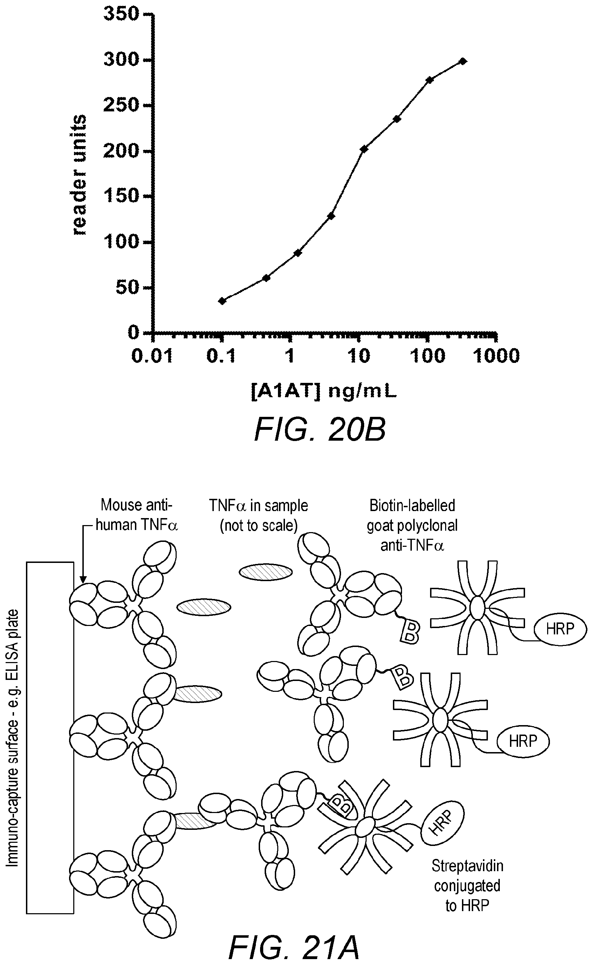

A schematic representation of a suitable assay format is shown in Figure 20A and a representative calibration curve for this assay is shown in Figure 20B.

The following example components may be used:

| | Name | Cat No | Source | Concentration |
|---|---|---|---|---|
| Capture antibody | Anti A1AT-BSA Fab | 01951 | Alere San Diego | 1mg/mL |
| Detector antibody | Anti A1AT Fab gold conjugated | 01521 | Alere San Diego | 15µg/mL |
| Control line | BSA biotin | N/A | Mologic | 0.25mg/mL |
| Control gold | Anti-biotin gold | BA.GAB | BBI | OD2 |
| Standard | A1AT | 178251 | Calbiochem | 0.44-360ng/mL |

Another marker useful in the present invention is TNFα, a cytokine and membrane bound protein involved in inflammation, apoptosis and lipid metabolism. It is shed from cell membranes by TACE/ADAM17 to yield TNFα cytokine. It is implicated in a variety of diseases, including autoimmune diseases, cancer and sepsis. It is its role in inflammation which implicates TNFα in sepsis, it is important in recruiting inflammatory cells to sites of infection. TNFα has been shown to be elevated in patients with sepsis, and has been shown to be higher in patients who died of sepsis than patients who recovered (Gogos, Drosou, Bassaris, & Skoutelis, 2000). Anti-TNFa is used as a treatment for Rheumatoid Arthritis and Crohn's disease. There has been work investigating the effectiveness of anti-TNFα as treatment for sepsis, and was found to reduce mortality in patients with severe sepsis, but not patients in septic shock or with elevated IL-6 levels (Lv, Han, Yi, Kwon, Dai, & Wang, 2014), though this contradicts other reports that blocking TNFα doesn't improve mortality rates in cases of sepsis. According to the invention TNFα may be detected by an immunoassay such as an ELISA (enzyme-linked immunosorbent assay). The method of the invention thus may include an ELISA for detecting TNFα in a sample comprising:
(a) contacting the sample with an immunocapture surface (such as an ELISA plate) on which is immobilised a reagent (such as an antibody, as defined herein, one specific example being mouse anti-human TNFα) that specifically binds to TNFα in the sample
(b) adding a further reagent (such as an antibody, as defined herein, one specific example being polyclonal TNFα antibody) that specifically binds to TNFα to the sample, which reagent is conjugated to a label (such as biotin)
(c) Adding an enzyme (such as horseradish peroxidase) conjugated to a binding partner for the label (for example streptavidin)
(d) removing reagent not bound to the immunocapture surface
(e) measuring the levels of enzyme activity at the immunocapture surface as an indication of the levels of TNFα in the sample.

More specifically, the method of the invention also may include an ELISA for detecting TNFα in a sample comprising:
(a) contacting the sample with an immunocapture surface (such as an ELISA plate) on which is immobilised a reagent (such as an antibody, as defined herein, one specific example being mouse anti-human TNFα, catalogue number 840119 R&D systems) that specifically binds to TNFα in the sample (for a defined time such as overnight)
(b) removing reagent not bound to the immunocapture surface with a wash step (wash buffer 50mM tris buffered saline pH8, supplemented with 0.1% (v/v) Tween 20)
(c) blocking unbound sites on the surface with the addition of a blocking solution (such as R&D diluent, catalogue number DY995 R&D systems) for a defined time such as 1 hour.
(d) removing reagent not bound to the immunocapture surface with a wash step (wash buffer 50mM tris buffered saline pH8, supplemented with 0.1% (v/v) Tween 20)
(e) addition of sample diluted 1 in 2 in sample diluent PBS, pH6.9, supplemented with 1% BSA incubated for a defined time such as 2 hours
(f) removing reagent not bound to the immunocapture surface with a wash step (wash buffer 50mM tris buffered saline pH8, supplemented with 0.1% (v/v) Tween 20)
(g) adding a further reagent (such as an antibody, as defined herein, one specific example being goat polyclonal TNFα antibody catalogue number 840121 R&D systems) that specifically binds to TNFα in the sample, which reagent is conjugated to a label (such as biotin) for a defined time such as 2 hours
(h) removing reagent not bound to the immunocapture surface with a wash step (wash buffer 50mM tris buffered saline pH8, supplemented with 0.1% (v/v) Tween 20)
(i) Adding an enzyme (such as horseradish peroxidase) conjugated to a binding partner for the label (for example streptavidin, catalogue number 893975 R&D systems) for a defined time such as 20 minutes
(j) Adding a substrate that initiates the colour change (such as OPD, catalogue number P9187 Sigma-Aldrich)
(k) measuring the levels of enzyme activity at the immunocapture surface as an indication of the levels of TNFα in the sample with a specified sensitivity (such as 15.6pg/mL) and an assay range (such as 31.2-2000pg/mL)

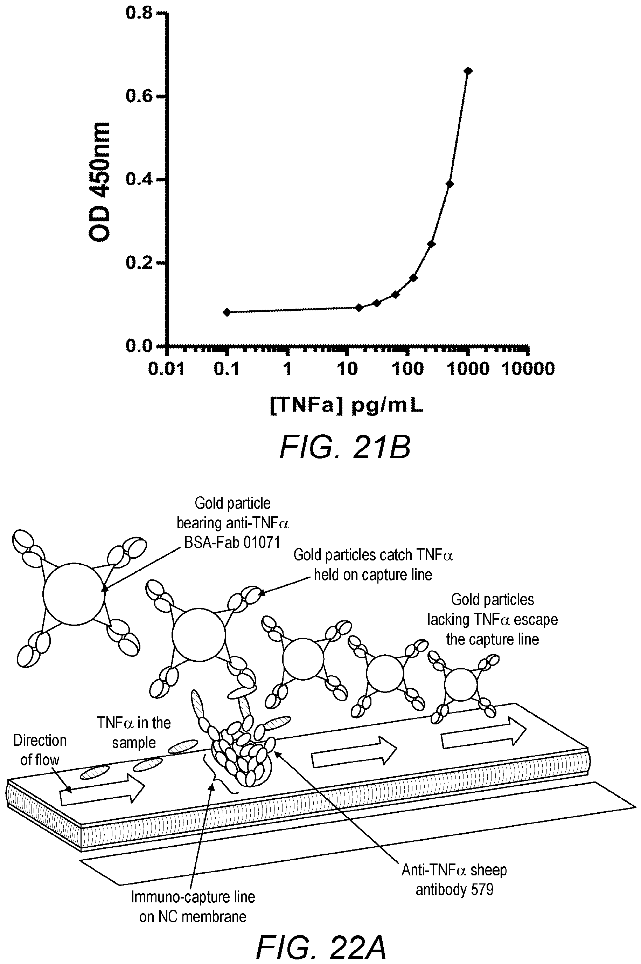

A schematic representation of a suitable assay format is shown in Figure 21A and a representative calibration curve for this assay is shown in Figure 21B.

TNFα may be detected in a lateral flow format in other embodiments. The method of the invention thus may include a lateral flow assay for detecting TNFα in a sample comprising:
(a) contacting the sample with an immunocapture surface (such as an immunocapture line on nitrocellulose membrane) on which is immobilised a reagent (such as an antibody, as defined herein, one specific example being anti-TNFa shep antibody 579) that specifically binds to TNFα in the sample
(b) adding a further reagent (such as an antibody, as defined herein, one specific example being anti-TNFa BSA-Fab 01071) that specifically binds to TNFα to the sample, which reagent is conjugated to a label (such as a gold particle, optionally a 40 nm gold particle)
(c) measuring the levels of label at the immunocapture surface as an indication of the levels of TNFα in the sample.

More specifically, TNFα may be detected in a lateral flow format comprising:
(a) contacting the sample with an immunocapture surface (such as an immunocapture line on nitrocellulose membrane) on which is immobilised a reagent (such as an antibody, as defined herein, one specific example being sheep anti-TNF catalogue number 579 Ig Innovations, immobilised at a concentration of 1mg/mL) that specifically binds to TNFα in the sample
(b) addition of sample (neat)
(c) TNFα in the sample complexes with a reagent present in conjugate pad (such as an antibody, as defined herein, one specific example being mouse anti- TNFα Fab 01071 antibody, Alere San Diego), which reagent is conjugated to a label (such as a gold particle, optionally a 40 nm gold particle at 20µg/mL in MES, pH5.3 buffer)
(d) measuring the levels of label at the immunocapture surface as an indication of the levels of TNFα in the sample after a specific read time (such as 10 minutes) with an immunochromatographic reader.
(e) A control system such as an BSA biotin capture (immobilised at a concentration of 0.25mg/mL) and an anti-biotin gold conjugate (catalogue number BA.GAB, BBI at OD2) indicates that the test is complete
(f) specified sensitivity (such as 11pg/mL) and an assay range (such as 11-8000pg/mL)

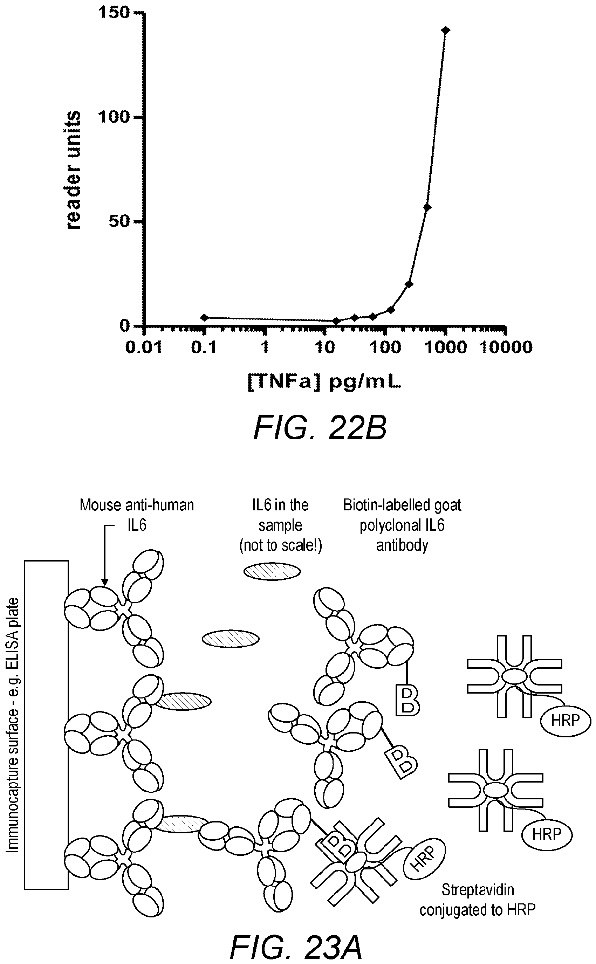

A schematic representation of a suitable assay format is shown in Figure 22A and a representative calibration curve for this assay is shown in Figure 22B.

The following example components may be used:

| | Name | Cat No | Source | Concentration |
|---|---|---|---|---|
| Capture antibody | Sheep polyclonal anti-TNFα | 579 | Ig Innovations | 1mg/mL |
| Detector antibody | Anti TNFα BSA-Fab 40nm gold conjugated | 01071 | Alere San Diego | 20µg/mL |
| Control line | BSA biotin | N/A | Mologic | 0.25mg/mL |
| Control gold | Anti-biotin gold | BA.GAB | BBI | OD2 |
| Standard | TNFα | 840121 | R&D systems | 11-8000pg/mL |

Another marker useful in the present invention is IL-6, a cytokine demonstrated to have a role in the immune response and in inflammation. It is elevated in infections and in chronic autoimmune diseases like rheumatoid arthritis. Pathogen Associated Molecular Patterns (PAMPs) bind to Pattern Recognition Receptors (PRRs) on macrophages, and induce the NFκβ intracellular cascade which leads to IL-6 expression, and the secretion of IL-6 as a pro-inflammatory cytokine. It is a pleiotropic cytokine, which is able to bind to IL-6 receptors and to the ubiquitous gp130 receptors when complexed to sIL-6R. As a part of the host response to infection, IL-6 levels have been shown to be elevated in sepsis patients (Hack, et al., 1989). Furthermore IL-6 levels have been shown to correlate well to sepsis severity (Damas, et al., 1992). According to the invention IL-6 may be detected by ELISA (enzyme-linked immunosorbent assay). The method of the invention thus may include an immunoassay such as an ELISA for detecting IL-6 in a sample comprising:
(a) contacting the sample with an immunocapture surface (such as an ELISA plate) on which is immobilised a reagent (such as an antibody, as defined herein, one specific example being mouse anti-human IL-6) that specifically binds to IL-6 in the sample
(b) adding a further reagent (such as an antibody, as defined herein, one specific example being mouse polyclonal IL-6 antibody) that specifically binds to IL-6 to the sample, which reagent is conjugated to a label (such as biotin)
(c) Adding an enzyme (such as horseradish peroxidase) conjugated to a binding partner for the label (for example streptavidin)
(d) removing reagent not bound to the immunocapture surface
(e) measuring the levels of enzyme activity at the immunocapture surface as an indication of the levels of IL-6 in the sample.

More specifically, the method of the invention also may include an ELISA for detecting IL-6 in a sample comprising:
(a) contacting the sample with an immunocapture surface (such as an ELISA plate) on which is immobilised a reagent (such as an antibody, as defined herein, one specific example being mouse anti-human IL-6, catalogue number 840113 R&D systems) that specifically binds to IL-6 in the sample (for a defined time such as overnight)
(b) removing reagent not bound to the immunocapture surface with a wash step (wash buffer 50mM tris buffered saline pH8, supplemented with 0.1% (v/v) Tween 20)
(c) blocking unbound sites on the surface with the addition of a blocking solution (such as R&D diluent, catalogue number DY995 R&D systems) for a defined time such as 1 hour.
(d) removing reagent not bound to the immunocapture surface with a wash step (wash buffer 50mM tris buffered saline pH8, supplemented with 0.1% (v/v) Tween 20)
(e) addition of sample diluted 1 in 2 in sample diluent PBS, pH6.9, supplemented with 1% BSA incubated for a defined time such as 2 hours
(f) removing reagent not bound to the immunocapture surface with a wash step (wash buffer 50mM tris buffered saline pH8, supplemented with 0.1% (v/v) Tween 20)
(g) adding a further reagent (such as an antibody, as defined herein, one specific example being goat polyclonal IL-6 antibody catalogue number 840114 R&D systems) that specifically binds to IL-6 in the sample, which reagent is conjugated to a label (such as biotin) for a defined time such as 2 hours
(h) removing reagent not bound to the immunocapture surface with a wash step (wash buffer 50mM tris buffered saline pH8, supplemented with 0.1% (v/v) Tween 20)
(i) Adding an enzyme (such as horseradish peroxidase) conjugated to a binding partner for the label (for example streptavidin, catalogue number 893975 R&D systems) for a defined time such as 20 minutes
(j) Adding a substrate that initiates the colour change (such as OPD, catalogue number P9187 Sigma-Aldrich)
(k) measuring the levels of enzyme activity at the immunocapture surface as an indication of the levels of IL-6 in the sample with a specified sensitivity (such as 9.4pg/mL) and an assay range (such as 18.75-1200pg/mL)

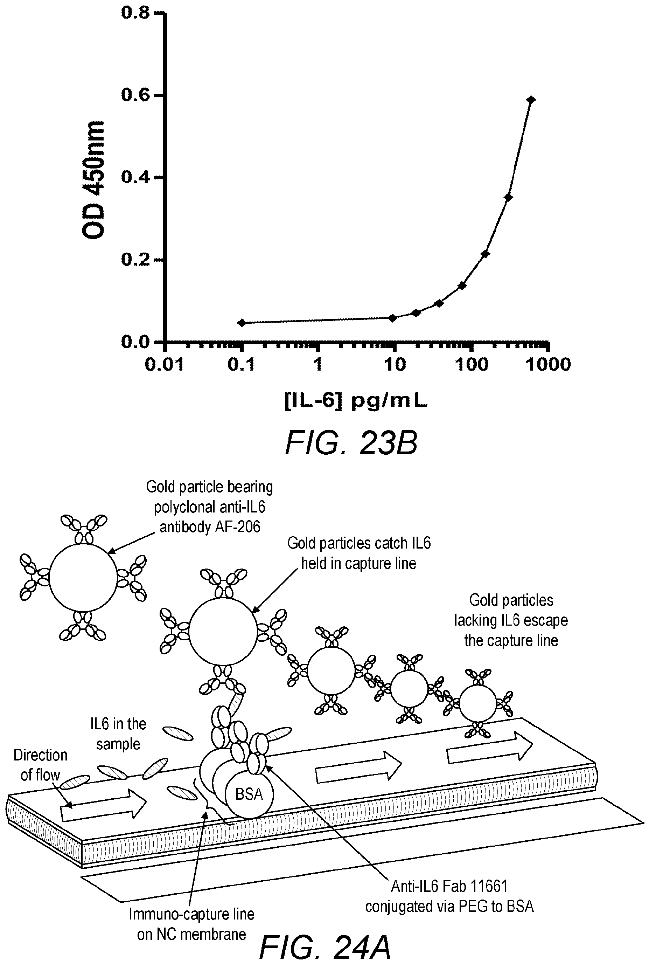

A schematic representation of a suitable assay format is shown in Figure 23A and a representative calibration curve for this assay is shown in Figure 23B.

IL-6 may be detected in a lateral flow format in other embodiments. The method of the invention thus may include a lateral flow assay for detecting IL-6 in a sample comprising:
(a) contacting the sample with an immunocapture surface (such as an immunocapture line on nitrocellulose membrane) on which is immobilised a reagent (such as an antibody, as defined herein, one specific example being anti-IL6 Fab 11661 conjugated via PEG to BSA) that specifically binds to IL-6 in the sample
(b) adding a further reagent (such as an antibody, as defined herein, one specific example being polyclonal anti-IL6 antibody AF-206-NA) that specifically binds to IL-6 to the sample, which reagent is conjugated to a label (such as a gold particle, optionally a 40 nm gold particle)
(c) measuring the levels of label at the immunocapture surface as an indication of the levels of IL-6 in the sample.

More specifically, IL-6 may be detected in a lateral flow format comprising:
(a) contacting the sample with an immunocapture surface (such as an immunocapture line on nitrocellulose membrane) on which is immobilised a reagent (such as an antibody, as defined herein, one specific example being mouse anti-IL-6 fab conjugated to BSA catalogue number 11661 Alere San Diego, immobilised at a concentration of 3mg/mL) that specifically binds to IL-6 in the sample
(b) addition of sample (neat)
(c) IL-6 in the sample complexes with a reagent present in conjugate pad (such as an antibody, as defined herein, one specific example being goat anti- IL-6 antibody, Catalogue number AF-206 R&D systems), which reagent is conjugated to a label (such as a gold particle, optionally a 40 nm gold particle at 11µg/mL in Borate, pH8.5 buffer)
(d) measuring the levels of label at the immunocapture surface as an indication of the levels of IL-6α in the sample after a specific read time (such as 10 minutes) with an immunochromatographic reader.
(e) A control system such as an BSA biotin capture (immobilised at a concentration of 0.25mg/mL) and an anti-biotin gold conjugate (catalogue number BA.GAB, BBI at OD2) indicates that the test is complete
(f) specified sensitivity (such as 37.5pg/mL) and an assay range (such as 37.5-2400pg/mL)

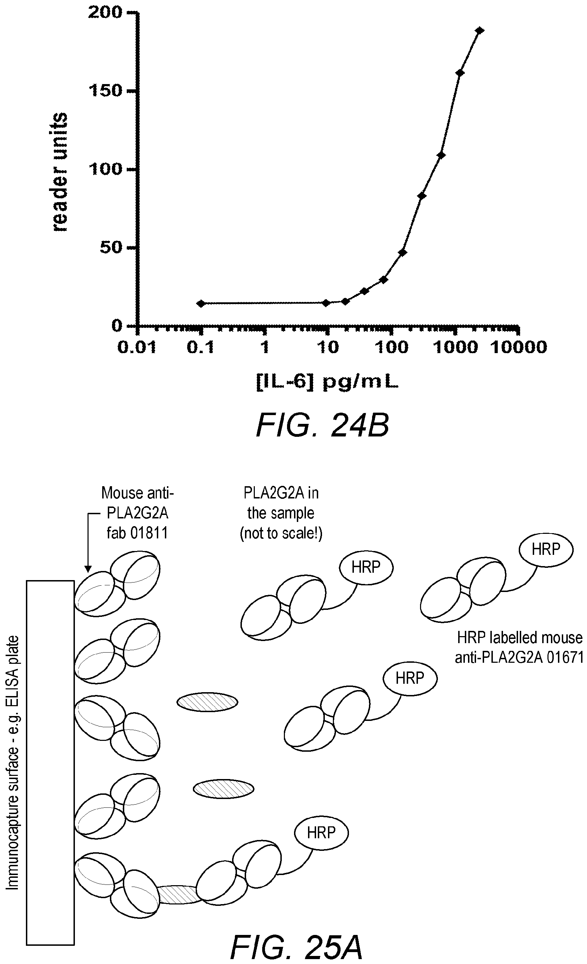

A schematic representation of a suitable assay format is shown in Figure 24A and a representative calibration curve for this assay is shown in Figure 24B.

The following example components may be used:

| | Name | Cat No | Source | Concentration |
|---|---|---|---|---|
| Capture antibody | Anti IL6 BSA Fab | 11661 | Alere San Diego | 3mg/mL |
| Detector antibody | Anti IL6 goat polyclonal 40nm gold conjugated | AF-206-NA | R&D systems | 11µg/mL |
| Control line | BSA biotin | N/A | Mologic | 0.25mg/mL |
| Control gold | Anti-biotin gold | BA.GAB | BBI | OD2 |
| Standard | IL6 | 840115 | R&D systems | 37.5-2400pg/mL |

Another marker useful in the present invention is PLA2G2A, a phospholipase which catabolises arachidonic acid. This enzymatic activity releases bioactive breakdown products which act as modulators of inflammation, such as eicosanoids (the target of NSAIDs). PLA2G2A is induced by a variety of cytokines, including IL-6 and TNFα (Crowl, Stoller, Conroy, & Stoner, 1991). Elevated PLA2G2A has been implicated in atherosclerosis and rheumatoid arthritis. Polymorphisms in the PLA2G2A gene have been investigated, and certain polymorphisms were found to predispose neonates to developing sepsis (Abu-Mazid, et al., 2010). According to the invention PLA2G2A may be detected by an immunoassay such as an ELISA (enzyme-linked immunosorbent assay). The method of the invention thus may include an ELISA for detecting PLA2G2A in a sample comprising:
(a) contacting the sample with an immunocapture surface (such as an ELISA plate) on which is immobilised a reagent (such as an antibody, as defined herein, one specific example being mouse anti-PLA2G2A fab 01811) that specifically binds to PLA2G2A in the sample
(b) adding a further reagent (such as an antibody, as defined herein, one specific example being anti- PLA2G2A 01671) that specifically binds to PLA2G2A to the sample, which reagent is conjugated to an enzyme (such as horseradish peroxidase)
(c) removing reagent not bound to the immunocapture surface
(d) measuring the levels of enzyme activity at the immunocapture surface as an indication of the levels of PLA2G2A in the sample.

More specifically, the method of the invention also may include an ELISA for detecting PLA2G2A in a sample comprising:
(a) contacting the sample with an immunocapture surface (such as an ELISA plate) on which is immobilised a reagent (such as an antibody, as defined herein, one specific example being mouse anti-human PLA2G2A fab, catalogue number 01811 Alere San Diego) that specifically binds to PLA2G2A in the sample (for a defined time such as overnight)
(b) removing reagent not bound to the immunocapture surface with a wash step (wash buffer 50mM tris buffered saline pH8, supplemented with 0.1% (v/v) Tween 20)
(c) blocking unbound sites on the surface with the addition of a blocking solution (such as PBS, pH6.9, supplemented with 1% BSA) for a defined time such as 1 hour.
(d) removing reagent not bound to the immunocapture surface with a wash step (wash buffer 50mM tris buffered saline pH8, supplemented with 0.1% (v/v) Tween 20)
(e) addition of sample diluted 1 in 10 in sample diluent PBS supplemented with 50% hispec assay diluent (BioRad), 10% FCS and 0.4% BSA, incubated for a defined time such as 1 hour
(f) removing reagent not bound to the immunocapture surface with a wash step (wash buffer 50mM tris buffered saline pH8, supplemented with 0.1% (v/v) Tween 20)
(g) adding a further reagent (such as an antibody, as defined herein, one specific example being mouse anti-human PLA2G2A fab, catalogue number 01671 Alere San Diego) that specifically binds to PLA2G2A in the sample, which reagent is conjugated to a label (such as horse radish peroxidase, catalogue number 702-0000, Innova bioscience) for a defined time such as 1 hour
(h) removing reagent not bound to the immunocapture surface with a wash step (wash buffer 50mM tris buffered saline pH8, supplemented with 0.1% (v/v) Tween 20)
(i) Adding a substrate that initiates the colour change (such as OPD, catalogue number P9187 Sigma-Aldrich)
(j) measuring the levels of enzyme activity at the immunocapture surface as an indication of the levels of PLA2G2A in the sample with a specified sensitivity (such as 0.78ng/mL) and an assay range (such as 7.8 - 500ng/mL)

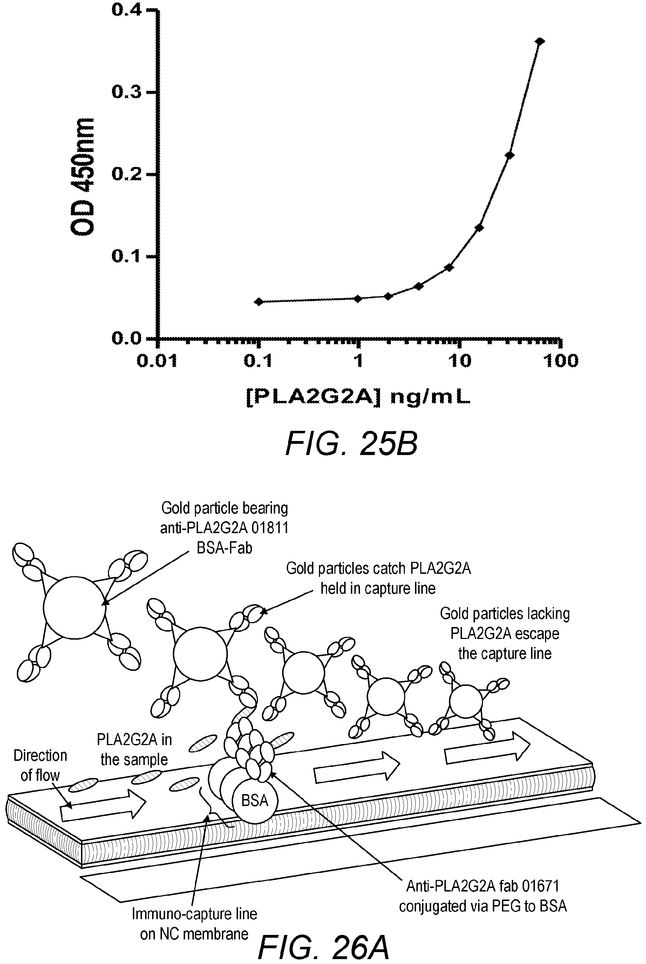

A schematic representation of a suitable assay format is shown in Figure 25A and a representative calibration curve for this assay is shown in Figure 25B.

The following example components may be used:

| | Name | Cat No | Source | Concentration |
|---|---|---|---|---|
| Capture antibody | Mouse Anti-PLA2G2A fab | 01811 | Alere San Diego | 2µg/mL |
| Detector antibody | Mouse Anti-PLA2G2A fab | 01671 | Alere San Diego | 100ng/mL |
| Standard | PLA2G2A | 5374-PL-010 | R&D Systems | 0.78-50ng/mL |

PLA2G2A may be detected in a lateral flow format in other embodiments. The method of the invention thus may include a lateral flow assay for detecting PLA2G2A in a sample comprising:
(a) contacting the sample with an immunocapture surface (such as an immunocapture line on nitrocellulose membrane) on which is immobilised a reagent (such as an antibody, as defined herein, one specific example being anti-PLA2G2A fab 01671 conjugated via PEG to BSA) that specifically binds to PLA2G2A in the sample
(b) adding a further reagent (such as an antibody, as defined herein, one specific example being anti-PLA2G2A 01811 BSA-Fab) that specifically binds to PLA2G2A to the sample, which reagent is conjugated to a label (such as a gold particle, optionally a 40 nm gold particle)
(c) measuring the levels of label at the immunocapture surface as an indication of the levels of PLA2G2A in the sample.

More specifically, PLA2G2A may be detected in a lateral flow format comprising:
(a) contacting the sample with an immunocapture surface (such as an immunocapture line on nitrocellulose membrane) on which is immobilised a reagent (such as an antibody, as defined herein, one specific example being mouse anti-PLA2G2A fab catalogue number 01671 Alere San Diego, immobilised at a concentration of 1.5mg/mL) that specifically binds to PLA2G2A in the sample
(b) addition of sample (neat)
(c) PLA2G2A in the sample complexes with a reagent present in conjugate pad (such as an antibody, as defined herein, one specific example being mouse anti- IL-6 antibody, Catalogue number 01811 Alere San Diego), which reagent is conjugated to a label (such as a gold particle, optionally a 40 nm gold particle at 10µg/mL in TAPS, pH8.5 buffer)
(d) measuring the levels of label at the immunocapture surface as an indication of the levels of PLA2G2A in the sample after a specific read time (such as 10 minutes) with an immunochromatographic reader.
(e) A control system such as an BSA biotin capture (immobilised at a concentration of 0.25mg/mL) and an anti-biotin gold conjugate (catalogue number BA.GAB, BBI at OD2) indicates that the test is complete
(f) specified sensitivity (such as 2ng/mL) and an assay range (such as 2-500ng/mL)

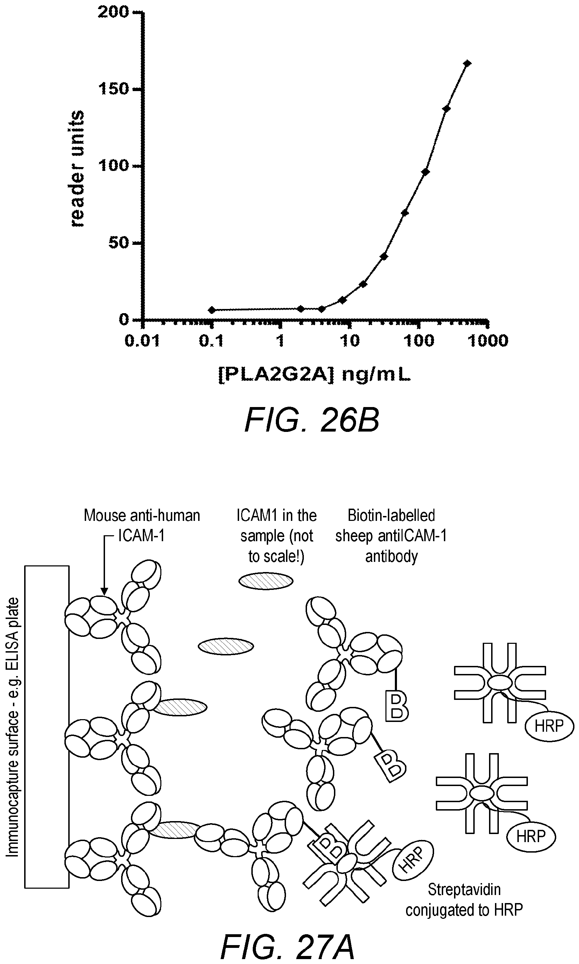

A schematic representation of a suitable assay format is shown in Figure 26A and a representative calibration curve for this assay is shown in Figure 26B. The following example components may be used:

| | Name | Cat No | Source | Concentration |
|---|---|---|---|---|
| Capture antibody | Anti PLA2G2A BSA Fab | 01671 | Alere San Diego | 1.5mg/mL |
| Detector antibody | Anti PLA2G2A BSA Fab 40nm gold conjugated | 01811 | Alere San Diego | 10µg/mL |
| Control line | BSA biotin | N/A | Mologic | 0.25mg/mL |
| Control gold | Anti-biotin gold | BA.GAB | BBI | OD2 |
| Standard | PLA2G2A | 5374-PL-010 | R&D Systems | 2-500ng/mL |

Another marker useful in the present invention is sICAM1 the cell-free, circulating version of ICAM1. ICAM1 interacts with LFA-1 to contribute towards T-cell activation and leukocyte endothelial migration. sICAM1 competitively binds LFA-1, preventing the LFA-1/ICAM1 complex forming (Meyer, Dustin, & Carron, 1995), reducing T-cell activation. sICAM1 has been implicated in a variety of diseases. sICAM1 levels have been linked to Blood Brain Barrier Dysfunction following traumatic injury, this is thought to be mediated by induction of MIP-2 (Otto, Heinzel-Pleines, Gloor, Trentz, Kossmann, & Morganti-Kossmann, 2000). Elevated sICAM levels have also been found in cardiovascular disease (Jenny, et al., 2006) and breast cancer (Merendino, et al., 2001). sICAM1 levels were found to be elevated in patients with Sepsis related Multiple Organ Failure (MOF) compared with patients with sepsis but without MOF, suggesting it may be a marker of disease severity (Endo, et al., 1995). According to the invention sICAM1may be detected by an immunoassay such as an ELISA (enzyme-linked immunosorbent assay). The method of the invention thus may include an ELISA for detecting sICAM1in a sample comprising:
(a) contacting the sample with an immunocapture surface (such as an ELISA plate) on which is immobilised a reagent (such as an antibody, as defined herein, one specific example being mouse anti-human ICAM1) that specifically binds to sICAM1 in the sample
(b) adding a further reagent (such as an antibody, as defined herein, one specific example being mouse mouse monoclonal anti-ICAM-1 antibody) that specifically binds to sICAM1 to the sample, which reagent is conjugated to a label (such as biotin)
(c) Adding an enzyme (such as horseradish peroxidase) conjugated to a binding partner for the label (for example streptavidin)
(d) removing reagent not bound to the immunocapture surface
(e) measuring the levels of enzyme activity at the immunocapture surface as an indication of the levels of sICAM1in the sample.

More specifically, the method of the invention thus may include an ELISA for detecting sICAM-1 in a sample comprising:
(a) contacting the sample with an immunocapture surface (such as an ELISA plate) on which is immobilised a reagent (such as an antibody, as defined herein, one specific example being mouse anti-human sICAM-1, catalogue number 840432 R&D systems) that specifically binds to CRP in the sample (for a defined time such as overnight)
(b) removing reagent not bound to the immunocapture surface with a wash step (wash buffer 50mM tris buffered saline pH8, supplemented with 0.1% (v/v) Tween 20)
(c) blocking unbound sites on the surface with the addition of a blocking solution (such as R&D diluent, catalogue number DY995 R&D systems) for a defined time such as 1 hour.
(d) removing reagent not bound to the immunocapture surface with a wash step (wash buffer 50mM tris buffered saline pH8, supplemented with 0.1% (v/v) Tween 20)
(e) addition of sample diluted 1 in 500 in sample diluent PBS, pH6.9, supplemented with 1% BSA incubated for a defined time such as 2 hours
(f) removing reagent not bound to the immunocapture surface with a wash step (wash buffer 50mM tris buffered saline pH8, supplemented with 0.1% (v/v) Tween 20)
(g) adding a further reagent (such as an antibody, as defined herein, one specific example being Sheep anti sICAM-1 catalogue number 840433 R&D systems) that specifically binds to sICAM-1 in the sample, which reagent is conjugated to a label (such as biotin) for a defined time such as 2 hours
(h) removing reagent not bound to the immunocapture surface with a wash step (wash buffer 50mM tris buffered saline pH8, supplemented with 0.1% (v/v) Tween 20)
(i) Adding an enzyme (such as horseradish peroxidase) conjugated to a binding partner for the label (for example streptavidin, catalogue number 893975 R&D systems) for a defined time such as 20 minutes
(j) Adding a substrate that initiates the colour change (such as OPD, catalogue number P9187 Sigma-Aldrich)
(k) measuring the levels of enzyme activity at the immunocapture surface as an indication of the levels of sICAM-1 in the sample with a specified sensitivity (such as 31.2pg/mL) and an assay range (such as 15.6-1000ng/mL)

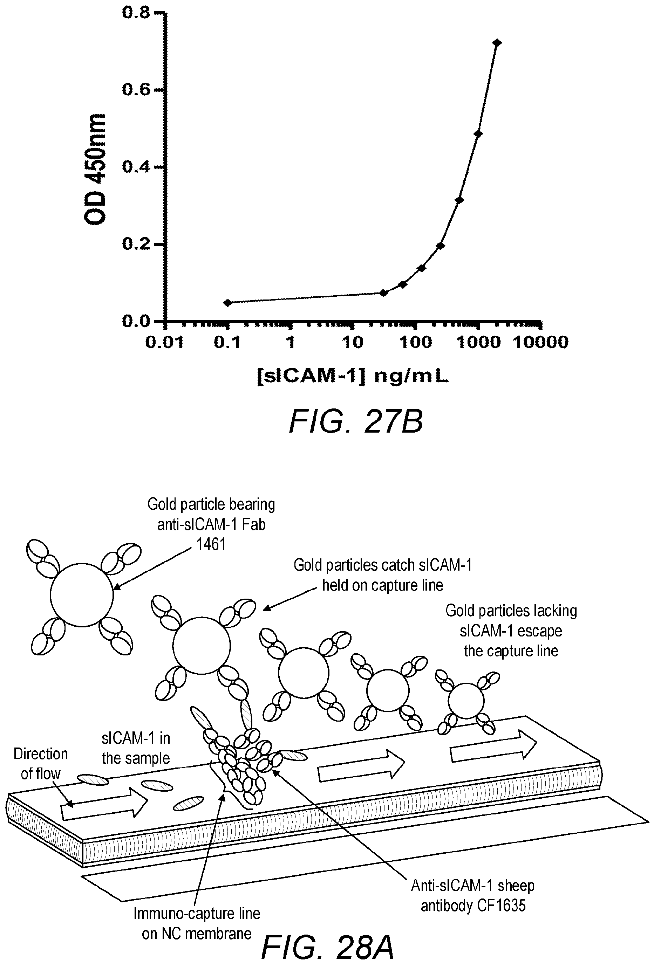

A schematic representation of a suitable assay format is shown in Figure 27A and a representative calibration curve for this assay is shown in Figure 27B.

slCAM1 may be detected in a lateral flow format in other embodiments. The method of the invention thus may include a lateral flow assay for detecting sICAM1 in a sample comprising:
(a) contacting the sample with an immunocapture surface (such as an immunocapture line on nitrocellulose membrane) on which is immobilised a reagent (such as an antibody, as defined herein, one specific example being anti-sICAM-1 sheep antibody CF 1635) that specifically binds to sICAM1 in the sample
(b) adding a further reagent (such as an antibody, as defined herein, one specific example being anti-sICAM-1 Fab 1461) that specifically binds to sICAM1 to the sample, which reagent is conjugated to a label (such as a gold particle, optionally a 40 nm gold particle)
(c) measuring the levels of label at the immunocapture surface as an indication of the levels of sICAM1 in the sample.

More specifically, sICAM-1 may be detected in a lateral flow format comprising:
(a) contacting the sample with an immunocapture surface (such as an immunocapture line on nitrocellulose membrane) on which is immobilised a reagent (such as an antibody, as defined herein, one specific example being sheep anti-sICAM-1 catalogue number CF1635 Ig Innovations, immobilised at a concentration of 1 mg/mL) that specifically binds to sICAM-1 in the sample
(b) addition of 1 in 30 in sample diluent PBS, pH6.9, supplemented with 1% (v/v) Tween 20 + 1% BSA
(c) sICAM-1 in the sample complexes with a reagent present in conjugate pad (such as an antibody, as defined herein, one specific example being mouse anti- IL-6 antibody, Catalogue number 1461 Alere San Diego), which reagent is conjugated to a label (such as a gold particle, optionally a 40 nm gold particle at 15µg/mL in TAPS, pH8.5 buffer)
(d) measuring the levels of label at the immunocapture surface as an indication of the levels of sICAM-1 in the sample after a specific read time (such as 10 minutes) with an immunochromatographic reader.
(e) A control system such as an BSA biotin capture (immobilised at a concentration of 0.25mg/mL) and an anti-biotin gold conjugate (catalogue number BA.GAB, BBI at OD2) indicates that the test is complete
(f) specified sensitivity (such as 42ng/mL) and an assay range (such as 0.042-32µg/mL)

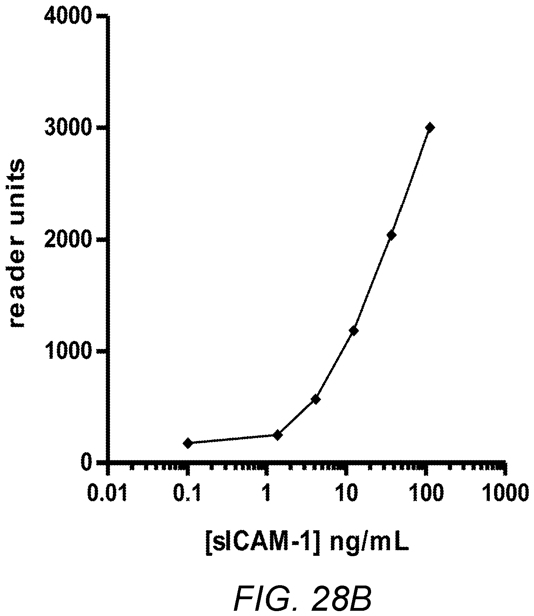

A schematic representation of a suitable assay format is shown in Figure 28A and a representative calibration curve for this assay is shown in Figure 28B.

The following example components may be used:

| | Name | Cat No | Source | Concentration |
|---|---|---|---|---|
| Capture antibody | Anti sICAM-1 Sheep antibody | CF1635 | Ig innovations | 1mg/mL |
| Detector antibody | Anti sICAM-1 Fab 40nm gold conjugated | 1461 | Alere San Diego | 15µg/m L |
| Control line | BSA biotin | N/A | Mologic | 0.25mg/mL |
| Control gold | Anti-biotin gold | BA.GAB | BBI | OD2 |
| Standard | sICAM1 | PR21065 | Alere San Diego | 1.4-1000ng/mL |

According to all aspects of the invention levels of at least CCL23, A1AT and CRP and optionally any additional markers may be calculated with reference to a threshold level of each marker. The threshold levels may be adapted (or personalised) to the subject. The invention may therefore rely upon a personalised baseline level of the relevant marker or markers against which the threshold is calculated. Calculation may be on an on-going basis to coincide with testing. Thus, the threshold may be a rolling threshold derived from the rolling baseline. In this context, it is apparent that levels of the marker or markers do not have to be measured in absolute terms and may be measured in absolute or relative terms. The markers simply have to be measured in a manner which permits a comparison to be made with marker levels in samples taken at different time points. Alternatively, the threshold level for each marker may be set based on a population analysis. The threshold level may be set to maximise sensitivity and/or specificity of detection as would be readily appreciated by one skilled in the art.

In some embodiments, the threshold level of the marker is set by determining the levels of the marker in samples taken from the subject at earlier time points (such as before surgery). In its simplest form, the invention may rely upon a simple comparison between the test sample and the level of the marker in the previously taken sample (i.e. a single earlier time point). However, the earlier time points may comprise at least two, and possibly 3, 4, 5, 6, 7, 8, 9, 10 etc, earlier measurements immediately preceding the determination of the level of the marker in the current sample.

Where marker levels are measured at multiple time points those levels may be averaged to provide the threshold for the test sample, above which a SIRS and/or sepsis is predicted or identified. In some embodiments, the threshold may be set with reference to a sliding window within which levels of the markers have been measured to provide a baseline. The threshold level is thus "learned" by the system. It is not a fixed threshold and is adapted to the subject, thereby taking into account insignificant fluctuations in marker levels from the baseline that are not predictive or indicative of SIRS and/or sepsis. Accordingly, the threshold may be set around the baseline to specify an allowable range of the marker levels beyond which a statistically significant increase (or decrease) in level is indicated. In the presence of drift of the baseline level of the marker, it is possible that the parameter limits may be narrowed such that a further change in level of the markers is deemed significant. For example, if the baseline marker level is drifting upwards over time, the difference between a measured increase and baseline may need to be smaller (compared to the situation in which the baseline is relatively stable) to be considered to have exceeded the threshold (i.e. to be significant). For example, a difference from baseline of at least 5, 10, 15, 20% or more may be considered significant generally. This difference may be reduced if there have been multiple previous measurements displaying a trend upwards or downwards but in each case by an amount less than the threshold difference. The difference (in order to be considered significant) may thus be reduced to at least 1, 2, 3, 4, 5% or more as appropriate in the event of a drift upwards or downwards in the baseline.

The threshold may be set in relation to multiple markers as discussed in greater detail herein. Thus, the prediction or diagnosis of SIRS and/or sepsis may be identified based upon a deviation from baseline that is cumulative according to the multiple markers measured. Typically, however, each marker will be measured individually with reference to a marker specific baseline and against a marker specific threshold. It is shown herein that use of multiple individual markers provides an improved ability to predict or diagnose SIRS and/or sepsis. Thus, the invention may rely upon a plurality of baselines/thresholds depending upon the individual markers employed. The methods and systems may weight the contribution of a plurality of markers.

Because the present invention relies upon measuring the levels of multiple markers, the final prediction or identification or sepsis and/or SIRS requires that the measured levels are integrated, ideally to provide a simply binary result that is readily interpreted. A suitable algorithm may be employed in order to interpret the data from the levels of the at least three markers and apply it to provide the prediction or diagnosis. In some embodiments, the marker levels may be inter-dependent and thus the algorithm is based on this predicted relationship. In certain embodiments, the determined levels of the at least three (or more) markers are analysed in a pre-determined sequence to monitor the subject. This may give rise to a decision tree, as explained further herein to predict or diagnose sepsis and/or SIRS. The levels of the first of the multiple markers may influence the subsequent thresholds required for the other markers in order to predict or diagnose sepsis and/or SIRS, as would readily be appreciated by one skilled in the art. The output of the methods may also guide future sampling and treatment of the subject. Thus, in some embodiments, for a given sample, the marker levels may be analysed in sequence until a marker is found with an increased level (or all markers have been examined). If a marker is detected at increased level the further markers may or may not also be assessed to determine if their level is also increased. The likelihood of SIRS and/or sepsis may be higher in the event that multiple markers are increased in a sample and the algorithm may account for this in the outcome, e.g. by weighting the observations. Thus, the sample may be "graded" based upon how many of the markers are increased in level compared to threshold. For example, Grade 1 may indicate only one of the markers is increased in the sample, Grade 2 may indicate two of the markers is increased etc. Grade 3 or above may predict or diagnose SIRS and/or sepsis. Alternatively, the presence of an increased level of one marker is enough to predict or diagnose sepsis (and/or SIRS) but an increased level of one, two or more further markers increases the confidence level of the prediction or diagnosis.

In some embodiments, the determined levels of at least CCL23, A1AT and CRP and optionally any additional markers are weighted. Weighting is a well-known method of applying a degree of relative significance to the multiple markers. The algorithm may be a threshold based algorithm as discussed herein.

The levels of the measured markers may be combined using logistic regression, decision tree analysis, neural networks and/or machine learning. Logistic regression analysis involves formulating a statistical model which adds different markers together in a weighted fashion. Similar to linear regression which can be resolved using the equation y=mx+c, logistic regression allows for the addition of multiple markers and only allows for a binary outcome so the y is replaced with the logit (defined as the In(odds) of being in the positive outcome group). Mathematically, the logistic regression equation is logit=(βnXn)+c. This uses quantitative data of all markers in a weighted fashion in the calculation.

In decision tree analysis, an individual is assessed for one marker at a time until they reach a terminal node which classifies the patient into the positive or negative outcome group. This uses cut-off values for each individual marker and does not necessarily use all markers in the algorithm, depending upon at which point they are categorised.

Use of neural network displays some similarity to logistic regression in that each node is a summation of the input (marker) multiplied by a weighting (beta coefficient). However, summation is performed a number of times; there are a number of nodes and the input to these nodes can be nodes themselves rather than the measured levels. The nodes are first entered at random with random weights, the difference between the expected output and the observed output is then calculated. If it is not 0 (which is likely to be the case) the weightings are altered in the preceding layer and then in the layer before that until the input variables are reached. The outputs are recalculated and the differences are calculated again, and the model weighting readjusted. This can continue indefinitely until the difference in expected and observed outputs is minimal.

In specific embodiments, where sepsis is diagnosed or predicted the subject is treated. Suitable treatments for sepsis are known in the art. They include antibiotics as discussed in more detail herein. The subject may continue monitoring during treatment in order to assess the effectiveness of the treatment.

The invention may be performed using systems or test kits as described herein. The invention may be performed using a testing device, testing kit or testing composition of matter as described herein.

Also disclosed are the computer applications used in the systems and test kits. The computer applications may also be used in the testing devices, testing kits or testing composition of matters described herein (in particular that incorporate a reader). Thus, in certain embodiments, the computer-implemented method, system, and computer program product may be embodied in a computer application, for example, that operates and executes on a processor, such as in the context of a computing machine.

As used herein, the processor may be comprised within any computer, server, embedded system, or computing system. The computer may include various internal or attached components such as a system bus, system memory, storage media, input/output interface, and a network interface for communicating with a network, for example.

The computer may be implemented as a conventional computer system, an embedded controller, a laptop, a server, a customized machine, any other hardware platform, such as a laboratory computer or device, for example, or any combination thereof. The computing machine may be a distributed system configured to function using multiple computing machines interconnected via a data network or bus system, for example.

The processor may be configured to execute code or instructions to perform the operations and functionality described herein, manage request flow and address mappings, and to perform calculations and generate commands. The processor may be configured to monitor and control the operation of the components in the computing machine. The processor may be a general purpose processor, a processor core, a multiprocessor, a reconfigurable processor, a microcontroller, a digital signal processor ("DSP"), an application specific integrated circuit ("ASIC"), a graphics processing unit ("GPU"), a field programmable gate array ("FPGA"), a programmable logic device ("PLD"), a controller, a state machine, gated logic, discrete hardware components, any other processing unit, or any combination or multiplicity thereof. The processor may be a single processing unit, multiple processing units, a single processing core, multiple processing cores, special purpose processing cores, co-processors, or any combination thereof. According to certain example embodiments, the processor, along with other components of the computing machine, may be a virtualized computing machine executing within one or more other computing machines.

The storage medium may be selected from a hard disk, a floppy disk, a compact disc read only memory ("CD-ROM"), a digital versatile disc ("DVD"), a Blu-ray disc, a magnetic tape, a flash memory, other non-volatile memory device, a solid-state drive ("SSD"), any magnetic storage device, any optical storage device, any electrical storage device, any semiconductor storage device, any physical-based storage device, any other data storage device, or any combination or multiplicity thereof. The storage media may store one or more operating systems, application programs and program modules such as module, data, or any other information. The storage media may be part of, or connected to, the computing machine. The storage media may also be part of one or more other computing machines that are in communication with the computing machine, such as servers, database servers, cloud storage, network attached storage, and so forth.

The storage media may therefore represent examples of machine or computer readable media on which instructions or code may be stored for execution by the processor. Machine or computer readable media may generally refer to any medium or media used to provide instructions to the processor. Such machine or computer readable media associated with the module may comprise a computer software product.

The input/output ("I/O") interface may be configured to couple to one or more external devices, to receive data from the one or more external devices, and to send data to the one or more external devices. Such external devices along with the various internal devices may also be known as peripheral devices. The I/O interface may include both electrical and physical connections for operably coupling the various peripheral devices to the computing machine or the processor. The I/O interface may be configured to communicate data, addresses, and control signals between the peripheral devices, the computing machine, or the processor. The I/O interface may be configured to implement any standard interface, such as small computer system interface ("SCSI"), serial-attached SCSI ("SAS"), fiber channel, peripheral component interconnect ("PCI"), PCI express (PCIe), serial bus, parallel bus, advanced technology attached ("ATA"), serial ATA ("SATA"), universal serial bus ("USB"), Thunderbolt, FireWire, various video buses, and the like. The I/O interface may be configured to implement only one interface or bus technology.

Alternatively, the I/O interface may be configured to implement multiple interfaces or bus technologies. The I/O interface may be configured as part of, all of, or to operate in conjunction with, the system bus. The I/O interface may include one or more buffers for buffering transmissions between one or more external devices, internal devices, the computing machine, or the processor.

The I/O interface may couple the computing machine to various input devices including mice, touch-screens, scanners, electronic digitizers, sensors, receivers, touchpads, trackballs, cameras, microphones, keyboards, any other pointing devices, or any combinations thereof. The I/O interface may couple the computing machine to various output devices including video displays, speakers, printers, projectors, tactile feedback devices, automation control, robotic components, actuators, motors, fans, solenoids, valves, pumps, transmitters, signal emitters, lights, and so forth.

The computing machine may operate in a networked environment using logical connections through the network interface to one or more other systems or computing machines across the network. The network may include wide area networks (WAN), local area networks (LAN), intranets, the Internet, wireless access networks, wired networks, mobile networks, telephone networks, optical networks, or combinations thereof. The network may be packet switched, circuit switched, of any topology, and may use any communication protocol. Communication links within the network may involve various digital or an analog communication media such as fiber optic cables, free-space optics, waveguides, electrical conductors, wireless links, antennas, radio-frequency communications, and so forth.

The processor may be connected to the other elements of the computing machine or the various peripherals discussed herein through the system bus. It should be appreciated that the system bus may be within the processor, outside the processor, or both. According to some embodiments, any of the processor, the other elements of the computing machine, or the various peripherals discussed herein may be integrated into a single device such as a system on chip ("SOC"), system on package ("SOP"), or ASIC device.

Embodiments may comprise a computer program that embodies the functions described and illustrated herein, wherein the computer program is implemented in a computer system that comprises instructions stored in a machine-readable medium and a processor that executes the instructions. However, it should be apparent that there could be many different ways of implementing embodiments in computer programming, and the embodiments should not be construed as limited to any one set of computer program instructions. Further, a skilled programmer would be able to write such a computer program to implement one or more of the disclosed embodiments described herein. Therefore, disclosure of a particular set of program code instructions is not considered necessary for an adequate understanding of how to make and use embodiments. Further, those skilled in the art will appreciate that one or more aspects of embodiments described herein may be performed by hardware, software, or a combination thereof, as may be embodied in one or more computing systems. Moreover, any reference to an act being performed by a computer should not be construed as being performed by a single computer as more than one computer may perform the act.

The example embodiments described herein can be used with computer hardware and software that perform the methods and processing functions described previously. The systems, methods, and procedures described herein can be embodied in a programmable computer, computer-executable software, or digital circuitry. The software can be stored on computer-readable media. For example, computer-readable media can include a floppy disk, RAM, ROM, hard disk, removable media, flash memory, memory stick, optical media, magneto-optical media, CD-ROM, etc. Digital circuitry can include integrated circuits, gate arrays, building block logic, field programmable gate arrays (FPGA), etc.

The methods, systems, test kits, testing devices, testing kits and testing compositions of matter may incorporate means for Automatic Identification and Data Capture (AIDC), such as a Radio-frequency identification tag or card (RIF)

For the avoidance of doubt, the discussion of the invention hereinabove applies to the systems, test kits, testing devices, testing kits and testing compositions of matter of the invention and all embodiments can be applied accordingly. However, for clarity and by way of exemplification of how the discussion applies directly to the systems, test kits, testing devices, testing kits and testing compositions of matter, further specific embodiments are outlined below.

In some embodiments, the systems, test kits, testing devices, testing kits and testing compositions of matter take the form of a portable system. An example system upon which the various products of the invention may be based is the Alere™ DDS®2 mobile test system. This system comprises an analyser, into which a test cartridge is inserted. The user then also inserts a sample collection device into the analyser. The analyser incorporates a full colour screen to read the results. The analyser thus houses the processor and storage medium which permits the assays to be run. The test cartridge represents the one or more testing devices for determining levels of the markers. The systems or test kits of the invention may incorporate a separate sample collection device or this may be integrated into the one or more testing devices.

In specific embodiments, the systems, test kits, testing devices, testing kits and testing compositions of matter further comprise a display for the output from the processor. This is intended to give a simple visual and/or audible read-out of the assays performed on the sample. The display may be operably connected to the processor running the computer application. The output or read-out may be an instruction to the subject in some embodiments. The output may be colour coded or numerical to reflect the various possible outcomes of monitoring as discussed herein. It is possible for the display to provide levels of the markers measured in the sample and provide suitable training and/or documentation to assist the user in interpretation of the data. However, this is not preferred for obvious reasons of susceptibility to human error. A combination of both types of information may, however, be presented in some embodiments. Thus, the display may present both quantitative and qualitative read-outs in some embodiments. Probability values related to the predictive and identification outcomes may also represent an output in some embodiments.

In specific embodiments, the one or more testing devices, testing kits or testing compositions of matter comprise disposable single use devices to which the sample is applied. Typically the one or more testing devices, testing kits or testing compositions of matter may comprise a sample receiving zone to which the sample is added. The devices typically also incorporate a solid support which defines a liquid/capillary flow path for the sample once applied to the sample receiving zone. The sample receiving zone may be an integral part of the solid support. The solid support may comprise a chromatographic medium, such as a membrane material in some embodiments (e.g. nitrocellulose). A sample applied to the sample application zone will typically rehydrate the necessary reagents to detect the marker. The reagents include binding reagents which specifically interact with the markers or a substrate for effector molecules where activity is measured. Further reagents immobilized further along the flow path bind to the complex of marker and binding reagent. The binding reagent is labelled to provide a signal at the site of immobilization of the complex of marker and binding reagent (through binding to the further reagent). Suitable labels include fluorescent labels, magnetic labels, latex or gold as would be readily understood by one skilled in the art.

When enzymatic activity is being assayed the binding reagent and/or further binding reagent may bind with a substrate only after it has been modified by the enzymatic activity, or may only bind if the substrate has not been modified by the enzymatic activity. Examples of enzymatic activity assays include those set forth in International Patent Applications WO2009/024805, WO2009/063208, WO2007/128980, WO2007/096642, WO2007/096637, WO2013/156794, WO2015/059487 and WO2013/156795 . The binding reagent and further reagent are typically antibodies (as defined herein). Thus, in specific embodiments, the one or more testing devices, testing kits or testing compositions of matter may comprise a lateral flow test strip. In some embodiments, a single lateral flow test strip is employed to permit detection of all markers that are to be determined in the test sample. In other embodiments, a separate lateral flow test strip is provided for each marker that is determined.

The devices, kits or compositions of matter may also include a control zone to confirm sample has passed through the device satisfactorily. In the event this is not the case the system or test kit or reader of the testing device, testing kit or testing composition of matter may indicate an invalid result to the user, for example via the display. The devices, kits or compositions of matter may act as competitive or sandwich assays, as discussed herein. ELISA (enzyme linked immunosorbent assay) is an example of a suitable assay format that may be incorporated in the testing devices used in the invention. Again, typically all reagents to detect the levels of the three or more markers are pre-loaded onto the testing device, kit or composition of matter such that they can interact with the sample once added to the device (for example via the sample receiving zone). This minimizes intervention and thus error caused by the subject. Thus, effectively, the device may only require the user to apply the sample and subsequently observe the output of the assay.

The systems, test kits, testing devices and testing compositions of matter may incorporate a suitable reader to provide a quantitative output (in conjunction with the processor and storage medium). As already mentioned this output can be an absolute or a relative output. Suitable readers may incorporate an illuminator to expose the device to a specific wavelength or wavelengths of light and a suitable detector for the reflected or emitted light. The systems, test kits, testing devices and testing compositions of matter may also incorporate a suitable processor and computer application to output the results based upon the detected signal. Thus, the processor running the computer application will be in operable connection with the reader. By "operable connection" is meant a functional connection that permits the exchange of a signal or information between the elements.

As discussed above, where protein levels are measured the binding reagent may comprise an antibody (to include derivatives, fragments and aptamers). Where RNA levels are measured suitable reagents may comprise nucleic acid amplification reagents such as primers, probes, dNTPs, polymerases etc. to permit amplification reactions to be run and results reported from the testing device.

The one or more testing devices, kits or compositions of matter may comprise an enzyme detection device. These devices may be particularly useful for investigating enzymatic activity.

The system or test kit may incorporate the appropriate number of testing devices to permit each marker to be determined. This is particularly the case where the markers are detecting using different platforms. Thus, in some embodiments, the one or more testing devices comprise one or more lateral flow activity assays, ELISAs, fluorogenic substrate assays etc. In some embodiments, the one or more testing devices comprise one or more lateral flow activity assays, ELISAs or competition assays. In some embodiments, the one or more testing devices comprise one or more lateral flow assays and ELISAs.

In certain embodiments, the computer application is configured to output from the processor a diagnosis or prediction of SIRS and/or sepsis if an increase in the levels of each of the at least CCL23, A1AT and CRP or more markers is calculated. In specific embodiments, the output is an indication that the subject should receive treatment.

It is also shown herein that certain markers are able to identify, within infected patients, the severity of the condition according to SOFA score. Thus, described herein is a method for discriminating a SOFA score of at least 2 from those with a SOFA score of less than 2 in a patient with systemic inflammatory response syndrome (SIRS) and an infection, the method comprising determining levels of at least one, two, three or more markers selected from PLA2, CRP, CCL23, sICAM, IL-6, procalcitonin, A1AT, MMP8, TNFalpha, AcPGP, enzymatic MMP activity, TIMP1, sRAGE and desmosine in a sample taken from the subject, wherein the combined levels of the at least three markers are used to discriminate a patient with a SOFA score of at least 2 from a SOFA score of less than 2. It is preferred that the markers are selected from 1, 2, 3, 4, 5, 6 or all of CCL23, A1AT, sICAM, desmosine, TNF alpha, IL-6 and PLA2g2A. The various systems, test kits, testing devices and testing compositions of matterdescribed herein can be adapted to perform such methods as would be readily understood by one skilled in the art.

### DESCRIPTION OF THE FIGURES

Figure 1 shows pie chart representations of the nature of infections detected in the clinical samples
Figure 2A shows the origins of infections in sepsis samples
Figure 2B shows the origins of infections in sepsis samples infected with gram negative bacteria
Figure 2C shows the origins of infections in sepsis samples infected with gram positive bacteria
Figure 3 shows multiple ROC curves for the various assays performed on the samples. The source of each curve is indicated.
Figure 4 is a scatter plot showing the ability of the CRP, sICAM and TNFalpha marker combination to distinguish sepsis from controls in the D samples
Figure 5 is a ROC curve showing the ability of the CRP, sICAM and TNFalpha marker combination to distinguish sepsis from controls in the D samples
Figure 6A is a scatter plot showing the ability of the CRP, sICAM and TNFalpha marker combination to distinguish sepsis from controls in the A samples
Figure 6B is a box plot showing the ability of the CRP, sICAM and TNFalpha marker combination to distinguish sepsis from controls in the A samples
Figure 7 is a ROC curve showing the ability of the CRP, sICAM and TNFalpha marker combination to distinguish sepsis from controls in the A samples
Figure 8A is a scatter plot showing the ability of the sICAM-1 (ELISA and LF), CCL23, A1AT, CRP, IL-6 and TNF-α marker combination to distinguish sepsis from SIRS
Figure 8B is a box plot showing the ability of the sICAM-1 (ELISA and LF), CCL23, A1AT, CRP, IL-6 and TNF-α marker combination to distinguish sepsis from SIRS
Figure 8C is a ROC curve showing the ability of the sICAM-1 (ELISA and LF), CCL23, A1AT, CRP, IL-6 and TNF-α marker combination to distinguish sepsis from SIRS
Figure 9A is a scatter plot showing the ability of the sICAM-1 (ELISA and LF), CCL23, A1AT, CRP, IL-6 and TNF-α marker combination to distinguish SIRS from controls
Figure 9B is a box plot showing the ability of the sICAM-1 (ELISA and LF), CCL23, A1AT, CRP, IL-6 and TNF-α marker combination to distinguish SIRS from controls
Figure 9C is a ROC curve showing the ability of the sICAM-1 (ELISA and LF), CCL23, A1AT, CRP, IL-6 and TNF-α marker combination to distinguish SIRS from controls
Figure 10A is a scatter plot showing the ability of the sICAM-1 (ELISA and LF), CCL23, A1AT, CRP, IL-6 and TNF-α marker combination to distinguish sepsis from controls
Figure 10B is a box plot showing the ability of the sICAM-1 (ELISA and LF), CCL23, A1AT, CRP, IL-6 and TNF-α marker combination to distinguish sepsis from controls
Figure 10C is a ROC curve showing the ability of the sICAM-1 (ELISA and LF), CCL23, A1AT, CRP, IL-6 and TNF-α marker combination to distinguish sepsis from controls
Figure 11A shows levels of CRP over time in sepsis versus controls
Figure 11B shows levels of IL-6 over time in sepsis versus controls
Figure 11C shows levels of TIMP1 over time in sepsis versus controls
Figure 11D shows levels of sICAM-1 over time in sepsis versus controls
Figure 12 shows a point of care lateral flow device useful in the present invention
Figure 13 sets out a possible personalised testing strategy that may be adopted according to the invention
Figure 14 is a box plot showing the ability of sRAGE to distinguish sepsis from controls in the tested samples
Figure 15A is a schematic representation of an ELISA assay format for measuring levels of CCL23 in a sample
Figure 15B shows a representative calibration curve for the ELISA assay format for measuring levels of CCL23 in a sample
Figure 16 is a schematic representation of a lateral flow assay format for measuring levels of CCL23 in a sample
Figure 17A is a schematic representation of an ELISA assay format for measuring levels of CRP in a sample
Figure 17B shows a representative calibration curve for the ELISA assay format for measuring levels of CRP in a sample
Figure 18A is a schematic representation of a lateral flow assay format for measuring levels of CRP in a sample
Figure 18B shows a representative calibration curve for the lateral flow assay format for measuring levels of CRP in a sample
Figure 19A is a schematic representation of an ELISA assay format for measuring levels of A1AT in a sample
Figure 19B shows a representative calibration curve for the ELISA assay format for measuring levels of A1AT in a sample
Figure 20A is a schematic representation of a lateral flow assay format for measuring levels of A1AT in a sample
Figure 20B shows a representative calibration curve for the lateral flow assay format for measuring levels of A1AT in a sample
Figure 21A is a schematic representation of an ELISA assay format for measuring levels of TNFalpha in a sample
Figure 21B shows a representative calibration curve for the ELISA assay format for measuring levels of TNFalpha in a sample
Figure 22A is a schematic representation of a lateral flow assay format for measuring levels of TNFalpha in a sample
Figure 22B shows a representative calibration curve for the lateral flow assay format for measuring levels of TNFalpha in a sample
Figure 23A is a schematic representation of an ELISA assay format for measuring levels of IL-6 in a sample
Figure 23B shows a representative calibration curve for the ELISA assay format for measuring levels of IL-6 in a sample
Figure 24A is a schematic representation of a lateral flow assay format for measuring levels of IL-6 in a sample
Figure 24B shows a representative calibration curve for the lateral flow assay format for measuring levels of IL-6 in a sample
Figure 25A is a schematic representation of an ELISA assay format for measuring levels of PLA2G2A in a sample
Figure 25B shows a representative calibration curve for the ELISA assay format for measuring levels of PLA2G2A in a sample
Figure 26A is a schematic representation of a lateral flow assay format for measuring levels of PLA2G2A in a sample
Figure 26B shows a representative calibration curve for the lateral flow assay format for measuring levels of PLA2G2A in a sample
Figure 27A is a schematic representation of an ELISA assay format for measuring levels of sICAM1 in a sample
Figure 27B shows a representative calibration curve for the ELISA assay format for measuring levels of sICAM1 in a sample
Figure 28A is a schematic representation of a lateral flow assay format for measuring levels of sICAM1 in a sample
Figure 28B shows a representative calibration curve for the lateral flow assay format for measuring levels of sICAM1 in a sample
Figure 29 shows a decision tree analysis of sepsis markers.
Figure 30 shows diagnostic performance of the LR1 markers in terms of distinguishing the infection +SIRS group from the SIRS only group. Figure 30A is a scatter plot and Figure 30B is a ROC curve.
Figure 31 shows diagnostic performance of the LR2 markers in terms of distinguishing the infection +SIRS group from the SIRS only group based on patients with SOFA scores of 2-6. Figure 31A is a scatter plot and Figure 31B is a ROC curve.
Figure 32 shows diagnostic performance of the LR2 markers in terms of distinguishing the infection +SIRS group from the SIRS only group across all patients, irrespective of SOFA score. Figure 32A is a scatter plot and Figure 32B is a ROC curve.
Figure 33 shows diagnostic performance of the LR3 markers in terms of distinguishing high SOFA score from low SOFA score in the infected group to indicate severity (sepsis). Figure 33A is a scatter plot and Figure 33B is a ROC curve.
Figure 34 is a scatter plot showing that the LR2 algorithm was able to positively identify the infected patients in the validation set (based on day 0 samples).
Figure 35 is a timecourse showing that the LR2 algorithm could also be used to discriminate survivors from non-survivors.

### EXAMPLES

The invention will be further understood with reference to the following experimental examples.

### EXAMPLE 1 - Improving on CRP for sepsis diagnosis

### Summary

### Background

Although many new biomarkers for sepsis diagnosis have been investigated, to be useful in clinical practice, and to improve sensitivity and specificity it may be necessary to combine them with traditional markers, particularly C-reactive protein (CRP). This study compared the diagnostic accuracy of CRP used alone and in combination with selected complementary markers for the diagnosis of sepsis.

### Methods

One hundred and two patients diagnosed with sepsis based on strict clinical criteria including positive blood cultures (51 with Gram-positive, 49 Gram-negative and 2 mixed Gram-positive and Gram-negative microorganisms) were compared to 102 patients with no evidence of sepsis. Serum levels were measured for CRP and procalcitonin (PCT), as well as seven selected potential markers. These comprised: two inflammatory cytokines, interleukin-6 (IL-6) and tumour necrosis factor alpha (TNFα); the vascular marker soluble intercellular adhesion molecule (sICAM-1); a chemokine, C-C motif ligand 23 (CCL23, also known as macrophage inflammatory protein 3, MIP-3); secreted type IIA phospholipase (sPLA2g2a); matrix metalloprotease 8 (MMP8) and the serpin protease inhibitor, α-1 antitrypsin (A1AT). The diagnostic accuracy of each marker alone was evaluated by receiver operator curve (ROC). Combinations of markers giving improved diagnostic performance were identified by logistic regression. Cut-off values and diagnostic algorithms for marker combinations were developed by classification and regression tree analysis.

### Findings

When markers were measured following confirmation of sepsis by positive blood culture, the accuracy of CRP for sepsis diagnosis was superior to that of the other markers investigated. sPLA2g2a was as sensitive as CRP, but its specificity was lower. Combining levels of sICAM-1 with CRP improved diagnostic accuracy of CRP alone. When levels were measured earlier, at the time the patient presented with symptoms of sepsis, sPLA2g2a displayed diagnostic accuracy equivalent to that of CRP. An increase in the diagnostic accuracy of CRP could be achieved by combining measurement of CRP with sPLA2g2a.

### Interpretation

Potential exists for improving the diagnostic accuracy of CRP in sepsis by combining measurement of its level with those of sPLA2g2a and sICAM-1. This approach would have value in supporting choice of treatment options prior to confirmation by culture and in culture-negative cases where sepsis is suspected.

### Introduction

Early and accurate diagnosis of sepsis is essential for initiation of appropriate therapy. C-reactive protein (CRP) and procalcitonin (PCT) are the most widely used markers, but they have limited ability to distinguish between sepsis and systemic inflammatory response due to non-infectious causes. Many other serum biological markers have been evaluated for this purpose, including pro- and anti-inflammatory cytokines, chemokines, cell receptors for microbial toxins and cellular adhesion molecules.^{1,2} The underlying pathobiology of sepsis is poorly understood and, owing to potential involvement of many organ systems, finding a specific marker that is reproducibly quantifiable in all septic patients has not been achieved. Currently, very few serum-based tests are routinely used in the diagnosis of sepsis. CRP, and to a lesser extent PCT, are used in the diagnosis of sepsis but are without the high levels of sensitivity and specificity that are required to fully/confidently support clinical decisions. When considering a pathology such as sepsis that has heterogeneous clinical presentations, the sensitivity and specificity of CRP and PCT might be improved by combination with other biological markers, reflecting different aspects of the host response to infection.^{3,4}

In this study we investigated whether combining a number of potential markers with CRP would improve its diagnostic accuracy in patients with blood culture-confirmed systemic infection, using a group of age- and sex-matched hospital outpatients free from infection as controls. The study was designed to select the optimum combination of markers, to establish the cut-off levels for interpretation and re-assess their accuracy for the early diagnosis of sepsis. Serum was collected following patient consent and confirmation of systemic infection by a positive blood culture and, for a subgroup of patients, sera were also analysed when symptoms of sepsis were first observed or when a septic patient presented to our hospital. The markers studied comprised two cytokines: IL6 5 and TNFα;^{5,6} a chemokine (CCL23, also known as MIP3);⁷ the soluble intercellular adhesion molecule (sICAM1) reflecting the vascular endothelial activation in infection;⁸ the tissue-degrading enzymes, matrix metalloprotease 8 (MMP8) and secreted type IIA phospholipase A2, PLA2g2a;^{9,10} and the protease inhibitor, α1-antitrypsin (A1AT).¹¹ The use of each marker in sepsis diagnosis has been previously reviewed by Pierrakos and Vincent.¹

Here, diagnostic accuracy of each marker was determined by area under the receiver operator curve (AUROC), sensitivity and specificity. Potential for improving diagnostic accuracy by combining markers was explored by logistic regression analysis. Effective cut-off levels and their application in diagnostic algorithms were investigated by classification and regression tree analysis. We examined the levels of each marker in serum collected following patient consent and confirmation of sepsis by a positive blood culture. To investigate the value of the markers in detecting sepsis at an early stage, we also measured marker levels in serum taken at intervals of one to eight days (median four days) prior to the time that symptoms of sepsis presented. This was carried out for 44 patients where surplus samples were available from measurement of other clinical parameters.

### Methods

### Patients

Serum samples were obtained from evaluable adult patients who had been suffering from SIRS.¹² All the patients had been acutely unwell for a duration of no more than five days and had positive blood cultures. All the patients' clinical details were reviewed independently by two Consultant Microbiologists (TE and MD) at the University Hospitals Birmingham NHS Foundation Trust and the blood culture isolates were considered to represent significant bacteraemia. Sepsis was defined as SIRS in the presence of a confirmed infection. Criteria for SIRS were temperature <36·6°C or ≥38°C and white cell count <4·0 x 10⁹/L or >12·0 x 10⁹/L. Bacteremia was confirmed in all sepsis patients by positive blood cultures. The causative microorganism was identified by Gram-staining and subculture onto appropriate media followed by routine biochemical identification tests. Serum samples were collected from 98 patients following patient consent and confirmation of sepsis by a positive blood culture. In addition, sera were obtained from 44 patients at various time intervals during the course of their sepsis. These were samples that had been taken for analysis of other blood markers as part of their routine clinical management. Samples were collected on the day of onset of symptoms or admittance to hospital (when the initial blood samples were taken for culture). Controls comprised an age- and sex-matched group of patients attending an ophthalmic outpatient clinic at UHBFT. None had any evidence of sepsis in the preceding 12 weeks and no evidence of an inflammatory condition nor immunosuppression.

### Study approvals

Research Ethics committee (NRES West Midlands - Coventry and Warwickshire, REC ref: 12/WM/0251) and R&D Department (The University Hospitals Birmingham NHS Foundation Trust) approvals were obtained prior to commencing the study. Informed written consent was received from all the study patients prior to participation in this study.

### Marker assays

Serum levels for IL-6, TNFα, sICAM-1, CCL23 and MMP8 were determined in triplicate using commercial ELISA kits (Duosets, R&D Systems, Abingdon, UK). PCT, PLA2 and A1AT were measured in triplicate by ELISA using assays developed by Mologic Ltd. CRP levels were determined by particle-enhanced immunoturbidimetry (Roche Diagnostics, Burgess Hill, West Sussex, UK) by Clinical Biochemistry at University Hospitals Birmingham NHS Foundation Trust.

### Statistical analysis

Descriptive statistics were calculated using Prism vs6 (GraphPad). The D'Agostino-Pearson omnibus normality test was used to check whether marker values followed a Gaussian distribution. The diagnostic accuracy of each marker to distinguish between sepsis and controls was determined as the area under the receiver operator curve (AUROC, sensitivity vs 1-specificity). Optimum cut-off levels for each marker used alone were calculated from the ROC curves at the maximum value of the Youden index (J = sensitivity+specificity-1). Forward stepwise logistic regression was used to investigate the value of combinations of markers in the prediction of sepsis (SPSS vs20.0, IBM). Classification and regression tree analysis was used to construct diagnostic algorithms by sequential application of marker cut-off values (SPSS).

### Results

The sepsis group comprised 102 patients, age range 20-97 years, mean 59·0 years, M/F 52/48. The sources of sepsis were; intravascular (IV) catheters (32), abdominal and biliary (21), renal and urinary (17), skin and soft tissue (15), respiratory (7), non-IV catheter vascular (5) and other/unknown (5). Causative organisms identified from blood were Gram-negative bacteria (51), Gram-positive bacteria (49) and 2 mixed Gram-positive and Gram-negative bacteria (2). Bacterial species are given in Table 1. The control group comprised 102 individuals, age range 23-88 years, mean 60·6 years, M/F 55/47. Median marker levels in sera taken from 98 patients following patient consent and confirmation of sepsis by a positive blood culture, and from controls are shown in Table 2. Because the marker levels were not normally distributed, the significance of the difference in median marker levels between sepsis and controls was determined by the Mann-Whitney U test. Performance of each marker in the diagnosis of sepsis was summarised as the area under the ROC curve (AUROC). The cut-off levels giving optimum diagnostic accuracy for each marker were determined at the maximum value of the Youden index on the ROC curves, sensitivity and specificity at this cut-off are listed in Table 2. Individually, CRP gave the clearest diagnosis of sepsis, followed by sPLA2g2a and sICAM-1, each marker being more accurate than PCT in terms of AUROC, sensitivity and specificity. IL6 and CCL23 gave poor specificity and sensitivity respectively, whilst the other markers were of very limited diagnostic value. To investigate whether different diagnostic performance would be obtained from samples taken earlier in the septic episode, marker levels were measured in sera at the time of the onset of symptoms of sepsis. These marker levels were compared with the marker levels from the serum taken following patient consent and confirmation of sepsis by a positive blood culture. Only IL6, PLA2g2a, TNFα and CCL23 showed significant differences (Mann Whitney p values <0·0001, 0·0191, 0·0384 and 0·0025 respectively), suggesting that these markers might provide better diagnostic accuracy when measured in samples taken early in the course of sepsis. Diagnostic accuracy of each of the markers was therefore assessed as before, using the earlier time samples for the 44 patients and 102 controls. The results are shown in Table 3. The median time between the early samples (Table 3) and those taken when blood cultures were positive (Table 2) was four days (mean = 4·038, SD = 1·311, SEM = 0·2571), minimum one day (one patient), maximum eight days (one patient), 25% percentile 3.75 days, 75% percentile five days. CRP and PLA2g2a showed the best diagnostic accuracy in terms of sensitivity at the optimum cut-off. Comparing the data in Tables 2 and 3 shows a notable improvement in diagnostic accuracy of IL6 and CCL23 when their levels are measured at the earlier stage of sepsis. This indicates their early production and subsequent reduction during the course of sepsis.

The value of combining markers with CRP to improve its diagnostic performance was explored by binary logistic regression. Logistic regression models were derived by entering each marker separately with CRP. The predictive performance of each model was then compared with that of CRP alone (Table 4). Using the marker levels from serum following patient consent and confirmation of sepsis by a positive blood culture, only sICAM-1 provided a logistic regression model that improved on the performance of CRP alone in both sensitivity and specificity. Whereas CRP alone correctly identified 92/98 sepsis patients and 99/102 controls, combination of sICAM-1 with CRP in the logistic regression model correctly identified 95/98 sepsis and 100/102 controls. When all markers were submitted to forward stepwise (likelihood ratio) logistic regression a model giving 96% sensitivity and 99% specificity was produced, based on combination of CRP, sICAM-1, IL6 and sPLA2g2a. Using the same approach for marker levels measured at the onset of sepsis symptoms, combination of PLA2 with CRP gave the greatest improvement in performance. Addition of this marker improved the diagnostic performance of CRP from 40/44 sepsis patients and 101/102 controls correctly identified to 43/44 sepsis patients and 101/102 controls. Separate combinations of either PCT, sICAM-1, IL6 or CCL23 with CRP also improved sensitivity from 40/44 sepsis to 41/44 without loss of specificity. When all markers were submitted to forward stepwise (likelihood ratio) logistic regression a model was produced based on combination of CRP, sICAM-1, IL6 and sPLA2g2a.

Combination of markers with CRP in diagnostic algorithms was also investigated by classification and regression tree analysis (CART). For the marker levels taken following patient consent and confirmation of sepsis by a positive blood culture, where combination of sICAM-1 with CRP improved the sensitivity by logistic regression, the initial application of sICAM-1 at a cut-off level of 302 µg/L correctly identified 68/98 sepsis patients with no false positives (i.e. no controls above this cut-off level). Application of a CRP cut-off level of 20·5 mg/L to the remaining samples (those with sICAM-1 levels below 302 ng/L, comprising 32 sepsis patients and 102 controls) correctly identified a further 29/32 sepsis patients with one false positive, leaving 101/102 controls with marker levels below the cut-off values for both markers. Overall sensitivity for this group of samples was therefore 99% with 99% specificity, compared with 92% and 97% for CRP alone. For the smaller number of samples analysed at the time of onset of sepsis symptoms, where logistic regression showed sPLA2g2a to improve the accuracy of CRP, CART analysis produced an algorithm involving application of CRP at a cut-off level of 16·5 mg/L, followed by sPLA2g2a at a cut-off of 57·6 µg/L to those samples falling below this CRP cut-off. This algorithm correctly identified 42/44 sepsis patients with CRP above the cut-off level and three false positives. Application of the PLA2 cut-off to those samples falling below the CRP cut-off correctly identified the two remaining sepsis patients with no additional false positives. The overall sensitivity was therefore 100% with a specificity of 97%, compared with 95% and 97% respectively with CRP alone.

When marker levels in patients with sepsis caused by Gram-positive and Gram-negative bacteria were compared, only CRP showed a significant difference in median levels. For the early onset samples, the CRP median was 113 mg/L (range 3-547) for Gram-positive sepsis (30), compared with a median CRP of 46 mg/L (range 4-42) for Gram-negative sepsis (12) (p=0.0005, Mann-Whitney U test). For samples taken following patient consent and confirmation of sepsis by a positive blood culture, the CRP median levels were: 112 mg/L (range 7-550) for Gram-positive sepsis (49), compared with 69 mg/L (range 5-278) for Gram-negative sepsis (48) (p=0·0102). None of the other markers showed a significant difference between marker levels for Gram-positive and Gram-negative sepsis.

**Table 1: Microorganisms responsible for sepsis in study patients (n=102).**

| **Gram-positive (n=51)** | **Gram-negative (n=49)** | **Mixed Gram-negative and -positive infection (n=2)** |
|---|---|---|
| *Staphylococcus aureus* (25) | *Escherichia coli* (28) | *E. coli* and *E*. *faecium* (1) |
| 'Viridans' streptococci (5) | *Pseudomonas aeruginosa (6)* | *P. aeruginosa* and 'viridans' |
| Coagulase-negative staphylococci (4) | *Klebsiella oxytoca (3)* | streptococci (1) |
| *Streptococcus pneumoniae* (4) | *Stenotrophamonas maltophilia* (3) | |
| *Streptococcus pyogenes* (4) | *Enterobacter aerogenes* (1) | |
| *Enterococcus faecium* (1) | *Acinetobacter ursingii* (1) | |
| *Enterococcus gallinarum* (1) | *Klebsiella pneumoniae* (1) | |
| *Streptococcus agalactiae* (2) | *Serratia marcescens* (1) | |
| *Group G Streptococci* (2) | *Bacillus fragilis* (1) | |
| *Streptococcus constellatus* (1) | *Salmonella* sp. (1) | |
| *Cardiobacterium hominis (*1) | *E. coli* and *K. oxytoca* (1) | |
| Coagulase-negative staphylococci | *E. coli* and *K. pneumoniae* (1) | |
| and *Enterococcus faecalis* (1) | *K. pneumoniae* and *Enterobacter cloacae* (1) | |

**Table 2: Marker levels in sera from patients with sepsis (n=98) and healthy controls (n=102). Markers were measured in sera taken following patient consent and confirmation of sepsis by a positive blood culture.**

| **Marker** | **group** | **min** | **25% percentile** | **median** | **75% percentile** | **max** | **p** | **AUROC** | **sensitivity, specificity (cut-off)** |
|---|---|---|---|---|---|---|---|---|---|
| CRP mg/L | control | 0 | 0 | 0 | 3 | 29 | <0·0001 | 992 | 94%, 97% (18 mg/L) |
| | sepsis | 5 | 41·5 | 96·5 | 162·3 | 550 | | | |
| PCT µg/L | control | 0 | 0 | 0 | 0 | 2 | <0·0001 | 810 | 72%, 86% (0·6 ng/L) |
| | sepsis | 0 | 0 | 2 | 6.5 | 75 | | | |
| sICAM-1 µg/L | control | 0 | 129·5 | 174·5 | 215·5 | 299 | <0·0001 | 883 | 85%, 87% (245 µg/L) |
| | sepsis | 0 | 265 | 374·5 | 545 | 1039 | | | |
| IL6 ng/L | control | 0 | 0 | 3 | 21 | 982 | <0·0001 | 772 | 84%, 67% (11 ng/L) |
| | sepsis | 0 | 15·25 | 39·5 | 124 | 1779 | | | |
| sPLA2g2a µg/L | control | 0 | 8 | 14 | 22 | 80 | <0·0001 | 959 | 94%, 88% (32 µg/L) |
| | sepsis | 8 | 47 | 102·5 | 266·8 | 806 | | | |
| A1AT µg/L | control | 0 | 782 | 1360 | 2152 | 5831 | 0·0016 | 628 | 66%, 49% (1314 mg/L) |
| | sepsis | 0 | 913 | 2028 | 3927 | 7989 | | | |
| TNF ng/L | control | 0 | 0 | 0 | 27 | 2857 | 0·0127 | 590 | 50%, 69% (3·2 ng/L) |
| | sepsis | 0 | 0 | 0·5 | 154 | 2764 | | | |
| CCL23 ng/L | control | 0 | 75 | 294 | 491 | 2969 | <0·0001 | 720 | 56%, 81% (570 ng/L) |
| | sepsis | 0 | 282·5 | 716 | 1374 | 7684 | | | |
| MMP8 µg/L | control | 0 | 6·25 | 11 | 18·75 | 49 | 0·0031 | 623 | 63%, 58% (12·5 µg/L) |
| | sepsis | 0 | 6 | 17 | 37·5 | 43 | | | |

| | | | | | | | | | |
|---|---|---|---|---|---|---|---|---|---|
| p = probability, Mann-Whitney *U* test. AUROC = area under the receiver operator curve (sensitivity vs 1-specificity). Sensitivity and specificity determined at the optimum cut-off (maximum Youden index on ROC curve). | | | | | | | | | |

**Table 3: Marker levels and diagnostic accuracy in sera from patients with sepsis (n=44) taken at the time blood was taken for culture, i.e. the time of symptom onset vs healthy controls (n=102)**

| **Marker** | **group** | **min** | **25% percentile** | **median** | **75% percentile** | **max** | **p** | **AUROC** | **sensitivity, specificity (cut-off)** |
|---|---|---|---|---|---|---|---|---|---|
| CRP mg/L | control | 0 | 0 | 0 | 3 | 29 | <0·0001 | 988 | 95%, 98% (17·5 mg/L) |
| | sepsis | 3 | 60 | 104 | 200 | 547 | | | |
| PCT µg/L | control | 0 | 0 | 0 | 0 | 2 | <0·0001 | 801 | 69%, 81% (0·5 ng/L) |
| | sepsis | 0 | 0 | 2 | 6·5 | 80 | | | |
| sICAM-1 µg/L | control | 0 | 129·5 | 174·5 | 215·5 | 299 | <0·0001 | 922 | 73%, 96% (284 µg/L) |
| | sepsis | 146 | 253·5 | 408 | 581 | 1059 | | | |
| IL6 ng/L | control | 0 | 0 | 3 | 21 | 982 | <0·0001 | 928 | 98%, 78% (28·5ng/L) |
| | sepsis | 0 | 113 | 395 | 1200 | 1664 | | | |
| sPLA2g2a µg/L | control | 0 | 8 | 14 | 22 | 80 | <0·0001 | 994 | 96%, 99% (54·5 ng/L) |
| | sepsis | 37 | 63 | 145 | 523·5 | 899 | | | |
| A1AT µg/L | control | 0 | 782 | 1360 | 2152 | 5831 | <0·0001 | 768 | 76%, 70% (1883 mg/L) |
| | sepsis | 0 | 1764 | 2788 | 4446 | 10048 | | | |
| TNFα ng/L | control | 0 | 0 | 0 | 27 | 2857 | <0·0001 | 692 | 60%, 79% (49 ng/L) |
| | sepsis | 0 | 0 | 64 | 263 | 2014 | | | |
| CCL23 ng/L | control | 0 | 75 | 294 | 491 | 2969 | <0·0001 | 911 | 89%, 81% (599 ng/L) |
| | Sepsis | 77 | 978 | 1833 | 3422 | 6271 | | | |
| MMP8 µg/L | control | 0 | 6·25 | 11 | 18·75 | 49 | 0·0011 | 686 | 50%, 95% (27·5 µg/L) |
| | sepsis | 0 | 8·5 | 31 | 40 | 45 | | | |

| | | | | | | | | | |
|---|---|---|---|---|---|---|---|---|---|
| p = probability, Mann-Whitney U test. AUROC = area under the receiver operator curve (sensitivity vs 1-specificity). Sensitivity and specificity determined at the optimum cut-off (maximum Youden index on ROC curve). | | | | | | | | | |

**Table 4: Sensitivity and specificity of CRP used alone or in combination with other markers in serum samples taken following patient consent and confirmation of sepsis by a positive blood culture and at the time of symptom onset.**

| **Marker combinations** | **Marker levels at time blood cultures were positive (n=98 sepsis, 102 controls)** | | **Marker levels at time of symptom onset (n=44 sepsis, 102 controls)** | |
|---|---|---|---|---|
| | **Sensitivity%** | **Specificity%** | **Sensitivity%** | **Specificity%** |
| CRP alone | 94 | 97 | 91 | 99 |
| CRP + PCT | 95 | 97 | 95 | 99 |
| CRP + sICAM-1 | 97 | 98 | 93 | 99 |
| CRP + IL6 | 94 | 97 | 93 | 98 |
| CRP + sPLA2g2a | 96 | 97 | 98 | 99 |
| CRP + A1AT | 96 | 97 | 91 | 99 |
| CRP + TNF | 94 | 97 | 91 | 99 |
| CRP + CCL23 | 94 | 97 | 93 | 98 |
| CRP + MMP8 | 94 | 97 | 91 | 99 |

### Discussion

When markers were measured at the time following patient consent and confirmation of sepsis by a positive blood culture, the accuracy of CRP for sepsis diagnosis was superior to that of the other markers investigated, including PCT. sPLA2g2a was as sensitive as CRP, but its specificity was lower. When levels were measured earlier, at the time of onset of symptoms/admittance of patient to hospital, sPLA2g2a displayed diagnostic accuracy equivalent to that of CRP. Using both logistic regression and CART, we showed that an increase in the diagnostic accuracy of CRP could be achieved by combining measurement of CRP with sPLA2g2a. When measured at the time of onset of symptoms/admittance of patient to hospital, combination of PLA2 and CRP levels in a logistic regression model gave 100% sensitivity and 100% specificity. Applying CART analysis to the combination we generated a diagnostic algorithm involving the application of CRP at a cut-off of 16·5 mg/L followed by sPLA2g2a at a cut-off of 57·6 µg/L. This gave an accuracy of 100% sensitivity and 97% specificity. If these two markers were measured at the time blood is taken for culture, their interpretation, either by the logistic regression model or the diagnostic algorithm, could provide support for patient management by predicting the blood culture result as either positive or negative. Most importantly, prediction of a negative blood culture result might support a decision to withhold antibiotic treatment and thereby avoid unnecessary therapy reducing the risk of emerging multi antimicrobial resistant microorganisms. Other markers identified for potential combination with CRP are PCT and sICAM-1, both of which improved the sensitivity of CRP without compromising its selectivity in the early samples. For serum samples taken following patient consent and confirmation of sepsis by a positive blood culture, sICAM-1 gave a greater improvement in the accuracy of CRP than either sPLA2g2a or PCT when used in combination with CRP. If a single additional marker were to be chosen for combination with CRP, sICAM-1 would be recommended for application to samples taken over the time course of sepsis before confirmation by positive blood culture. In practice, many blood cultures taken from patients with sepsis are negative due to prior use of antibiotics or inadequate blood volume sampling. In such cases, positive prediction of infection from a marker combination would also support initiation or continuation of antibiotic therapy. Similarly, the use of such combinations of markers may identify when positive blood cultures represent contamination rather than true sepsis. This study points the way to those markers that have greatest potential for combination with CRP. Clearly, the combination of sPLA2g2a, sICAM-1 or PCT as complementary markers with CRP must be rigorously tested in multicentre studies involving other patient groups, including sepsis of fungal aetiology and non-infectious conditions causing elevated inflammatory and vascular markers. Assays for sPLA2g2a and sICAM-1 could easily be adapted for use in a routine clinical laboratory.

### References

1. Pierrakos C, Vincent J-L. Sepsis biomarkers: a review. Crit Care 2010; 14: R15.
2. Tsalik EL, Jaggers LB, Glickman SW, et al. Discriminatory value of inflammatory biomarkers for suspected sepsis. J Emerg Med 2012; 43: 97-106.
3. Gaini S, Koldjaer OG, Pedersen C, Pedersen SS (2006). Procalcitonin, lipopolysaccharide-binding protein, interleukin-6 and C-reactive protein in community-acquired infections and sepsis: a prospective study. Crit Care 2006; 10: R53.
4. Xing K, Murthy S, Liles WC, Singh JM. Clinical utility of biomarkers of endothelial activation in sepsis-a systematic review. Critical Care 2012 ; 16: R7.
5. Uusitalo-Seppälä R, Koskinen P, Leino A, Peuravuori H, Vahlberg T, Rintala EM. Early detection of severe sepsis in the emergency room: diagnostic value of plasma C-reactive protein, procalcitonin, and interleukin-6. Scand J Infect Dis 2011; 43: 883-90.
6. Mera S, Tatulescu D, Cismaru C, et al. (2011). Multiplex cytokine profiling in patients with sepsis. Acta Path Microbiol Immunol Scand 2011; 119: 155-63.
7. Ginde AA, Blatchford PJ, Trzeciak S, et al. (2014). Age-related differences in biomarkers of acute inflammation during hospitalization for sepsis. Shock 2014; 42: 99-107.
8. Kung CT, Hsiao SY, Su CM, et al. Serum adhesion molecules as predictors of bacteremia in adult severe sepsis patients at the emergency department. Clinica Chimica Acta. 2013; 421: 116-20.
9. Yazdan-Ashoori P, Liaw P, Toltl L, et al. Elevated plasma matrix metalloproteinases and their tissue inhibitors in patients with severe sepsis. J Crit Care 2011; 26:556-65.
10. Rintala EM, Aittoniemi J, Laine S, Nevalainen TJ, Nikoskelainen J. Early identification of bacteremia by biochemical markers of systemic inflammation. Scand J Clin Lab Invest 2001; 61: 523-30.
11. Bossink AW, Groeneveld AB, Thijs LG. Prediction of microbial infection and mortality in medical patients with fever: plasma procalcitonin, neutrophilic elastase-alpha1-antitrypsin, and lactoferrin compared with clinical variables. Clinical Infectious Diseases. 1999; 29: 398-407.
12. Bone RC, Balk RA, Cerra FB, et al. Definitions for sepsis and organ failure and guidelines for the use of innovative therapies in sepsis. The ACCP/SCCM Consensus Conference Committee. American College of Chest Physicians/Society of Critical Care Medicine. 1992. Chest 2009; 136(5suppl): e28.

### EXAMPLE 2 - UHB STUDY AND RESULTS

Clinical samples made available from a clinical study performed by University Hospital Birmingham (UHB) were tested. They were categorised according to the time of sample:
A = admission/onset of symptoms
B = time blood culture (BC) taken *values in bold represent (Number of A/B samples = 44)
C = time BC positive = 32 samples
D = time of consent (large volume) = 88 samples
E = last sample prior to discharge =18 samples
Controls = 102 samples

They were tested using the following assays:

| **Marker** | **Assay Type** | **Format** | **Supplier** |
|---|---|---|---|
| CRP | ELISA | Sandwich | R&D Systems |
| CRP | Lateral flow | Sandwich | Mologic |
| sICAM1 | ELISA | Sandwich | R&D Systems |
| sICAM1 | Lateral flow | Sandwich | Mologic |
| CRP/sICAM1 Duplex | Lateral flow | Sandwich | Mologic |
| IL-6 | ELISA | Sandwich | R&D Systems |
| IL-6 | Lateral flow | Sandwich | Mologic |
| PLA2 | ELISA | Sandwich | Mologic |
| PLA2 | Lateral flow | Sandwich | Mologic |
| IL-6/PLA2 Duplex | Lateral flow | Sandwich | Mologic |
| A1AT | ELISA | Sandwich | Mologic |
| A1AT | Lateral flow | Sandwich | Mologic |
| TNFα | ELISA | Sandwich | R&D Systems |
| TNFα | Lateral flow | Sandwich | Mologic |
| CCL23 | ELISA | Sandwich | R&D Systems |
| Ac-PGPv3 | ELISA | Competition | Mologic |
| MMP activity | Substrate | Fluorescent | Mologic |
| Creatinine | Substrate | Colourmetric | R&D Systems |
| Desmosine | ELISA | Competition | Mologic |
| MMP8 | ELISA | Sandwich | R&D Systems |
| Procalcitonin | ELISA | Sandwich | Abcam |
| Procalcitonin | ELISA | Sandwich | Mologic |

Patient demographics were as follows:

| | All sepsis | | | Gram Negative | | | Gram Positive | | | Control | | |
|---|---|---|---|---|---|---|---|---|---|---|---|---|
| | All (n=105) | Male (n=59) | Female (n=46) | All (n=53) | Male (n=29) | Female (n=24) | All (n=50) | Male (n=103) | Female (n=103) | All (n=103) | Male (n=28) | Female (n=22) |
| Median age | 60 | 64 | 55.5 | 64 | 69 | 60.5 | 54 | 54.5 | 46.5 | 63 | 63 | 65 |
| Min Age | 20 | 28 | 20 | 20 | 20 | 33 | 20 | 28 | 20 | 23 | 29 | 23 |
| Max Age | 97 | 97 | 80 | 88 | 88 | 80 | 97 | 97 | 76 | 88 | 88 | 86 |

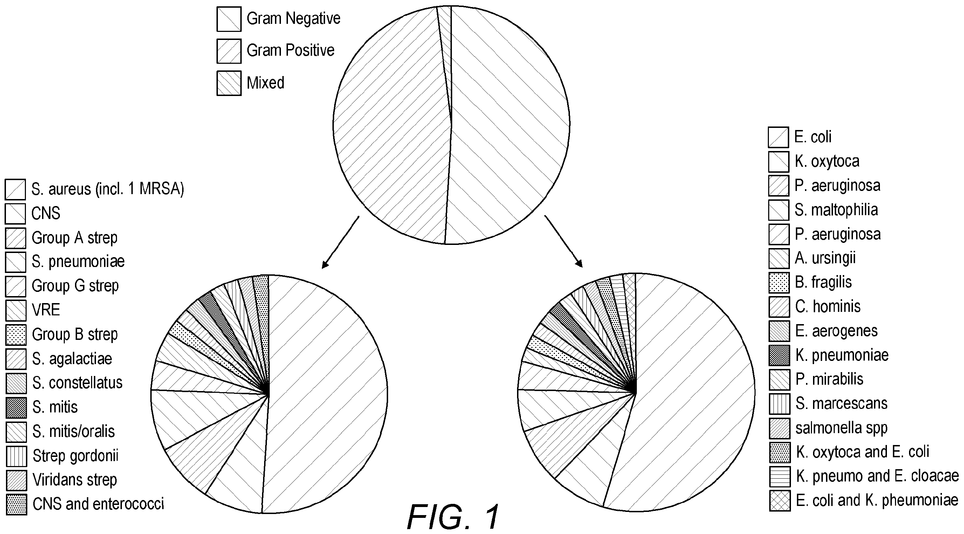

The nature of the infections is shown in more detail in Figure 1.

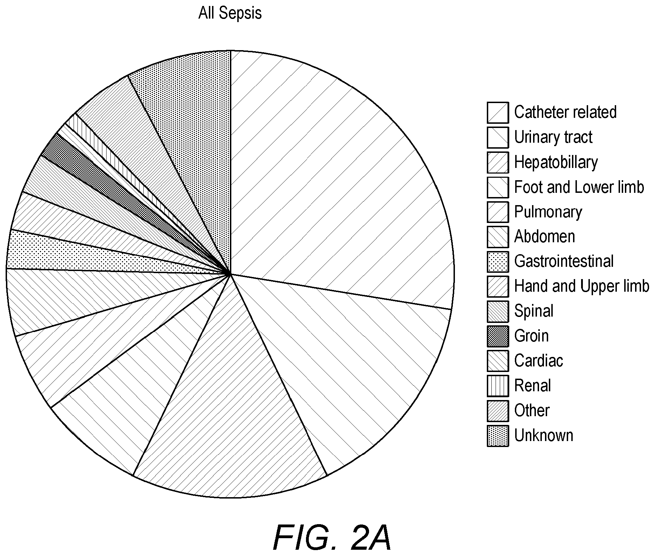

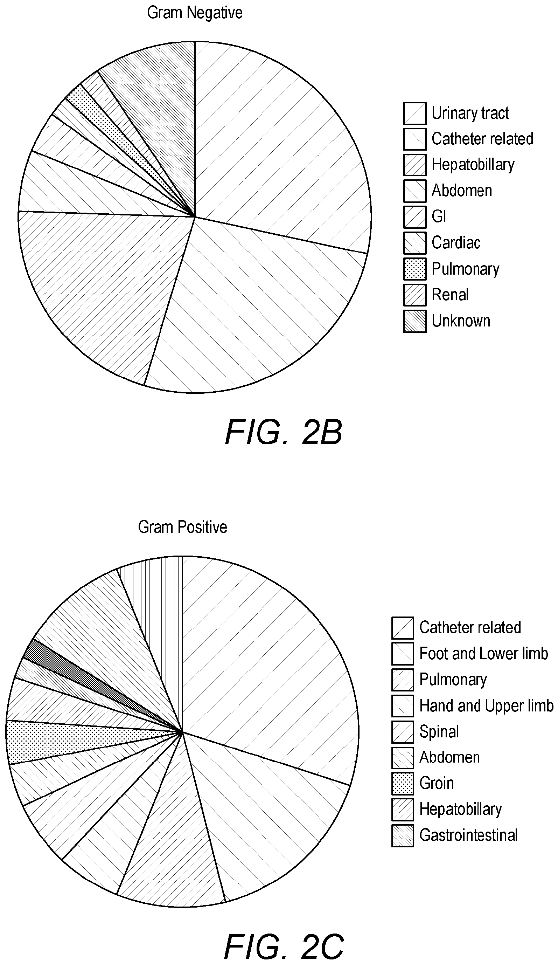

The origins of the infections are summarised in Figure 2A for all sepsis patients and in Figures 2B for gram negative infections and Figure 2C for gram positive infections respectively.

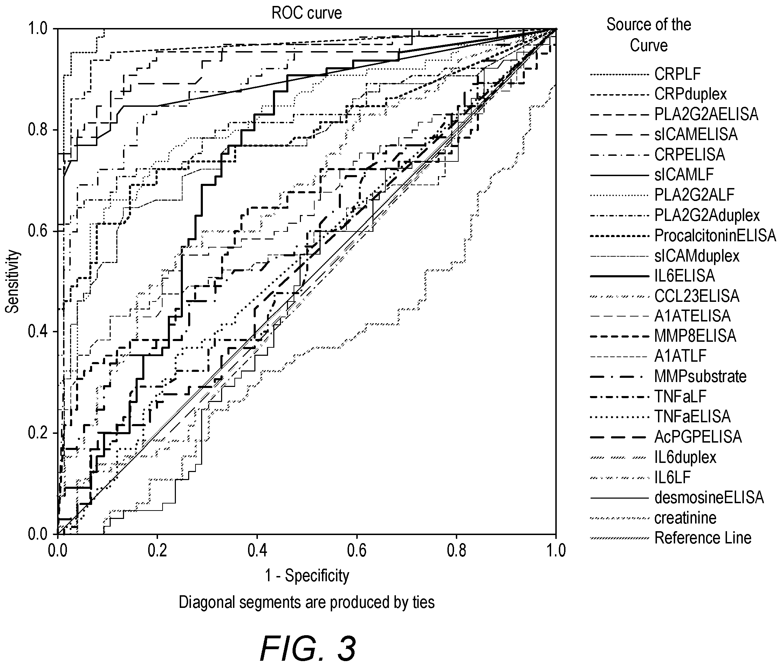

For each assay logistic regression analysis was performed on the D samples versus controls. ROC curves for each of the assays performed are shown in Figure 3. The results are also summarised in the table below.

| **Area Under the Curve** | | Test Result Variable(s) | Area |
|---|---|---|---|
| Test Result Variable(s) | Area | CCL23ELISA | .681 |
| CRPLF | .993 | A1ATELISA | .657 |
| CRPduplex | .964 | MMP8ELISA | .627 |
| PLA2G2AELISA | .950 | A1ATLF | .613 |
| sICAMELISA | .935 | MMPsubstrate | .609 |
| CRPELISA | .900 | TNFaLF | .546 |
| sICAMLF | .897 | TNFaELISA | .541 |
| PLA2G2ALF | .827 | AcPGPELISA | .530 |
| PLA2G2Aduplex | .819 | IL6duplex | .493 |
| ProcalcitoninELISA | .791 | IL6LF | .482 |
| sICAMduplex | .774 | desmosineELISA | .471 |
| IL6ELISA | .731 | creatinine | .354 |

As can be seen, some markers displayed good performance when used alone.

Applying a logistic regression analysis of D samples against control samples, the following markers were selected for use in combination: CRP, sICAM and TNFalpha. The classification table is provided below:

| **Classification Table^{a}** | | | | | |
|---|---|---|---|---|---|
| Observed | | | Predicted | | |
| | | | VAR00001 | | Percentage Correct |
| | | | Control | Sepsis D | |
| Step 1 | VAR00001 | Control | 89 | 2 | 97.8 |
| | | Sepsis D | 5 | 77 | 93.9 |
| | Overall Percentage | | | | 96.0 |
| Step 2 | VAR00001 | Control | 89 | 2 | 97.8 |
| | | Sepsis D | 4 | 78 | 95.1 |
| | Overall Percentage | | | | 96.5 |
| Step 3 | VAR00001 | Control | 90 | 1 | 98.9 |
| | | Sepsis D | 4 | 78 | 95.1 |
| | Overall Percentage | | | | 97.1 |
| a. The cut value is .500 | | | | | |

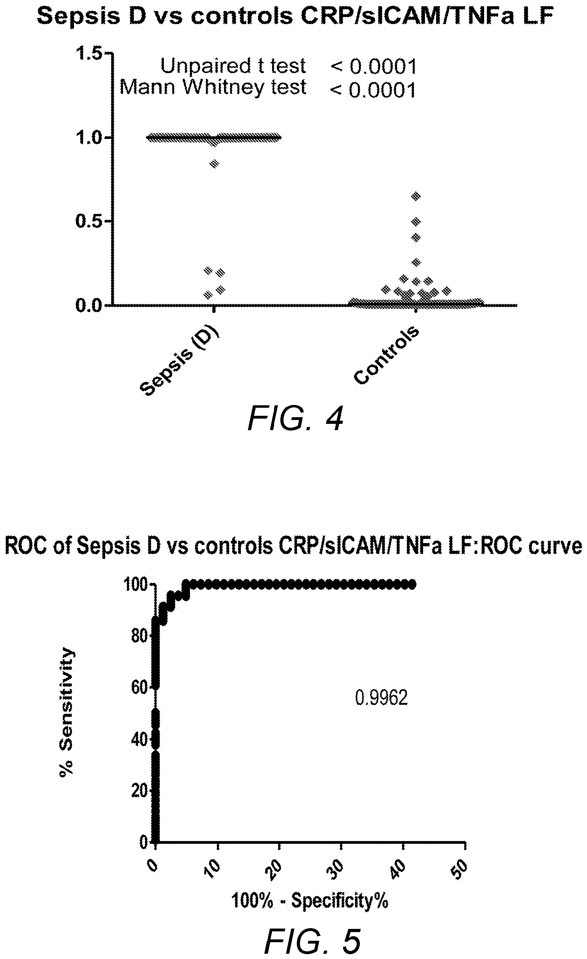

As can be seen from the scatter plot in Figure 4 and the ROC plot in Figure 5, this combination of markers was able to sensitively and specifically detect sepsis (as compared to control samples).

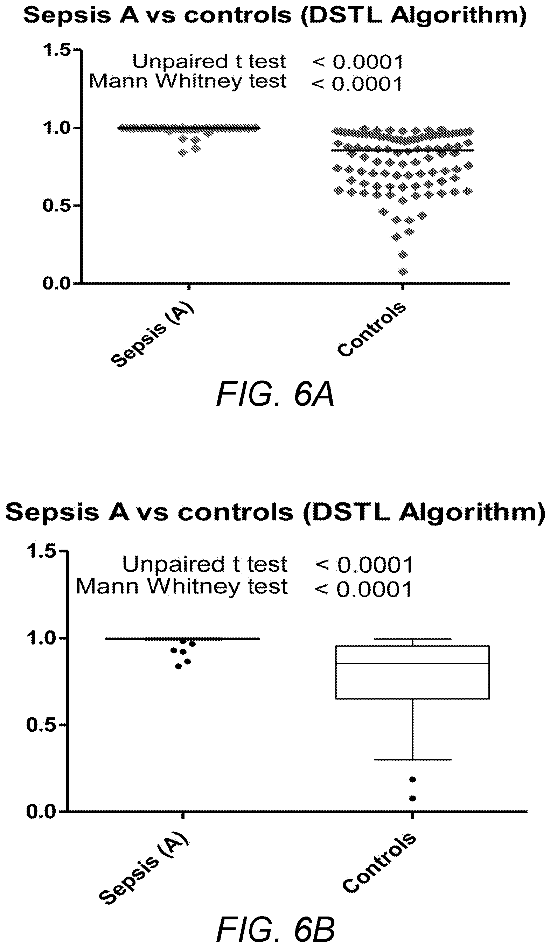

The logistic regression model was also applied to A samples verus controls and the scatter and boxplot results using this model are shown in Figures 6A and 6B respectively.

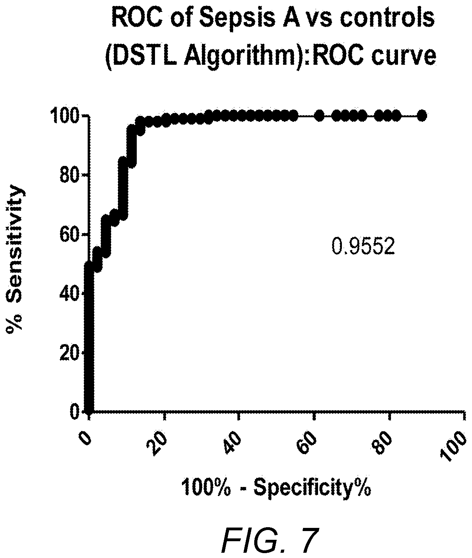

As can be seen from the ROC plot in Figure 7, this combination of markers was also able to sensitively and specifically detect sepsis in the admission/onset of symptoms samples (as compared to control samples).

### EXAMPLE 3 - DSTL STUDY AND RESULTS

910 samples were received and tested on 10 assays at Mologic. The samples were collected from patients admitted for elective surgery and daily samples were collected for up to 7 days post-surgery. The patients were stratified into 3 different groups:
1. Control group (n=70) - those that recovered with no SIRS symptoms
2. SIRS group (n=66) - those that presented with SIRS symptoms within the 7 days
3. Sepsis group (n=70) - those that developed sepsis within the 7 days Some samples were missing where patients refused consent or the research nurses failed to get good venous access, and as for the sepsis group, focus was on days -1, -2 and -3 pre-sepsis diagnosis.

### Differentiation between SIRS and sepsis

Samples were grouped according to DSTL defined SIRS diagnosis based on heart rate, respiratory rate, WCC and temperature and other parameters. The first step was to analyse all samples from patients with sepsis (with SIRS) (n=117) and SIRS (n=173) to come up with the best combination to discriminate between the two groups. The markers identified were sICAM-1 (ELISA and LF), CCL23, A1AT, CRP, IL-6 and TNF-α.

Logistic regression analysis was applied to generate a model for stratifying the patients, as shown in the table below:

| sICAM ELISA/LF, CCL23, A1AT, CRP, IL-6 and TNFα | | | | | |
|---|---|---|---|---|---|
| Observed | | | Predicted | | |
| | | | VAR00001 | | Percentage Correct |
| | | | SIRS | SEPSIS | |
| Step 1 | VAR00001 | SIRS | 147 | 26 | 85.0 |
| | | SEPSIS | 26 | 91 | 71.8 |
| | Overall Percentage | | | | 82.1 |
| a. The cut value is .500 | | | | | |

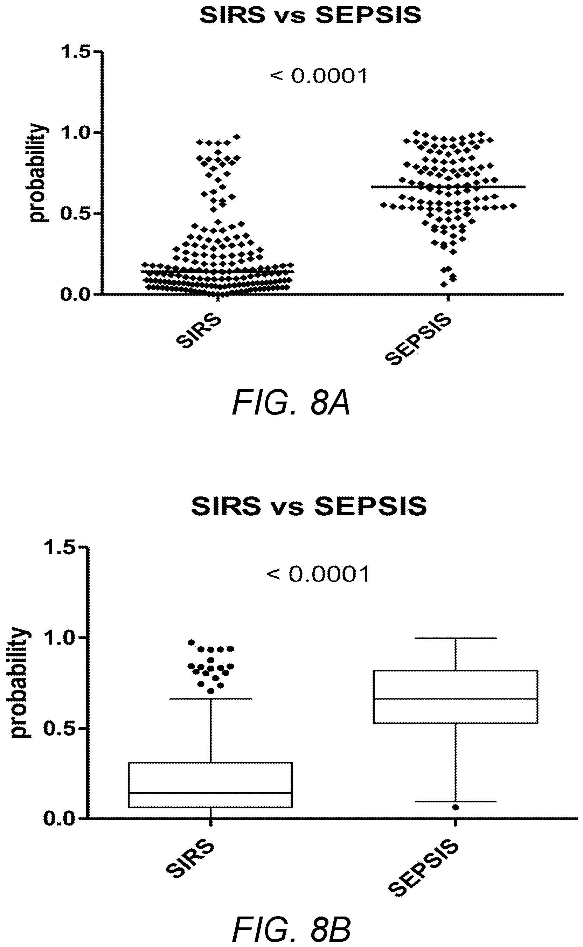

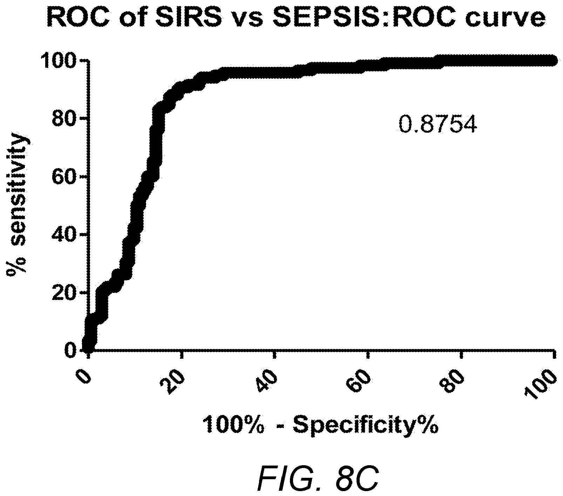

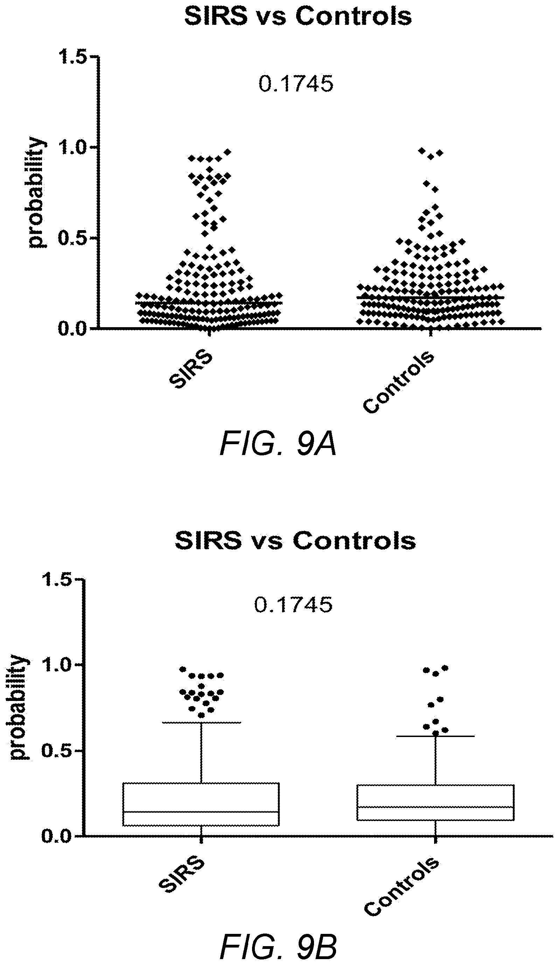

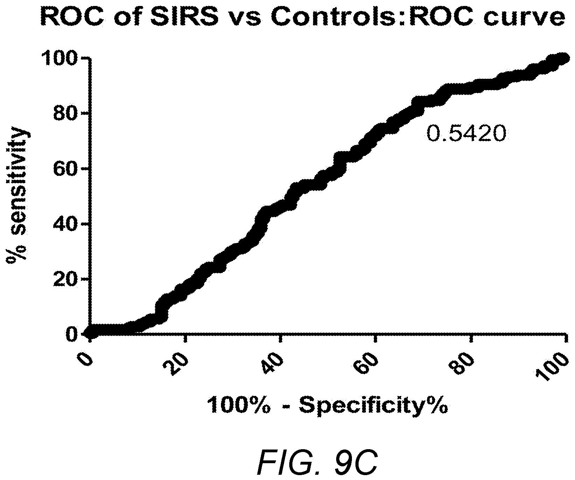

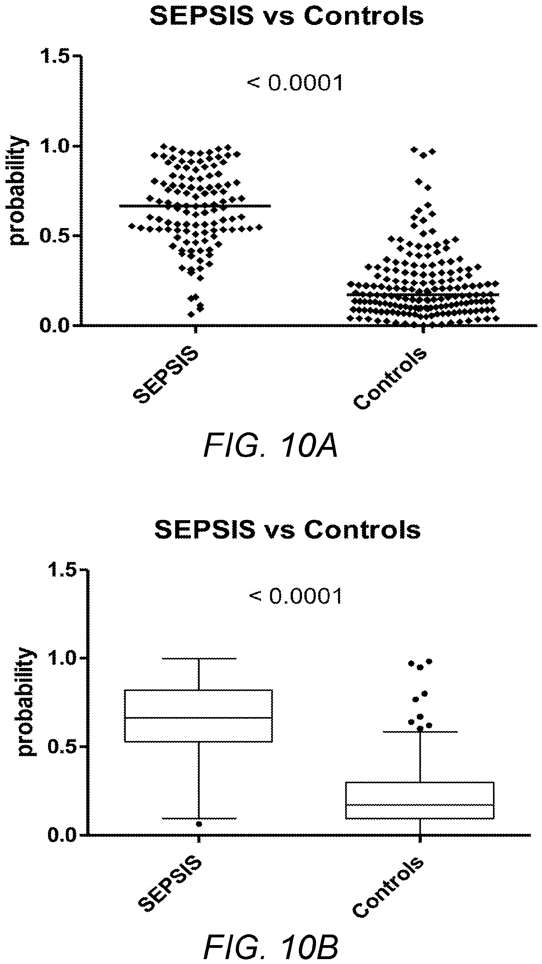

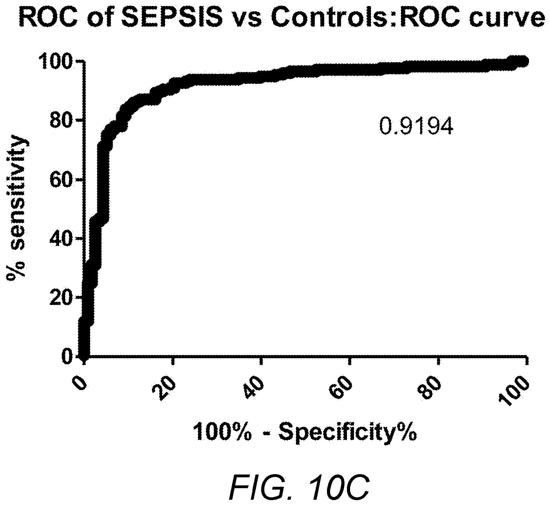

Using the logistic regression model scatter plots and ROC curves were created to show comparisons with all groups. Results for SIRS versus sepsis are shown in Figure 8, for SIRS v controls in Figure 9 and for sepsis versus controls in Figure 10. As can be seen, the model was very effective in distinguishing sepsis from SIRS and also sepsis from controls.

A variety of statistical methods was used to test the significance of any differences between the three groups. The logistic regression derived predictive algorithm developed in this report to distinguish individuals with sepsis compared to SIRS or no SIRS symptoms identified seven biomarkers, these being CRP, A1AT, IL-6, sICAM-1 (ELISA and LF) and TNFα. With these biomarker combinations 85% sensitivity for SIRS and 71.8% for sepsis was achieved with a p value of <0.0001 (Mann-Whitney t test) and an AUC value of 0.8754. Overall, these results provide a very strong basis for the construction of meaningful algorithms and strongly support the feasibility of accurate patient monitoring/prediction of sepsis through the assay of selected biomarkers.

### EXAMPLE 4 - RAT STUDY AND RESULTS

Rats were housed in a metabolic monitoring cage for acclimatisation purposes. They were anaesthetised for 30 minutes for a transthoracic heart scan, and vascular lines were inserted. This was done by making an incision in the centre of the neck and inserting one line into the right jugular vein and one into the left carotid artery. Sepsis animals were given an injection of faecal slurry into the peritoneal cavity, but naive animals were not. Blood was taken from the animals from the inserted lines.

There were eight rats in the study, four Naïve and four "Sepsis" following time-points: 0, 3, 6, 12 and 24 hours.

The following assays were run:

| Marker | Assay Type | Dilution |
|---|---|---|
| IL-6 | Quantikine | 1 in 2 |
| CRP | Duoset | 1 in 40k |
| sICAM | Duoset | 1 in 100, 1in 200 |
| PLA | myBiosource | Neat |
| TIMP1 | Duoset | 1 in 20, 1 in 1000 |
| MMP8 | In-house | Neat |
| alAT | myBiosource | 1 in 50k |

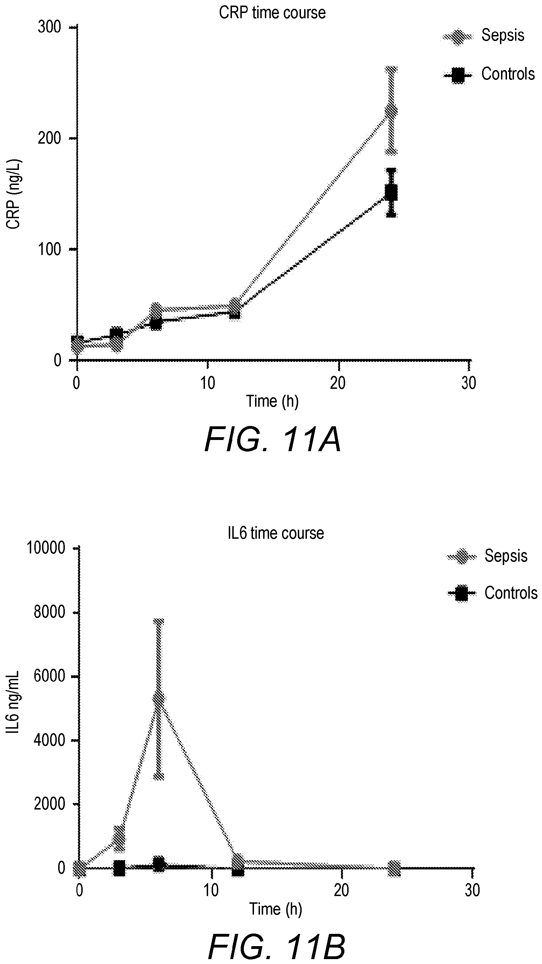

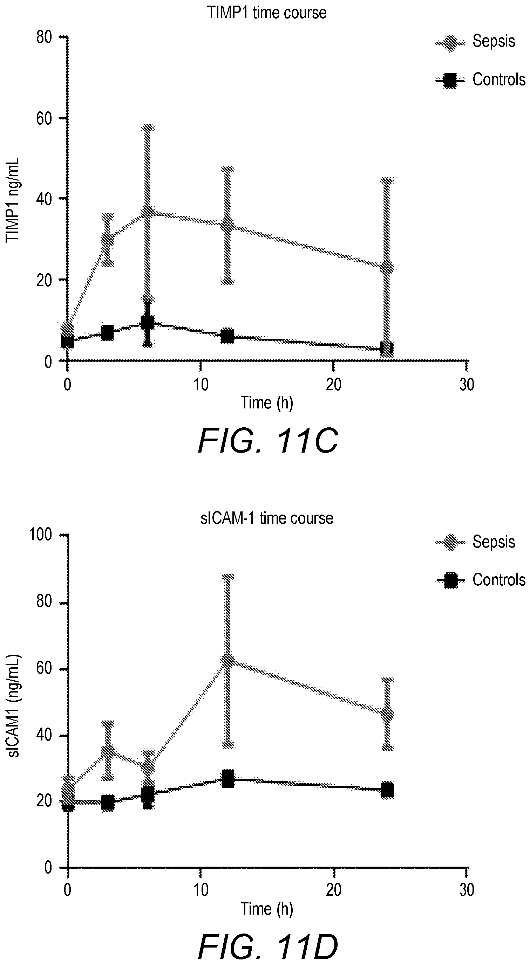

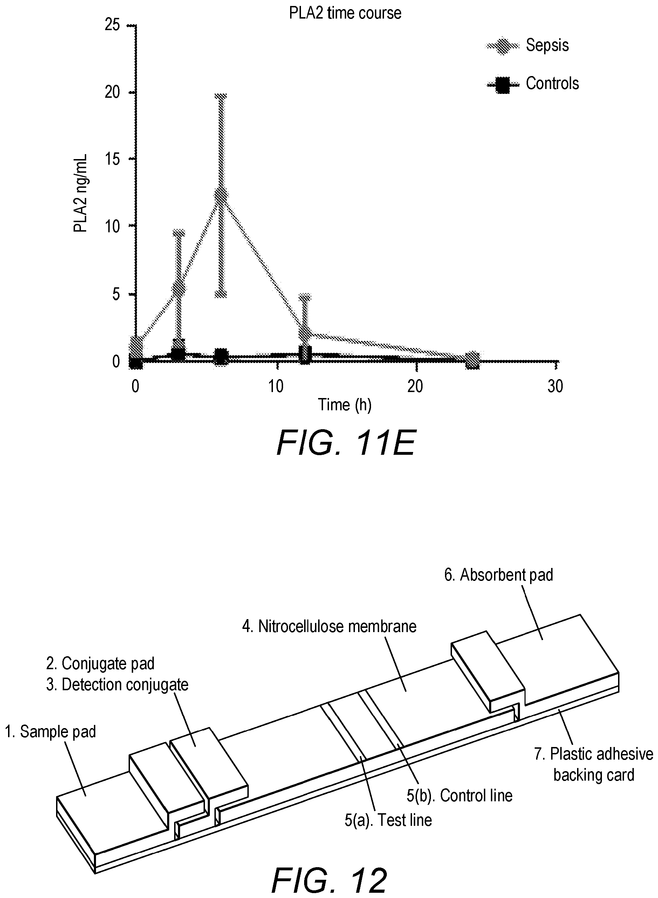

These experiments enabled time courses to be analysed, for example to detect early markers of sepsis which may be of value in predictive methods. Marker time courses are shown for selected markers in Figures 11A-D.

Marker time course features:
- IL6 and PLA2 peak at 6h, rapid decline to 24h
- TIMP1 peaks at 6h, slow decline to 24h
- sICAM-1 peaks 12h, slow decline to 24h
- CRP peaks >24h

### EXAMPLE 5 - SEPSIS PROTOTYPE POINT OF CARE TEST

The prototype device is shown schematically in Figure 12 and consists of a sample pad to which 80µL of a diluted or neat serum sample is added, a conjugate pad containing an optimised mixture of gold colloid-conjugated antibodies for both the test line(s) and control line. The sample containing unknown concentrations of analyte bind to the respective antibodies and travels up the nitrocellulose membrane towards the capture lines. The test line consists of a second antibody to the target analyte and if present in the sample will form a complex resulting in a visible test line. The control line consists of an antigen to the control line antibody conjugated to gold i.e. BSA-biotin to anti-biotin gold, this will tell the user that the test has run successfully. All lines are quantifiable within 10 minutes with a lateral flow device reader such as the Cube (Optricon, Germany).

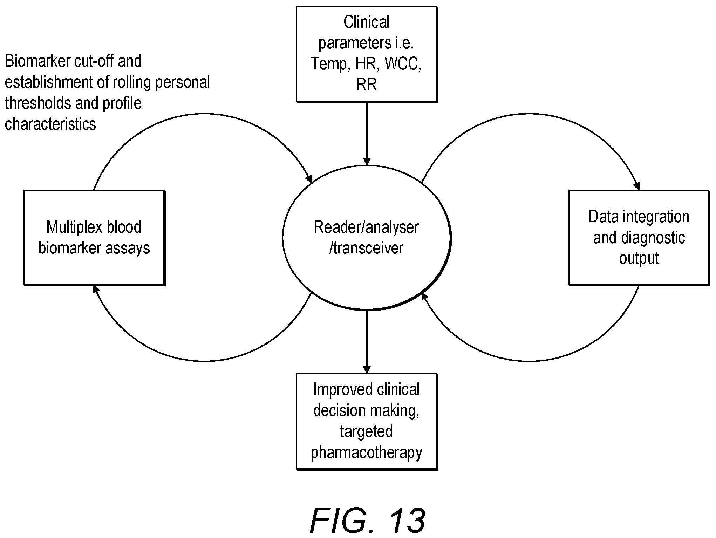

A personalised testing strategy is set out schematically in Figure 13.

### EXAMPLE 6 - sRAGE IN SEPSIS

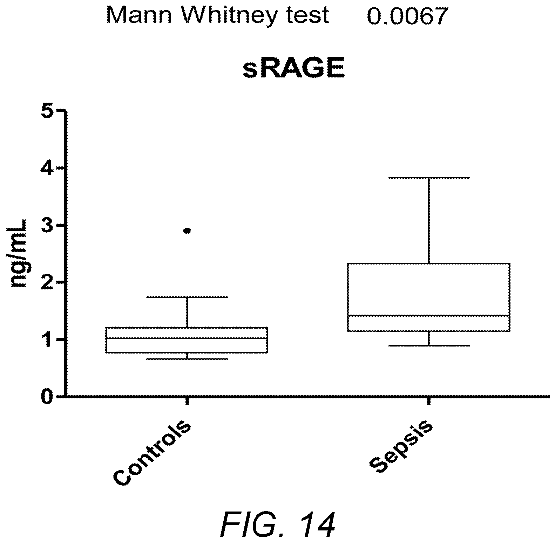

Sixteen samples collected from sepsis patients and 16 samples collected from control subjects were analysed to determine levels of soluble receptor for advanced glycation end products (sRAGE). Higher levels of sRAGE was found in sepsis patients with a median of 1.425 (IQR 1.154-2.337) compared to the control subjects with a median of 1.028 (IQR 0.7678 -1.212). A significant Mann-Whitney test of 0.0062 was obtained where a value <0.5 is deemed significant. Box plot results are shown in Figure 14

ELISA Method:
Reagents: Disposable 96-well polystyrene plates were obtained from Costar (9018 flat bottomed). Human sRAGE was supplied by Novoprotein (Cat No, C423). Capture antibody sheep anti-sRAGE was obtained from Orygen antibodies, (Cat No. SA056). Detection antibody Rabbit anti-sRAGE (Cat No, RA040, Orygen antibodies) conjugated to alkaline phosphatase (Innova bioscience 702-0005). PNPP solution was obtained from Biopanda (Cat No. PNPP-001). Sample diluent prepared at Mologic consisted of PBS, pH6.9, supplemented with 1% (v/v) Tween 20) 1% BSA. Wash buffer prepared at Mologic consisted of 50mM tris buffered saline pH8, supplemented with 0.1% (v/v) Tween 20.
Pilot production process: Microtitre plates were coated overnight with 100µL of sheep anti-sRAGE SA056 (1µg/mL in PBS) per well. Plates were washed 3 times with wash buffer between the blocking step and each of the following incubation steps. Each microtitre well was blocked with 120µl of sample diluent for 1h at room temperature, to minimise nonspecific binding.
Basic assay process: sRAGE was diluted in the sample diluent to give concentrations between 5ng/mL and 0.078ng/mL to generate the dose-response curve. Samples were added neat. 100µL Standards and/or samples were added to microtitre wells and incubated for 1h at room temperature with gentle agitation. Alk-phos conjugated a Rabbit anti-sRAGE RA040 was diluted 1 in 6000 in sample diluent and 100µL subsequently added to microtitre wells and incubated for 1 hours at room temperature with gentle agitation. 100µL of pNPP solution was added and incubated for a further 20 minutes. The absorbance was measured at 405nm using a BMG omega plate reader.

### EXAMPLE 7 - DECISION TREE ANALYSIS OF SEPSIS MARKERS

Sera from patients suffering from post-surgical sepsis (n=211) and post-surgical SIRS (n=288) was analysed by decision tree analysis with a CRT growing method. The combination of markers is CRP, IL-6 and sICAM1.

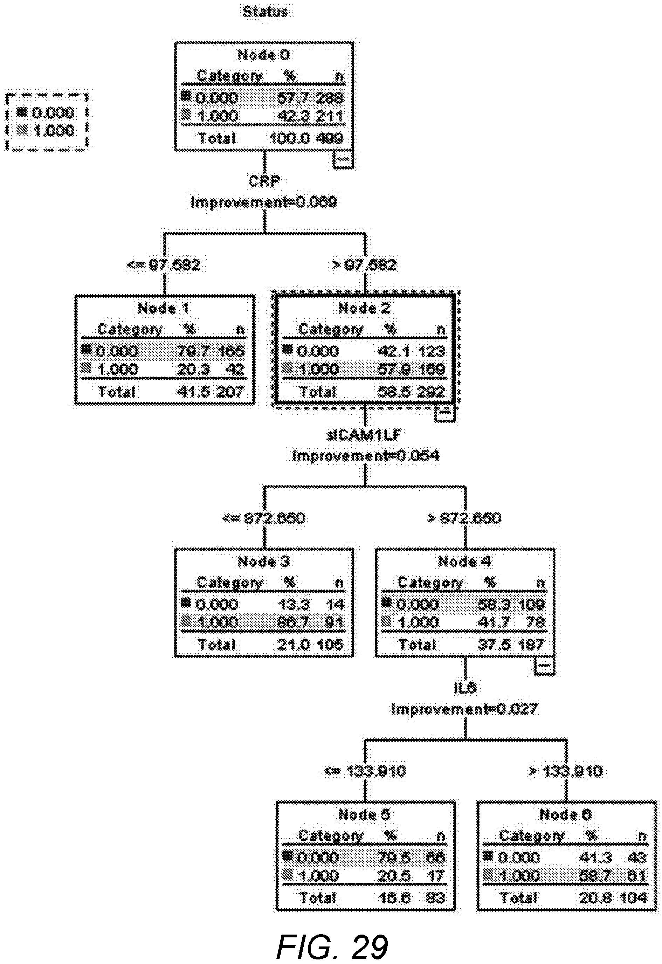

This decision tree, shown in Figure 29 has four terminal nodes with SIRS defined as either CRP<97.582mg/L or as IL-6 <133.910pg/mL when CRP>97.582µg/mL and sICAM1 >872.650ng/mL. Sepsis is defined as sICAM1<872.650ng/mL when CRP is >97.582 or IL-6 > 133.910pg/mL when sICAM1 > 872.650ng/mL and CRP > 97.582µg/mL. This decision tree accurately identifies 80.2% of SIRS patients and 72.0% of Sepsis patients, as shown in the table below:

| | | Predicted | | |
|---|---|---|---|---|
| | | SIRS | Sepsis | % correct |
| observed | SIRS | 231 | 57 | 80.2 |
| | Sepsis | 59 | 152 | 72.0 |

### EXAMPLE 8 - DIAGNOSTIC PERFORMANCE USING SOFA SCORES TO DEFINE SEPSIS

This study was designed to recruit patients who had undergone elective major surgery; these individuals are at risk of developing post-surgical infections and sepsis.

One serum sample was taken pre-surgery as well as 7 days following surgery. The day of onset of SIRS symptoms is designated day 0, preceding days termed day -1, day -2 etc. and days post symptoms of SIRS termed day 1, day 2 etc.

3 cohorts were recruited to the study:
i) Individuals that developed 2 or more SIRS symptoms + confirmed or suspected infection
ii) Individuals that developed 2 or more SIRS symptoms with no evidence of infection
iii) Individuals that did not develop any SIRS symptoms

Our analysis here is limited to only include groups (i) an (ii) and limited to the first day of SIRS symptoms.

The markers panel that were tested on these samples:
CRP, acute phase response
IL6 and TNFα, inflammatory cytokines
CCL23 (MIP3), a chemokine
sICAM-1, vascular endothelial activation*
secreted type IIA phospholipase A2 (sPLA2g2a), pro-inflammatory enzyme
acetylated-PGP and desmosine, products of collagen and elastase respectively
α1-antitrypsin (A1AT), protease inhibitor

*sICAM-1 was measured by ELISA and LF. Both these methods of analysis do not correlate to a high degree, suggesting that they are recognising different (albeit related) targets. These immunoassays contain different antibody pairs and it is believed that the LF assay is detecting different forms of ICAM including ICAM-1 and VCAM-1.

The patients were differentiated into 2 groups based on having sepsis or not having sepsis (day one presentation of symptoms):
1. Infection + SIRS (Infection + 2 SIRS criteria (RR, WBC, HR, Temp))
2. SIRS: (2+ SIRS criteria (RR, WBC, HR, Temp))

The groups were further defined according to SOFA scores: those based on having SOFA 2-6 were grouped:
1. Infection + SIRS group: Number of patients n = 23
2. SIRS group: Number of patients n= 25

The median SOFA score for both groups was 4.

### SOFA scores for all patients:

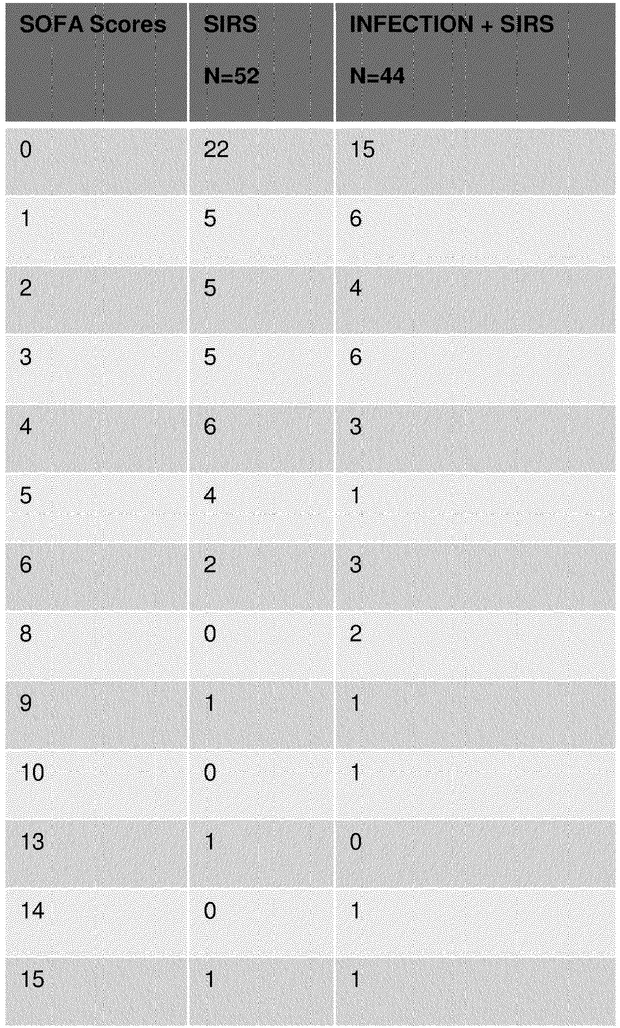

### Diagnostic performance with logistic regression (LR) models

Diagnostic performance was initially measured in relation to distinguish the infection +SIRS group from the SIRS only group.

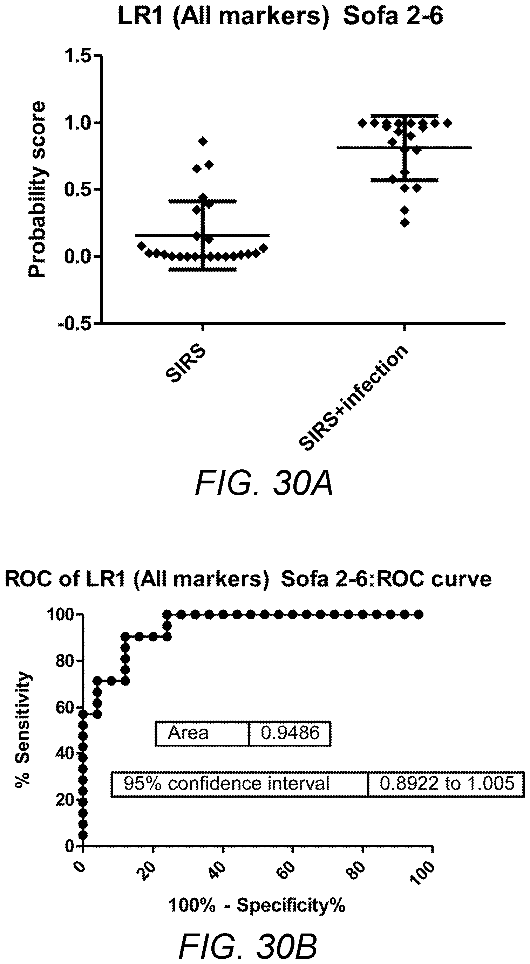

A first LR model, LR1, was generated using 10 markers: Desmosine, TNF, IL-6, AcPGP, PLA2g2A, CCL23, A1AT, sICAM1 (ELISA), sICAM1 (LF), CRP. LR1 gave a Sensitivity of 90.5% and specificity of 88.0% as shown in the table below and in Figures 30A and 30B.

**Classification Table - LR1**

| Observed | Predicted | | |
|---|---|---|---|
| | VAR00001 | | Percentage Correct |
| | SIRS | Infection + SIRS | |
| Step 1 VAR00001 SIRS | 22 | 3 | 88.0 |
| Infection + SIRS | 2 | 19 | 90.5 |
| Overall Percentage | | | 89.1 |

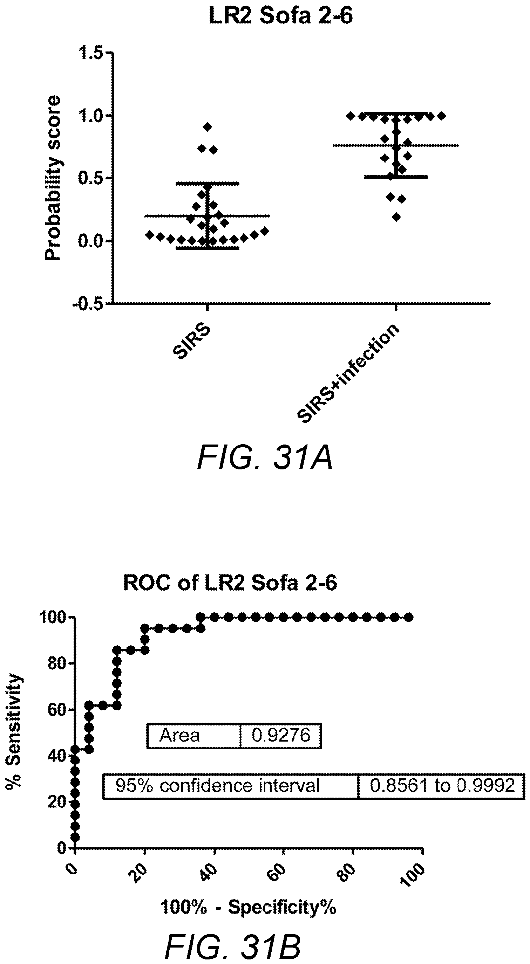

A second model, LR2, was generated using a backwards conditional Logistic regression function. Five markers were selected: CCL23, A1AT, CRP, sICAMLF and sICAM ELISA. LR2 gave a sensitivity of 85.7% and specificity of 88.0% as shown in the table below and in Figures 31A and 31B.

**Classification Table - LR2**

| Observed | Predicted | | |
|---|---|---|---|
| | VAR00001 | | Percentage Correct |
| | SIRS | Infection + SIRS | |
| Step 5 VAR00001 SIRS | 22 | 3 | 88.0 |
| Infection + SIRS | 3 | 18 | 85.7 |
| Overall Percentage | | | 87.0 |

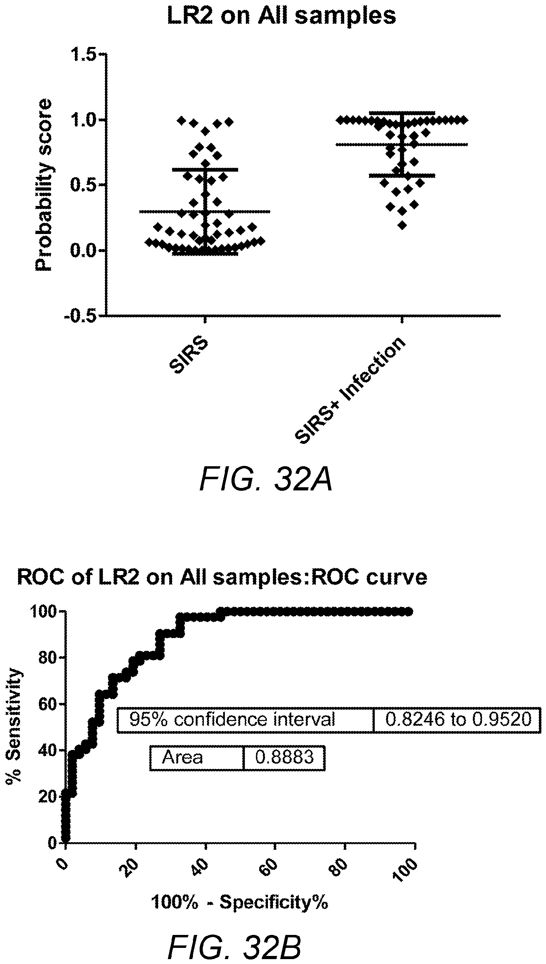

LR2 was also shown to reliably detect SIRS + infection across all patients, irrespective of SOFA score in sensitive and specific fashion. Results are presented in Figures 32A and 32B.

### Diagnosing severity

Diagnostic performance was further measured in relation to distinguishing the infection group with SOFA scores of at least 2 from the infection group with SOFA score less than 2.

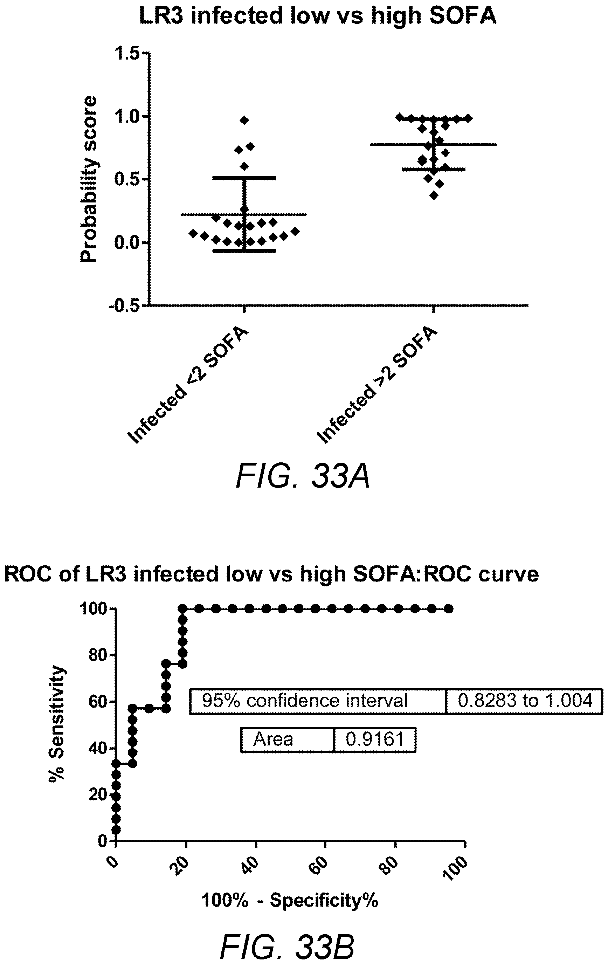

When tested, LR2 was not able to differentiate SOFA scores of at least 2 from SOFA scores less than 2 in either group (data not shown). Accordingly, a third model, LR3, was generated. Seven markers were selected: CCL23, A1AT, sICAM, desmosine, TNF alpha, IL-6 and PLA2g2A. LR3 was shown to reliably distinguish high SOFA score from low SOFA score in the infected group to indicate severity (sepsis). Results are presented in Figures 33A and 33B.

### EXAMPLE 9 - VALIDATION STUDY

The validation study was a prospective, observational cohort study of critically ill adult patients admitted to the ICU. Following study approval from an ethics committee, all patients were screened on a daily basis to assess those meeting the following inclusion and exclusion criteria:

### Inclusion criteria

∘ Multi-organ failure (at least 2 organ systems involved)
∘ Initial SOFA score >3
∘ Predicted length of ICU stay >3 days

Following consent, patients were enrolled into the study and data were recorded on a daily basis for the first week and weekly thereafter. Blood sampling was undertaken on the day of admission (Day 0) and, subsequently, on Days 1, 2, 3, 5, 7 and weekly thereafter.

### Patient recruitment

A total of 556 patients were screened for enrolment. Of 177 eligible for inclusion, 51 consented to taking part in the study. Of these, 24 patients with either faecal peritonitis or community acquired pneumonia had samples analysed by Mologic.

### Results

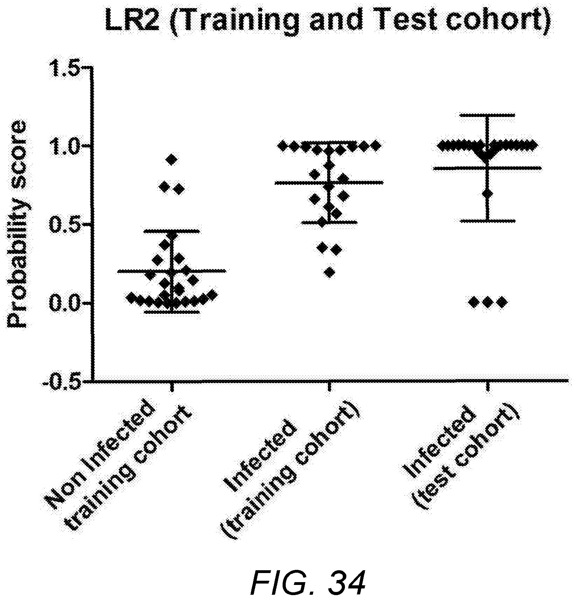

As shown in Figure 34, the LR2 algorithm was able to positively identify the infected patients in the validation set (based on day 0 samples).

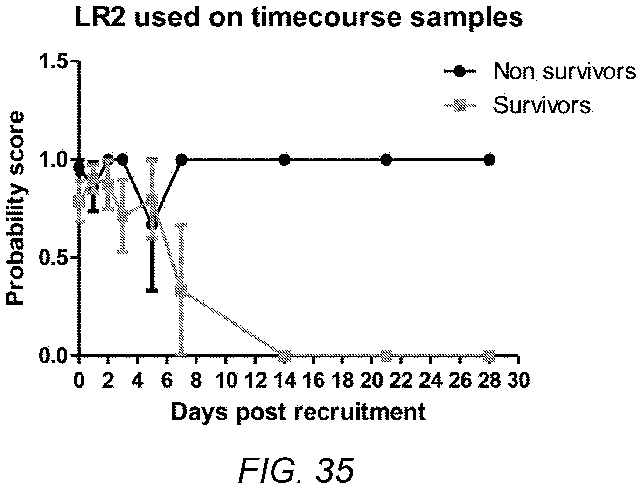

It was also shown that the LR2 algorithm could also be used to discriminate survivors from non-survivors, see Figure 35.

## Claims

1. A method for predicting sepsis or diagnosing systemic inflammatory response syndrome (SIRS) and/or sepsis in a subject, the method comprising determining levels of at least CCL23, A1AT and CRP, optionally together with at least one or more of sICAM, PLA2, IL-6, procalcitonin, MMP8, TNFalpha, AcPGP, enzymatic MMP activity, TIMP1, sRAGE and desmosine in a sample taken from the subject, wherein the combined levels of the at least three markers are used to predict or diagnose SIRS and/or sepsis.

2. The method of claim 1 performed on a subject with SIRS and which is used to identify an infection in the subject.

3. The method of claim 1 or 2 wherein:
a) the markers are all of desmosine, TNF, IL-6, AcPGP, PLA2g2A, CCL23, A1AT, sICAM (optionally measured by ELISA), sICAM/VCAM-1 (optionally measured by lateral flow) and CRP;
b) the markers are all of CCL23, A1AT, sICAM (optionally measured by ELISA), sICAM/VCAM-1 (optionally measured by lateral flow) and CRP;
c) the markers include CRP, PLA2 and sICAM;
d) the markers comprise CRP, sICAM and TNFalpha or CRP, sICAM and IL-6; or
e) the method comprises determining levels of at least four markers, optionally wherein the markers comprise PLA2, IL-6, CRP and TNFalpha.

4. A method for monitoring a subject at risk of developing sepsis, the method comprising determining levels of at least CCL23, A1AT and CRP in samples taken from the subject at multiple time points, wherein the monitored levels of CCL23, A1AT and CRP are used to predict or diagnose SIRS and/or sepsis.

5. The method of claim 4 wherein the subject is a hospitalised patient and/or an immunocompromised patient.

6. The method of claim 4 wherein the subject has been subjected to a surgical procedure and the multiple time points include a first sample taken from the subject prior to the surgery to provide baseline levels of the markers and at least one further sample taken from the subject following surgery, wherein the monitored levels of CCL23, A1AT and CRP are used to predict or diagnose SIRS and/or sepsis following surgery.

7. The method of any one of claims 1 to 6 wherein:
a) the method discriminates SIRS from sepsis;
b) the method provides a prediction of impending sepsis;
c) the markers further comprise one or more, up to all, of IL-6, TIMP1, sICAM-1 and PLA2;
d) a prediction of impending sepsis results in treatment of the infection; or
e) the subject is not treated (e.g. with an antibiotic) unless and until impending sepsis is predicted or sepsis is diagnosed.

8. The method of any one of claims 4 to 7 wherein:
a) the method further comprises determining the levels of at least one further marker selected from sICAM, PLA2, IL-6, procalcitonin, MMP8, TNFalpha, AcPGP, enzymatic MMP activity, TIMP1, sRAGE and desmosine; or
b) the method is performed on a subject with SIRS and which is used to identify an infection in the subject.

9. The method of claim 8 wherein:
a) the markers are all of desmosine, TNF, IL-6, AcPGP, PLA2g2A, CCL23, A1AT, sICAM (optionally measured by ELISA), sICAM/VCAM-1 (optionally measured by lateral flow) and CRP;
b) the markers are all of CCL23, A1AT, sICAM (optionally measured by ELISA), sICAM/VCAM-1 (optionally measured by lateral flow) and CRP; or
c) the markers include CRP, PLA2 and sICAM.

10. The method of any one of claims 4 to 9 wherein:
a) at least 2, 3, 4, 5, 6, 7, 8, 9, 10, 11, 12, 13 or 14 or more samples are taken from the subject at different times and the levels of the at least three markers is determined;
b) the samples are taken every 6 to 24 hours, such as daily, or every 3, 4, 5, 6, 7 or 14 days; or
c) the method comprises determining levels of at least four markers.

11. The method of any one of claims 1 to 10 wherein:
a) the method comprises determining levels of at least five or six markers, optionally wherein the markers comprise sICAM, CCL23, A1AT, CRP, IL-6 and TNFalpha; or
b) the markers comprise sICAM alone (optionally when measured by an ELISA) and sICAM/VCAM-1 (optionally sICAM when measured by a lateral flow assay).

12. A method of selecting a subject for treatment with an antibiotic comprising performing the method of any one of claims 1 to 11 and selecting the subject for treatment where sepsis is predicted or diagnosed.

13. An antibiotic for use in a method of treating sepsis, wherein the subject has been selected for treatment by performing the method of any one of claims 1 to 12.

14. An antibiotic for use in a method of treating sepsis, wherein the subject displays, in a sample, an altered level of at least CCL23, A1AT and CRP optionally together with an altered level of at least one of sICAM, PLA2, IL-6, procalcitonin, MMP8, TNFalpha, AcPGP, enzymatic MMP activity, TIMP1, sRAGE and desmosine.

15. The antibiotic for use of claims 13 to 14 wherein the sample is a whole blood, plasma or serum sample.

16. A system or test kit for predicting or diagnosing systemic inflammatory response syndrome (SIRS) and/or sepsis in a subject, comprising:
a. One or more testing devices for determining levels of at least CCL23, A1AT and CRP optionally together with at least one of sICAM, PLA2, IL-6, procalcitonin, MMP8, TNFalpha, AcPGP, enzymatic MMP activity, TIMP1, sRAGE and desmosine in a sample
b. A processor; and
c. A storage medium comprising a computer application that, when executed by the processor, is configured to:
i. Access and/or calculate the determined levels of each marker in the sample on the one or more testing devices
ii. Calculate a test score from the levels of the markers in the sample that predicts or diagnoses SIRS and/or sepsis; and
iii. Output from the processor the predicted or diagnostic result for the subject.

17. A system or test kit for monitoring a subject, comprising:
a. One or more testing devices for determining levels of at least CCL23, A1AT and CRP optionally together with at least one of sICAM, PLA2, IL-6, procalcitonin, MMP8, TNFalpha, AcPGP, enzymatic MMP activity, TIMP1, sRAGE and desmosine in a sample at multiple time points
b. A processor; and
c. A storage medium comprising a computer application that, when executed by the processor, is configured to:
i. Access and/or calculate the determined levels of each marker in the sample on the one or more testing devices
ii. Calculate a test score from the levels of the markers in the sample, optionally including a comparison of the levels with those taken at one or more earlier time points, to thereby predict or diagnose SIRS and/or sepsis; and
iii. Output from the processor the predicted or diagnostic result for the subject;
optionally wherein the subject has been subjected to a surgical procedure and the multiple time points include a first sample taken from the subject prior to the surgery to provide baseline levels of the markers and at least one further sample taken from the subject following surgery.

18. A testing device, testing kit or testing composition of matter comprising:
a. A sample receiving zone to which a sample from a subject is added
b. A conjugate zone comprising at least three labelled binding reagents, each of which specifically binds to one of CCL23, A1AT and CRP, optionally together with at least one further labelled binding reagent which specifically binds to sICAM, PLA2, IL-6, procalcitonin, MMP8, TNFalpha, AcPGP, enzymatic MMP activity, TIMP1, sRAGE or desmosine
c. A solid support defining a liquid flow path for the sample and comprising corresponding test lines for each of the at least three markers, each test line comprising an immobilised further binding reagent that also specifically binds to one of CCL23, A1AT and CRP thereby immobilising the marker at the test line to produce a signal via the labelled binding reagent also specifically bound to the marker.

19. The testing device, testing kit or testing composition of matter of claim 18 wherein:
a) the sample receiving zone is proportioned to receive between 10 and 100 µl of serum, such as around 80 µl of serum;
b) the solid support comprises a chromatographic medium;
c) the solid support comprises a capillary flow device;
d) the testing device, testing kit or testing composition is a test strip.

20. The testing device, testing kit or testing composition of matter of claim 18 or 19 further comprising a reader to quantify levels of the markers at the respective test lines, optionally wherein the reader further comprises:
A)
a. A processor; and
b. A storage medium comprising a computer application that, when executed by the processor, is configured to:
i. Access and/or calculate the determined levels of each marker in the sample on the one or more testing devices
ii. Calculate a test score from the levels of the markers in the sample that predicts or diagnoses SIRS and/or sepsis; and
iii. Output from the processor the predicted or diagnostic result for the subject; or
B)
a. A processor; and
b. A storage medium comprising a computer application that, when executed by the processor, is configured to:
i. Access and/or calculate the determined levels of each marker in the sample on the one or more testing devices
ii. Calculate a test score from the levels of the markers in the sample by comparing the levels with those taken at one or more earlier time points to thereby predict or diagnose SIRS and/or sepsis; and
iii. Output from the processor the predicted or diagnostic result for the subject.

## Patentansprüche

1. Verfahren zur Vorhersage von Sepsis oder zur Diagnose von systemischem Entzündungsreaktionssyndrom (SIRS) und/oder Sepsis bei einem Individuum, wobei das Verfahren die Ermittlung der Spiegel von mindestens CCL23, A1AT und CRP, optional zusammen mit mindestens einem oder mehreren von sICAM, PLA2, IL-6, Procalcitonin, MMP8, TNFalpha, AcPGP, enzymatischer MMP-Aktivität, TIMP1, sRAGE und Desmosin in einer dem Individuum entnommenen Probe umfasst, wobei die kombinierten Werte der mindestens drei Marker zur Vorhersage oder Diagnose von SIRS und/oder Sepsis verwendet werden.

2. Verfahren nach Anspruch 1, das bei einem Patienten mit SIRS durchgeführt wird und das zur Identifizierung einer Infektion bei dem Patienten verwendet wird.

3. Verfahren nach Anspruch 1 oder 2, wobei:
a) die Marker alle von Desmosin, TNF, IL-6, AcPGP, PLA2g2A, CCL23, A1AT, sICAM (optional durch ELISA gemessen), sICAM/VCAM-1 (optional durch Lateral Flow gemessen) und CRP sind;
b) die Marker alle von CCL23, A1AT, sICAM (optional durch ELISA gemessen), sICAM/VCAM-1 (optional durch Lateral Flow gemessen) und CRP sind;
c) die Marker CRP, PLA2 und sICAM enthalten;
d) die Marker CRP, sICAM und TNFalpha oder CRP, sICAM und IL-6 umfassen; oder
e) das Verfahren die Ermittlung von Spiegeln von mindestens vier Markern umfasst, wobei die Marker optional PLA2, IL-6, CRP und TNFalpha umfassen.

4. Verfahren zum Überwachen eines Individuums, das gefährdet ist, eine Sepsis zu entwickeln, wobei das Verfahren die Ermittlung von Spiegeln von mindestens CCL23, A1AT und CRP in Proben umfasst, die von dem Individuum zu mehreren Zeitpunkten genommen wurden, wobei die überwachten Spiegel von CCL23, A1AT und CRP verwendet werden, um SIRS und/oder Sepsis vorherzusagen oder zu diagnostizieren.

5. Verfahren nach Anspruch 4, bei dem das Individuum ein hospitalisierter Patient und/oder ein immungeschwächter Patient ist.

6. Verfahren nach Anspruch 4, bei dem das Individuum einer chirurgischen Operation unterzogen wurde und die mehreren Zeitpunkte eine erste Probe, die dem Individuum vor der Operation entnommen wurde, um Grundlinienspiegel der Marker bereitzustellen, und mindestens eine weitere Probe, die dem Individuum nach der Operation entnommen wurde, umfassen, wobei die überwachten Werte von CCL23, A1AT und CRP zur Vorhersage oder Diagnose von SIRS und/oder Sepsis nach der Operation verwendet werden.

7. Verfahren nach einem der Ansprüche 1 bis 6, bei dem:
a) das Verfahren SIRS von Sepsis unterscheidet;
b) das Verfahren eine Vorhersage einer bevorstehenden Sepsis liefert;
c) die Marker ferner einen oder mehrere, bis zu allen, von 1L-6, TIMP1, sICAM-1 und PLA2 umfassen;
d) eine Vorhersage einer bevorstehenden Sepsis zur Behandlung der Infektion führt; oder
e) das Individuum nicht behandelt wird (z.B. mit einem Antibiotikum), es sei denn dass, und bis, eine drohende Sepsis vorhergesagt oder eine Sepsis diagnostiziert wird.

8. Verfahren nach einem der Ansprüche 4 bis 7, bei dem:
a) das Verfahren ferner die Ermittlung der Spiegel mindestens eines weiteren Markers umfasst, der aus sICAM, PLA2, IL-6, Procalcitonin, MMP8, TNFalpha, AcPGP, enzymatischer MMP-Aktivität, TIMP1, sRAGE und Desmosin ausgewählt ist; oder
b) das Verfahren an einem Individuum mit SIRS durchgeführt wird und zur Identifizierung einer Infektion des Individuums verwendet wird.

9. Verfahren nach Anspruch 8, bei dem:
a) die Marker alle von Desmosin, TNF, IL-6, AcPGP, PLA2g2A, CCL23, A1AT, sICAM (optional durch ELISA gemessen), sICAM/VCAM-1 (optional durch Lateral Flow gemessen) und CRP sind;
b) die Marker alle von CCL23, A1AT, sICAM (optional durch ELISA gemessen), sICAM/VCAM-1 (optional durch Lateral Flow gemessen) und CRP sind; oder
c) die Marker CRP, PLA2 und sICAM beinhalten.

10. Verfahren nach einem der Ansprüche 4 bis 9, bei dem:
a) dem Individuum zu verschiedenen Zeiten mindestens 2, 3, 4, 5, 6, 7, 8, 9, 10, 11, 12, 13 oder 14 oder mehr Proben entnommen werden und die Spiegel der mindestens drei Marker ermittelt werden;
b) die Proben alle 6 bis 24 Stunden, z.B. täglich, oder alle 3, 4, 5, 6, 7 oder 14 Tage entnommen werden; oder
c) das Verfahren die Ermittlung von Spiegeln von mindestens vier Markern umfasst.

11. Verfahren nach einem der Ansprüche 1 bis 10, bei dem:
a) das Verfahren die Ermittlung von Spiegeln von mindestens fünf oder sechs Markern umfasst, wobei die Marker optional sICAM, CCL23, A1AT, CRP, IL-6 und TNFalpha umfassen; oder
b) die Marker sICAM allein (optional gemessen mit einem ELISA) und sICAM/VCAM-1 (optional sICAM bei Messung mit einem Lateral Flow Assay) umfassen.

12. Verfahren zum Auswählen eines Individuums zur Behandlung mit einem Antibiotikum, umfassend die Durchführung des Verfahrens nach einem der Ansprüche 1 bis 11 und die Auswahl des Individuums zur Behandlung, bei dem eine Sepsis vorhergesagt oder diagnostiziert wird.

13. Antibiotikum zur Verwendung in einem Verfahren zur Behandlung von Sepsis, bei dem das Individuum zur Behandlung durch Durchführung des Verfahrens nach einem der Ansprüche 1 bis 12 ausgewählt worden ist.

14. Antibiotikum zur Verwendung in einem Verfahren zur Behandlung von Sepsis, wobei das Individuum in einer Probe einen veränderten Spiegel von mindestens CCL23, A1AT und CRP zeigt, optional zusammen mit einem veränderten Spiegel von mindestens einem von sICAM, PLA2, 1L-6, Procalcitonin, MMP8,
TNFalpha, AcPGP, enzymatischer MMP-Aktivität, TIMP1, sRAGE und Desmosin.

15. Antibiotikum zur Verwendung nach den Ansprüchen 13 bis 14, wobei die Probe eine Vollblut-, Plasma- oder Serumprobe ist.

16. System oder Testkit zur Vorhersage oder Diagnose von systemischem Entzündungsreaktionssyndrom (SIRS) und/oder Sepsis bei einem Individuum, umfassend:
a. eine oder mehrere Testvorrichtungen zur Ermittlung von Spiegeln von mindestens CCL23, A1AT und CRP, optional zusammen mit mindestens einem von sICAM, PLA2, IL-6, Procalcitonin, MMP8, TNFalpha, AcPGP, enzymatischer MMP-Aktivität, TIMP1, sRAGE und Desmosin in einer Probe,
b. einen Prozessor; und
c. ein Speichermedium, das eine Computeranwendung umfasst, die, wenn sie vom Prozessor ausgeführt wird, konfiguriert ist zum
i. Zugreifen auf die und/oder Berechnen der ermittelten Spiegel jedes Markers in der Probe auf einer oder mehreren Testvorrichtungen;
ii. Berechnen eines Testergebnisses aus den Spiegeln der Marker in der Probe, das SIRS und/oder Sepsis vorhersagt oder diagnostiziert; und
iii. Ausgeben des vorhergesagten oder diagnostizierten Ergebnisses für das Individuum durch den Prozessor.

17. System oder Testkit zur Überwachung eines Individuums, umfassend
a. eine oder mehrere Testvorrichtungen zur Ermittlung von Spiegeln von mindestens CCL23, A1AT und CRP, optional zusammen mit mindestens einem von sICAM, PLA2, IL-6, Procalcitonin, MMP8, TNFalpha, AcPGP, enzymatischer MMP-Aktivität, TIMP1, sRAGE und Desmosin in einer Probe zu mehreren Zeitpunkten;
b. einen Prozessor; und
c. ein Speichermedium, das eine Computeranwendung umfasst, die, wenn sie vom Prozessor ausgeführt wird, konfiguriert ist zum
i. Zugreifen auf die und/oder Berechnen der ermittelten Spiegel jedes Markers in der Probe auf der einen oder den mehreren Testvorrichtungen,
ii. Berechnen eines Testergebnisses aus den Spiegeln der Marker in der Probe, optional einschließlich eines Vergleichs der Spiegel mit solchen, die zu einem oder mehreren früheren Zeitpunkten genommen wurden, um dadurch SIRS und/oder Sepsis vorherzusagen oder zu diagnostizieren; und
iii. Ausgeben des vorhergesagten oder diagnostizierten Ergebnisses für das Individuum durch den Prozessor; optional, wobei das Individuum einer chirurgischen Operation unterzogen wurde und die mehreren Zeitpunkte eine erste Probe, die dem Individuum vor der Operation entnommen wurde, um Grundlinienspiegel der Marker bereitzustellen, und mindestens eine weitere Probe, die dem Individuum nach der Operation entnommen wurde, umfassen.

18. Testvorrichtung, Testkit oder Testzusammensetzung, die umfasst:
a. eine Probenaufnahmezone, zu der eine Probe von einem Individuum hinzugefügt wird;
b. eine Konjugatzone, umfassend mindestens drei markierte Bindungsreagenzien, von denen jedes spezifisch an eines von CCL23, A1AT und CRP bindet, optional zusammen mit mindestens einem weiteren markierten Bindungsreagenz, das spezifisch an sICAM, PLA2, 1L-6, Procalcitonin, MMP8, TNFalpha, AcPGP, enzymatische MMP-Aktivität, TIMP1, sRAGE oder Desmosin bindet;
c. einen festen Träger, der einen Flüssigkeitsströmungsweg für die Probe definiert und entsprechende Testlinien für jeden der mindestens drei Marker umfasst, wobei jede Testlinie ein immobilisiertes weiteres Bindungsreagenz umfasst, das auch spezifisch an eines von CCL23, A1AT und CRP bindet, wodurch der Marker an der Testlinie immobilisiert wird, um ein Signal über das ebenfalls spezifisch an den Marker gebundene markierte Bindungsreagenz zu erzeugen.

19. Testvorrichtung, Testkit oder Testzusammensetzung nach Anspruch 18, wobei:
a) die Probenaufnahmezone so proportioniert ist, dass sie zwischen 10 und 100 µl Serum, wie etwa 80 µl Serum, aufnimmt;
b) der feste Träger ein chromatographisches Medium umfasst;
c) der feste Träger eine Kapillarflussvorrichtung umfasst;
d) die Testvorrichtung, der Testkit oder die Testzusammensetzung ein Teststreifen ist.

20. Testvorrichtung, Testkit oder Testzusammensetzung nach Anspruch 18 oder 19, die ferner ein Lesegerät zur Quantifizierung der Markermengen an den jeweiligen Testlinien umfasst, wobei das Lesegerät optional ferner umfasst:
A)
a. einen Prozessor; und
b. ein Speichermedium, das eine Computeranwendung umfasst, die, wenn sie vom Prozessor ausgeführt wird, konfiguriert ist zum
i. Zugreifen auf die und/oder Berechnen der ermittelten Spiegel jedes Markers in der Probe auf der einen oder den mehreren Testvorrichtungen;
ii. Berechnen eines Testergebnisses aus den Spiegeln der Marker in der Probe, das SIRS und/oder Sepsis vorhersagt oder diagnostiziert; und
iii. Ausgeben des vorhergesagten oder diagnostizierten Ergebnisses für das Individuum durch den Prozessor; oder
B)
a. einen Prozessor; und
b. ein Speichermedium, das eine Computeranwendung umfasst, die, wenn sie vom Prozessor ausgeführt wird, konfiguriert ist zum
i. Zugreifen auf die und/oder Berechnen der ermittelten Spiegel jedes Markers in der Probe auf der einen oder den mehreren Testvorrichtungen,
ii. Berechnen eines Testergebnisses aus den Spiegeln der Marker in der Probe durch Vergleichen der Werte mit denen zu einem oder mehreren früheren Zeitpunkten, um dadurch SIRS und/oder Sepsis vorherzusagen oder zu diagnostizieren; und
iii. Ausgeben des vorhergesagten oder diagnostizierten Ergebnisses für das Individuum durch den Prozessor.

## Revendications

1. Procédé de prédiction de la sepsie ou de diagnostic du syndrome de réponse inflammatoire systémique (SRIS) et/ou de la sepsie chez un sujet, le procédé comprenant l'étape consistant à déterminer des niveaux d'au moins CCL23, A1AT et CRP, éventuellement conjointement avec au moins un ou plusieurs parmi sICAM, PLA2, IL-6, la procalcitonine, MMP8, TNFalpha, AcPGP, l'activité MMP enzymatique, TIMP1, sRAGE et la desmosine dans un échantillon prélevé sur le sujet, où les niveaux combinés des au moins trois marqueurs sont utilisés pour prédire ou diagnostiquer le SRIS et/ou la sepsie.

2. Procédé de la revendication 1 réalisé sur un sujet atteint de SRIS et qui est utilisé pour identifier une infection chez le sujet.

3. Procédé de la revendication 1 ou 2, dans lequel :
a) les marqueurs sont l'ensemble de la desmosine, TNF, IL-6, AcPGP, PLA2g2A, CCL23, A1AT, sICAM (éventuellement mesurée par ELISA), sICAM/VCAM-1 (éventuellement mesurées par écoulement latéral) et CRP ;
b) les marqueurs sont l'ensemble de CCL23, A1AT, sICAM (éventuellement mesurée par ELISA), sICAM/VCAM-1 (éventuellement mesurées par écoulement latéral) et CRP ;
c) les marqueurs comportent CRP, PLA2 et sICAM ;
d) les marqueurs comprennent CRP, sICAM et TNFalpha ou CRP, sICAM et IL-6 ; ou
e) le procédé comprenant l'étape consistant à déterminer des niveaux d'au moins quatre marqueurs, éventuellement où les marqueurs comprennent PLA2, IL-6, CRP et TNFalpha.

4. Procédé de surveillance d'un sujet présentant un risque de développer une sepsie, le procédé comprenant l'étape consistant à déterminer des niveaux d'au moins CCL23, A1AT et CRP dans des échantillons prélevés sur le sujet à plusieurs instants, où les niveaux surveillés de CCL23, A1AT et CRP sont utilisés pour prédire ou diagnostiquer le SRIS et/ou la sepsie.

5. Procédé de la revendication 4, dans lequel le sujet est un patient hospitalisé et/ou un patient immunodéprimé.

6. Procédé de la revendication 4, dans lequel le sujet a été soumis à une intervention chirurgicale et les plusieurs instants comportent le prélèvement d'un premier échantillon sur le sujet avant la chirurgie pour fournir des niveaux de base des marqueurs et le prélèvement d'au moins un échantillon supplémentaire sur le sujet après la chirurgie, où les niveaux surveillés de CCL23, A1AT et CRP sont utilisés pour prédire ou diagnostiquer le SRIS et/ou une sepsie après une chirurgie.

7. Procédé de l'une quelconque des revendications 1 à 6, où :
a) le procédé distingue le SRIS de la sepsie ;
b) le procédé fournit une prédiction de la sepsie imminente ;
c) les marqueurs comprennent en outre un ou plusieurs, jusqu'à l'ensemble, de IL-6, TIMP1, sICAM-1 et PLA2 ;
d) une prédiction d'une sepsie imminente conduit au traitement de l'infection ; ou
e) le sujet n'est pas traité (par exemple avec un antibiotique) tant qu'une sepsie imminente n'est pas prédite ou une sepsie n'est pas diagnostiquée.

8. Procédé de l'une quelconque des revendications 4 à 7, où :
a) le procédé comprend en outre l'étape consistant à déterminer les niveaux d'au moins un marqueur supplémentaire choisi entre sICAM, PLA2, IL-6, la procalcitonine, MMP8, TNFalpha, AcPGP, l'activité MMP enzymatique, TIMP1, sRAGE et la desmosine ; ou
b) le procédé est réalisé sur un sujet atteint de SRIS et qui est utilisé pour identifier une infection chez le sujet.

9. Procédé de la revendication 8, dans lequel :
a) les marqueurs sont l'ensemble de la desmosine, TNF, IL-6, AcPGP, PLA2g2A, CCL23, A1AT, sICAM (éventuellement mesurée par ELISA), sICAM/VCAM-1 (éventuellement mesurées par écoulement latéral) et CRP ;
b) les marqueurs sont l'ensemble de CCL23, A1AT, sICAM (éventuellement mesurée par ELISA), sICAM/VCAM-1 (éventuellement mesurées par écoulement latéral) et CRP ; ou
c) les marqueurs comportent CRP, PLA2 et sICAM.

10. Procédé de l'une quelconque des revendications 4 à 9, où :
a) au moins 2, 3, 4, 5, 6, 7, 8, 9, 10, 11, 12, 13 ou 14 échantillons ou plus sont prélevés sur le sujet à des moments différents et les niveaux des au moins trois marqueurs sont déterminés ;
b) les échantillons sont prélevés toutes les 6 à 24 heures, par exemple quotidiennement, ou tous les 3, 4, 5, 6, 7 ou 14 jours ; ou
c) le procédé comprend l'étape consistant à déterminer les niveaux d'au moins quatre marqueurs.

11. Procédé de l'une quelconque des revendications 1 à 10, où :
a) le procédé comprend l'étape consistant à déterminer les niveaux d'au moins cinq ou six marqueurs, éventuellement où les marqueurs comprennent sICAM, CCL23, A1AT, CRP, IL-6 et TNFalpha ; ou
b) les marqueurs comprennent sICAM seule (éventuellement lorsqu'elle est mesurée par ELISA) et sICAM/VCAM-1 (éventuellement sICAM lorsqu'elle est mesurée par un dosage à écoulement latéral).

12. Procédé de sélection d'un sujet pour un traitement avec un antibiotique comprenant les étapes consistant à réaliser le procédé de l'une quelconque des revendications 1 à 11 et à sélectionner le sujet pour le traitement lorsqu'une sepsie est prédite ou diagnostiquée.

13. Antibiotique pour utilisation dans un procédé de traitement de la sepsie, où le sujet a été sélectionné pour le traitement en réalisant le procédé de l'une quelconque des revendications 1 à 12.

14. Antibiotique pour utilisation dans un procédé de traitement de la sepsie, où le sujet présente, dans un échantillon, un niveau modifié d'au moins CCL23, A1AT et CRP éventuellement conjointement avec un niveau modifié d'au moins l'un parmi sICAM, PLA2, IL-6, la procalcitonine, MMP8, TNFalpha, AcPGP, l'activité MMP enzymatique, TIMP1, sRAGE et la desmosine.

15. Antibiotique pour utilisation selon les revendications 13 et 14, où l'échantillon est un échantillon de sang total, de plasma ou de sérum.

16. Système ou kit de test pour prédire ou diagnostiquer le syndrome de réponse inflammatoire systémique (SRIS) et/ou la sepsie chez un sujet, comprenant :
a. un ou plusieurs dispositifs de test pour déterminer les niveaux d'au moins CCL23, A1AT et CRP éventuellement conjointement avec au moins l'un parmi sICAM, PLA2, IL-6, la procalcitonine, MMP8, TNFalpha, AcPGP, l'activité MMP enzymatique, TIMP1, sRAGE et la desmosine dans un échantillon ;
b. un processeur ; et
c. un support de stockage comprenant une application informatique qui, lorsqu'elle est exécutée par le processeur, est configurée pour :
i. accéder aux et/ou calculer les niveaux déterminés de chaque marqueur dans l'échantillon sur les un ou plusieurs dispositifs de test ;
ii. calculer un score de test à partir des niveaux des marqueurs dans l'échantillon qui prédit ou diagnostique le SRIS et/ou la sepsie ; et
iii. délivrer en sortie à partir du processeur le résultat prédit ou diagnostique pour le sujet.

17. Système ou kit de test pour surveiller un sujet, comprenant :
a. un ou plusieurs dispositifs de test pour déterminer les niveaux d'au moins CCL23, A1AT et CRP éventuellement conjointement avec au moins l'un parmi sICAM, PLA2, IL-6, la procalcitonine, MMP8, TNFalpha, AcPGP, l'activité MMP enzymatique, TIMP1, sRAGE et la desmosine dans un échantillon à plusieurs instants ;
b. un processeur ; et
c. un support de stockage comprenant une application informatique qui, lorsqu'elle est exécutée par le processeur, est configurée pour :
i. accéder aux et/ou calculer les niveaux déterminés de chaque marqueur dans l'échantillon sur les un ou plusieurs dispositifs de test ;
ii. calculer un score de test à partir des niveaux des marqueurs dans l'échantillon, comportant éventuellement une comparaison des niveaux avec ceux pris à un ou plusieurs instants antérieurs, pour ainsi prédire ou diagnostiquer leSRIS et/ou la sepsie ; et
iii. délivrer en sortie à partir du processeur le résultat prédit ou diagnostique pour le sujet ; éventuellement où le sujet a été soumis à une intervention chirurgicale et les plusieurs instants comportent le prélèvement d'un premier échantillon sur le sujet avant la chirurgie pour fournir des niveaux de base des marqueurs et le prélèvement d'au moins un échantillon supplémentaire sur le sujet après la chirurgie.

18. Dispositif de test, kit de test ou composition de matière de test comprenant :
a. une zone de réception d'échantillon à laquelle un échantillon provenant d'un sujet est ajouté
b. une zone de conjugué comprenant au moins trois réactifs de liaison marqués, dont chacun se lie spécifiquement à l'un de CCL23, A1AT et CRP, éventuellement conjointement avec au moins un réactif de liaison marqué supplémentaire qui se lie spécifiquement à sICAM, à PLA2, à IL-6, à la procalcitonine, à MMP8, au TNFalpha, à AcPGP, à l'activité MMP enzymatique, au TIMP1, au sRAGE ou à la desmosine
c. un support solide définissant un trajet d'écoulement de liquide pour l'échantillon et comprenant des lignes de test correspondantes pour chacun des au moins trois marqueurs, chaque ligne de test comprenant un réactif de liaison supplémentaire immobilisé qui se lie également spécifiquement à l'un de CCL23, A1AT et CRP, immobilisant ainsi le marqueur à la ligne de test pour produire un signal par l'intermédiaire du réactif de liaison marqué également lié spécifiquement au marqueur.

19. Dispositif de test, kit de test ou composition de matière de test de la revendication 18, où :
a) la zone de réception d'échantillon est proportionnée de manière à recevoir entre 10 et 100 µl de sérum, par exemple environ 80 µl de sérum ;
b) le support solide comprend un milieu chromatographique ;
c) le support solide comprend un dispositif à écoulement capillaire ;
d) le dispositif de test, le kit de test ou la composition de test est une bandelette réactive.

20. Dispositif de test, kit de test ou composition de matière de test de la revendication 18 ou 19 comprenant en outre un lecteur pour quantifier les niveaux des marqueurs aux lignes de test respectives, éventuellement où le lecteur comprend en outre :
A)
a. un processeur ; et
b. un support de stockage comprenant une application informatique qui, lorsqu'elle est exécutée par le processeur, est configurée pour :
i. accéder aux et/ou calculer les niveaux déterminés de chaque marqueur dans l'échantillon sur les un ou plusieurs dispositifs de test ;
ii. calculer un score de test à partir des niveaux des marqueurs dans l'échantillon qui prédit ou diagnostique leSRIS et/ou la sepsie ; et
iii. délivrer en sortie à partir du processeur le résultat prédit ou diagnostique pour le sujet ; ou
B)
a. un processeur ; et
b. un support de stockage comprenant une application informatique qui, lorsqu'elle est exécutée par le processeur, est configurée pour :
i. accéder aux et/ou calculer les niveaux déterminés de chaque marqueur dans l'échantillon sur les un ou plusieurs dispositifs de test ;
ii. calculer un score de test à partir des niveaux des marqueurs dans l'échantillon en comparant les niveaux à ceux pris à un ou plusieurs instants antérieurs pour ainsi prédire ou diagnostiquer le SRIS et/ou la sepsie ; et
iii. délivrer en sortie à partir du processeur le résultat prédit ou diagnostique pour le sujet.
